(19) Europäisches Patentamt
European Patent Office
Office européen des brevets

(11) EP 3 128 005 B1

(12) **EUROPEAN PATENT SPECIFICATION**

(45) Date of publication and mention of the grant of the patent:
**14.04.2021 Bulletin 2021/15**

(51) Int Cl.:
***C07K 14/705*** *(2006.01)* ***C12N 15/62*** *(2006.01)*
***A61K 38/17*** *(2006.01)*

(21) Application number: **16183261.3**

(22) Date of filing: **08.08.2016**

(54) **SIRP-ALPHA VARIANT CONSTRUCTS AND USES THEREOF**

SIRP-ALPHA-VARIANTEN-KONSTRUKTE UND VERWENDUNGEN DAVON

CONSTRUCTIONS DE VARIANTS SIRP-ALPHA ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **07.08.2015 US 201562202772 P**
**07.08.2015 US 201562202775 P**
**07.08.2015 US 201562202779 P**
**10.08.2015 TW 104125902**
**16.12.2015 US 201514971931**

(43) Date of publication of application:
**08.02.2017 Bulletin 2017/06**

(60) Divisional application:
**21161416.9**

(73) Proprietor: **ALX Oncology Inc.**
**Burlingame, CA 94010 (US)**

(72) Inventors:
- **DEMING, Laura**
  **South San Francisco, CA 94080 (US)**
- **GOODMAN, Corey**
  **South San Francisco, CA 94080 (US)**
- **PONS, Jaume**
  **South San Francisco, CA 94080 (US)**
- **SIM, Bang Janet**
  **South San Francisco, CA 94080 (US)**
- **VRLJIC, Marija**
  **South San Francisco, CA 94080 (US)**

(74) Representative: **J A Kemp LLP**
**14 South Square**
**Gray's Inn**
**London WC1R 5JJ (GB)**

(56) References cited:
**WO-A1-2010/070047** **WO-A1-2011/076781**
**WO-A1-2012/172521** **WO-A1-2013/063076**
**WO-A1-2013/109752** **WO-A1-2014/121093**
**WO-A1-2016/023040**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 128 005 B1

## Description

### BACKGROUND OF THE INVENTION

Signal-regulatory protein α (SIRP-α) is a protein widely expressed on the membrane of myeloid cells. SIRP-α interacts with CD47, a protein broadly expressed on many cell types in the body. The interaction of SIRP-α with CD47 prevents engulfment of "self" cells, which could otherwise be recognized by the immune system. SIRP-α was first discovered as a binder of SHP-2 (an SH-2 domain containing tyrosine phosphatase). CD47 was early characterized as an overexpressed antigen on ovarian carcinoma cells.

In 2000, Oldenborg et al. showed that administration of CD47-deficient red blood cells (RBCs) in a mouse model resulted in rapid clearance of the RBCs from the system, demonstrating CD47 to be a "protective" signal on some subset of "self" cells. Subsequently, the potential link between the SIRP-α and cancer was further explored. It was found that high CD47 expression on tumor cells acted, in acute myeloid leukemia (AML) and several solid tumor cancers, as a negative prognostic factor for survival. Strategies focused on disrupting the interaction between CD47 and SIRP-α, such as administration of agents that mask either CD47 or SIRP-α, have been explored as potential anticancer therapies.

WO 2016/023040 describes SIRP-α variant constructs and uses thereof. WO 2010/070047 describes soluble CD47 binding polypeptides for use in treating autoimmune and inflammatory disorders.

However, in considering these therapeutic strategies, it is a concerning issue that SIRP-α could bind to CD47 on many different cell types in the human body. Thus, there exists a need to engineer SIRP-α to preferentially bind to CD47 only on diseased cells or on cells at a diseased site.

### SUMMARY OF THE INVENTION

The invention features signal-regulatory protein α (SIRP-α) variant constructs. The present invention provides a signal-regulatory protein α (SIRP-α) variant construct, comprising a SIRP-α variant attached to a CD47-based blocking peptide by way of a cleavable linker, wherein the CD47-based blocking peptide has at least 80% sequence identity to a sequence of wild-type, IgSF domain of CD47 (SEQ ID NO: 35) or a fragment thereof that can bind a SIRP-α variant, and wherein the SIRP-α variant comprises the amino acid sequence set forth in $EEEX_1QX_2IQPDKSVLVAAGETX_3TLRCTX_4TSLX_5PVGPIQWFRGAGPGRX_6LIYNQX_7X_8$ $GX_9FPRVTTVSDX_{10}TX_{11}RNNMDFSIRIGX_{12}ITX_{13}ADAGTYYCX_{14}KX_{15}RKGSPDDVEX_{16}$ KSGAGTELSVRAKPS (SEQ ID NO: 13), wherein $X_1$ is L, I, or V; $X_2$ is V, L, or, I; $X_3$ is A or V; $X_4$ is A, I, or L; $X_5$ is I, T, S, or F; $X_6$ is E, V, or L; $X_7$ is K or R; $X_8$ is E or Q; $X_9$ is H, P, or R; $X_{10}$ is L, T, or G; $X_{11}$ is K or R; $X_{12}$ is N, A, C, D, E, F, G, H, I, K, L, M, P, Q, R, S, T, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V. The SIRP-α variant constructs include SIRP-α variants. In some embodiments, the SIRP-α variant constructs have preferential activity at a diseased site (e.g., at the site of a tumor than at a non-diseased site). In certain embodiments, the SIRP-α variant constructs have higher binding affinity to CD47 on diseased cells (e.g., tumor cells). In some embodiments, the SIRP-α variants bind with higher affinity to CD47 under acidic pH (e.g., less than around pH 7) and/or under hypoxic condition than under physiological conditions. In some embodiments, the SIRP-α variants contain one or more substitutions of amino acids with histidine residues or with other amino acids that allow preferential binding of SIRP-α variant constructs at a diseased site. In some embodiments, the SIRP-α variant constructs are prevented from binding to CD47 in a non-diseased site by a blocking peptide. In some embodiments, the SIRP-α variant constructs are targeted to the diseased site (e.g., the tumor) by a targeting moiety (e.g., an antibody directed to a tumor-associated antigen or an antibody-binding peptide). The invention also provides pharmaceutical compositions comprising: a) a therapeutically effective amount of the SIRP-α variant construct of the invention, and (b) a pharmaceutically acceptable excipient. The present invention further provides the SIRP-α variant constructs or the pharmaceutical compositions of the invention for use in methods of treating various diseases, such as cancer, preferably solid tumor cancer and hematological cancer.

In one aspect, the invention features a signal-regulatory protein α (SIRP-α) variant construct, wherein the SIRP-α variant construct preferentially binds CD47 on diseased cells or at a diseased site than on non-diseased cells. In some embodiments, the SIRP-α variant construct binds to CD47 on diseased cells or at a diseased site with higher affinity than it binds CD47 on non-diseased cells.

In some embodiments, the SIRP-α variant construct includes a SIRP-α variant attached to a blocking peptide. In some embodiments, the blocking peptide binds with higher affinity to a wild-type SIRP-α than to the SIRP-α variant. In some embodiments, the SIRP-α variant binds with higher affinity to a wild-type CD47 than to the blocking peptide.

The blocking peptide is a CD47-based blocking peptide. In some embodiments, the CD47-based blocking peptide includes a portion that has at least 80% amino acid sequence identity to the sequence of the wild-type, IgSF domain of CD47 (SEQ ID NO: 35) or a fragment thereof that can bind a SIRP-α variant. In some embodiments, the CD47-based blocking peptide has the sequence of SEQ ID NO: 38 or 40.

Provided herein are SIRP-α variant constructs comprising a SIRP-α variant described herein, wherein said SIRP-α variant is attached to a blocking peptide described herein by use of at least one cleavable linker. In some embodiments, the SIRP-α variant may comprise the same CD47 binding site as a wild type SIRP-α. In some embodiments, the SIRP-α variant may comprise one or more mutations, or insertions as compared to a wild type SIRP-α. In some embodiments, the SIRP-α variant may be a truncated form of the wild type SIRP-α. In some embodiments, the blocking peptide may be a CD47 mimic, variant, or fragment described herein. In some embodiments, the blocking peptide may exhibit a higher affinity for a wild-type SIRP-α, as compared to the SIRP-α variant in the SIRP-α variant construct. In some embodiments, the blocking peptide may be a CD47 variant polypeptide that demonstrates a lower affinity for a SIRP-α variant as compared to the wild-type CD47. In some embodiments, the linker between the SIRP-α variant and the blocking peptide may be at least one linker that is optionally cleavable by one or more proteases. In some embodiments, the linker optionally also comprises one or more spacers.

The SIRP-α variant is attached to a blocking peptide by way of a cleavable linker and optionally one or more spacers. In some embodiments, the cleavable linker is cleaved under acidic pH and/or hypoxic condition. In some embodiments, the cleavable linker is cleaved by a tumor-associated enzyme. In some embodiments, the tumor-associated enzyme is a protease. In some embodiments, the protease is selected from the group consisting of matriptase (MTSP1), urinary-type plasminogen activator (uPA), legumain, PSA (also called KLK3, kallikrein-related peptidase-3), matrix metalloproteinase-2 (MMP-2), MMP9, human neutrophil elastase (HNE), and proteinase 3 (Pr3). In some embodiments, the protease is matriptase. In some embodiments, the cleavable linker has the sequence of LSGRSDNH (SEQ ID NO: 47) or any one of the sequences listed in Table 7. In some embodiments, the cleavable linker includes one or a combination of the following sequences (see, e.g., Table 7): PRFKIIGG, PRFRIIGG, SSRHRRALD, RKSSIIIRMRDVVL, SSSFDKGKYKKGDDA, SSSFDKGKYKRGDDA, IEGR, IDGR, GGSIDGR, PLGLWA, GPLGIAGI, GPEGLRVG, YGAGLGVV, AGLGVVER, AGLGISST, DVAQFVLT, VAQFVLTE, AQFVLTEG, PVQPIGPQ, L/S/G-/R--/S-/D/N/H, - /s/gs/Rk-/rv/-/-/-, SGR-SA, L/S/G-/R--/S-/D/N/H, r/-/-/Rk-/v-/-/g/-, RQAR-VV, r/-/-/Rk/v/-/g, /Kr/RKQ/gAS/RK/A, L/S/G-/R-/S-/D/N/H, -/-/-/N/-/-/-, AAN-L, ATN-L, si/sq/-/yqrs/s/-/-, S/S/K/L/Q, -/p/-/- /li/- /-/-, g/pa/-/gl/-/g/-, G/P/L/G/I/A/G/Q, P/V/G/L/I/G, H/P/V/G/L/L/A/R, -/-/-/viat-/-/-/-, and -/y/y/vta -/-/-/-, where "-" means any amino acid (i.e., any naturally occurring amino acid), capital case indicates an strong preference for that amino acid, lower case indicates a minor preference for that amino acid, and "/" separates amino acid positions in cases where more than one amino acid at a position adjacent to the "/"is possible.

In some embodiments, the SIRP-α variant is attached to an antibody-binding peptide. In some embodiments, the antibody-binding peptide binds to a constant region of an antibody reversibly or irreversibly. In some embodiments, the antibody-binding peptide binds to the fragment antigen-binding (Fab) region of an antibody reversibly or irreversibly. In some embodiments, the antibody-binding peptide binds to a variable region of an antibody reversibly or irreversibly. In some embodiments, the antibody is Cetuximab. In some embodiments, the antibody-binding peptide has at least 75% amino acid sequence identity to the sequence of a disease localization peptide (DLP) (CQFDLSTRRLKC (SEQ ID NO: 64) or CQYNLSSRALKC (SEQ ID NO: 65)) or a fragment thereof. In some embodiments, the antibody-binding peptide has the sequence of SEQ ID NO: 64.

In some embodiments, the SIRP-α variant is attached to an Fc domain monomer. In some embodiments, the SIRP-α variant is attached to a human serum albumin (HSA). In some embodiments, the HSA includes amino acid substitution C34S and/or K573P, relative to SEQ ID NO: 67. In some embodiments, the HSA has the sequence of

DAHKSEVAHRFKDLGEENFKALVLIAFAQYLQQSPFEDHVKLVNEVTEFAKTCVA
DESAENCDKSLHTLFGDKLCTVATLRETYGEMADCCAKQEPERNECFLQHKDDN

PNLPRLVRPEVDVMCTAFHDNEETFLKKYLYEIARRHPYFYAPELLFFAKRYKAA FTECCQAADKAACLLPKLDELRDEGKASSAKQRLKCASLQKFGERAFKAWAVAR LSQRFPKAEFAEVSKLVTDLTKVHTECCHGDLLECADDRADLAKYICENQDSISSK LKECCEKPLLEKSHCIAEVENDEMPADLPSLAADFVESKDVCKNYAEAKDVFLGM FLYEYARRHPDYSVVLLLRLAKTYETTLEKCCAAADPHECYAKVFDEFKPLVEEP QNLIKQNCELFEQLGEYKFQNALLVRYTKKVPQVSTPTLVEVSRNLGKVGSKCCK HPEAKRMPCAEDYLSWLNQLCVLHEKTPVSDRVTKCCTESLVNRRPCFSALEVDE TYVPKEFNAETFTFHADICTLSEKERQIKKQTALVELVKHKPKATKEQLKAVMDD FAAFVEKCCKADDKETCFAEEGKKLVAASQAALGL (SEQ ID NO: 68).

In some embodiments, the SIRP-α variant is attached to an albumin-binding peptide. In some embodiments, the albumin-binding peptide has the sequence of SEQ ID NO: 2. In some embodiments, the SIRP-α variant is attached to a polymer, wherein the polymer is polyethylene glycol (PEG) chain or polysialic acid chain.

In some embodiments, the SIRP-α variant is attached to an antibody. In some embodiments, the antibody is a tumor-specific antibody. In some embodiments, the antibody (e.g., a tumor-specific antibody) is selected from the group consisting of cetuximab, pembrolizumab, nivolumab, pidilizumab, MEDI0680, MEDI6469, Ipilimumab, tremelimumab, urelumab, vantictumab, varlilumab, mogamalizumab, anti-CD20 antibody, anti-CD19 antibody, anti-CS1 antibody, herceptin, trastuzumab, and pertuzumab. In some embodiments, the antibody (e.g., a tumor-specific antibody) may bind to one or more of the following: 5T4, AGS-16, ALK1, ANG-2, B7-H3, B7-H4, c-fms, c-Met, CA6, CD123, CD19, CD20, CD22, EpCAM, CD30, CD32b, CD33, CD37, CD38, CD40, CD52, CD70, CD74, CD79b, CD98, CEA, CEACAM5, CLDN18.2, CLDN6, CS1, CXCR4, DLL-4, EGFR, EGP-1, ENPP3, EphA3, ETBR, FGFR2, fibronectin, FR-alpha, GCC, GD2, glypican-3, GPNMB, HER-2, HER3, HLA-DR, ICAM-1, IGF-1R, IL-3R, LIV-1, mesothelin, MUC16, MUC1, NaPi2b, Nectin-4, Notch 2, Notch 1, PD-L1, PD-L2, PDGFR-α, PS, PSMA, SLTRK6, STEAP1, TEM1, VEGFR, CD25, CD27L, DKK-1, and/or CSF-1 R.

In some embodiments, the SIRP-α variant in the SIRP-α variant construct has at least 80% (e.g., at least 85%, 87%, 90%, 91 %, 92%, 93%, 94%, 95%, 96%, 97%, 98%, or 99%) sequence identity to a sequence of any one of SEQ ID NOs: 3-12 and 24-34.

In some embodiments, the SIRP-α variant in the SIRP-α variant construct has a sequence of EEEX$_1$QX$_2$IQPDKSVLVAAGETX$_3$TLRCTX$_4$TSLX$_5$PVGPIQWFRGAGPGRX$_6$LIYN QX$_7$X$_8$GX$_9$FPRVTTVSDX$_{10}$TX$_{11}$RNNMDFSIRIGX$_{12}$ITX$_{13}$ADAGTYYCX$_{14}$KX$_{15}$RKG SPDDVEX$_{16}$KSGAGTELSVRAKPS (SEQ ID NO: 13), wherein X$_1$ is L, I, or V; X$_2$ is V, L, or, I; X$_3$ is A or V; X$_4$ is A, I, or L; X$_5$ is I, T, S, or F; X$_6$ is E, V, or L; X$_7$ is K or R; X$_8$ is E or Q; X$_9$ is H, P, or R; X$_{10}$ is L, T, or G; X$_{11}$ is K or R; X$_{12}$ is N, A, C, D, E, F, G, H, I, K, L, M, P, Q, R, S, T, V, W, or Y; X$_{13}$ is P, A, C, D, E, F, G, H, I, K, L, M, N, Q, R, S, T, V, W, or Y; X$_{14}$ is V or I; X$_{15}$ is F, L, or V; and X$_{16}$ is F or V.

In some aspects, the SIRP-α variant in the SIRP-α variant construct has a sequence of EEGX$_1$QX$_2$IQPDKSVSVAAGESX$_3$ILHCTX$_4$TSLX$_5$PVGPIQWFRGAGPGRX$_6$LIYNQ X$_7$X$_8$GX$_9$FPRVTTVSDX$_{10}$TX$_{11}$RNNMDFSIRIGX$_{12}$ITX$_{13}$ADAGTYYCX$_{14}$KX$_{15}$RKGSP DDVEX$_{16}$KSGAGTELSVRAKPS (SEQ ID NO: 16), wherein X$_1$ is L, I, or V; X$_2$ is V, L, or, I; X$_3$ is A or V; X$_4$ is A, I, or L; X$_5$ is I, T, S, or F; X$_6$ is E, V, or L; X$_7$ is K or R; X$_8$ is E or Q; X$_9$ is H, P, or R; X$_{10}$ is L, T, or G; X$_{11}$ is K or R; X$_{12}$ is N, A, C, D, E, F, G, H, I, K, L, M, P, Q, R, S, T, V, W, or Y; X$_{13}$ is P, A, C, D, E, F, G, H, I, K, L, M, N, Q, R, S, T, V, W, or Y; X$_{14}$ is V or I; X$_{15}$ is F, L, or V; and X$_{16}$ is F or V.

In some aspects, the SIRP-α variant in the SIRP-α variant construct has a sequence of EEEX$_1$QX$_2$IQPDKFVLVAAGETX$_3$TLRCTX$_4$TSLX$_5$PVGPIQWFRGAGPGRX$_6$LIYNQX $_7$X$_8$GX$_9$FPRVTTVSDX$_{10}$TX$_{11}$RNNMDFSIRIGX$_{12}$ITX$_{13}$ADAGTYYCX$_{14}$KX$_{15}$RKGSPD DVEX$_{16}$KSGAGTELSVRAKPS (SEQ ID NO: 17), wherein X$_1$ is L, I, or V; X$_2$ is V, L, or, I; X$_3$ is A or V; X$_4$ is A, I, or L; X$_5$ is I, T, S, or F; X$_6$ is E, V, or L; X$_7$ is K or R; X$_8$ is E or Q; X$_9$ is H, P, or R; X$_{10}$ is L, T, or G; X$_{11}$ is K or R; X$_{12}$ is N, A, C, D, E, F, G, H, I, K, L, M, P, Q, R, S, T, V, W, or Y; X$_{13}$ is P, A, C, D, E, F, G, H, I, K, L, M, N, Q, R, S, T, V, W, or Y; X$_{14}$ is V or I; X$_{15}$ is F, L, or V; and X$_{16}$ is F or V.

In someaspects, the SIRP-α variant in the SIRP-α variant construct has a sequence of EEEX$_1$QX$_2$IQPDKSVLVAAGETX$_3$TLRCTX$_4$TSLX$_5$PVGPIQWFRGAGPGRX$_6$LIYNQX $_7$X$_8$GX$_9$FPRVTTVSDX$_{10}$TX$_{11}$RNNMDFPIRIGX$_{12}$ITX$_{13}$ADAGTYYCX$_{14}$KX$_{15}$RKGSPD DVEX$_{16}$KSGAGTELSVRAKPS

(SEQ ID NO: 18), wherein $X_1$ is L, I, or V; $X_2$ is V, L, or, I; $X_3$ is A or V; $X_4$ is A, I, or L; $X_5$ is I, T, S, or F; $X_6$ is E, V, or L; $X_7$ is K or R; $X_8$ is E or Q; $X_9$ is H, P, or R; $X_{10}$ is L, T, or G; $X_{11}$ is K or R; $X_{12}$ is N, A, C, D, E, F, G, H, I, K, L, M, P, Q, R, S, T, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V.

In some aspects, the SIRP-α variant in the SIRP-α variant construct has a sequence of EEE$X_1$Q$X_2$IQPDKSVLVAAGET$X_3$TLRCT$X_4$TSL$X_5$PVGPIQWFRGAGPGR$X_6$LIYNQ$X_7$$X_8$G$X_9$FPRVTTVSD$X_{10}$T$X_{11}$RNNMDFSIRIS$X_{12}$IT$X_{13}$ADAGTYYC$X_{14}$K$X_{15}$RKGSPDDVE$X_{16}$KSGAGTELSVRAKPS (SEQ ID NO: 21), wherein $X_1$ is L, I, or V; $X_2$ is V, L, or, I; $X_3$ is A or V; $X_4$ is A, I, or L; $X_5$ is I, T, S, or F; $X_6$ is E, V, or L; $X_7$ is K or R; $X_8$ is E or Q; $X_9$ is H, P, or R; $X_{10}$ is L, T, or G; $X_{11}$ is K or R; $X_{12}$ is N, A, C, D, E, F, G, H, I, K, L, M, P, Q, R, S, t, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V.

In some aspects, the SIRP-α variant in the SIRP-α variant construct has a sequence of EEE$X_1$Q$X_2$IQPDKSVSVAAGES$X_3$ILHCT$X_4$TSL$X_5$PVGPIQWFRGAGPAR$X_6$LIYNQ$X_7$$X_8$G$X_9$FPRVTTVSE$X_{10}$T$X_{11}$RENMDFSISIS$X_{12}$IT$X_{13}$ADAGTYYC$X_{14}$K$X_{15}$RKGSPDTE$X_{16}$KSGAGTELSVRAKPS (SEQ ID NO: 14), wherein $X_1$ is L, I, or V; $X_2$ is V, L, or, I; $X_3$ is A or V; $X_4$ is V, I, or L; $X_5$ is I, T, S, or F; $X_6$ is E, V, or L; $X_7$ is K or R; $X_8$ is E or Q; $X_9$ is H, P, or R; $X_{10}$ is S, T, or G; $X_{11}$ is K or R; $X_{12}$ is N, A, C, D, E, F, G, H, I, K, L, M, P, Q, R, S, T, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V.

In some aspects, the SIRP-α variant in the SIRP-α variant construct has a sequence of EEE$X_1$Q$X_2$IQPDKSVSVAAGES$X_3$ILLCT$X_4$TSL$X_5$PVGPIQWFRGAGPAR$X_6$LIYNQ$X_7$$X_8$G$X_9$FPRVTTVSE$X_{10}$T$X_{11}$RENMDFSISIS$X_{12}$IT$X_{13}$ADAGTYYC$X_{14}$K$X_{15}$RKGSPDTE$X_{16}$KSGAGTELSVRAKPS (SEQ ID NO: 15), wherein $X_1$ is L, I, or V; $X_2$ is V, L, or, I; $X_3$ is A or V; $X_4$ is V, I, or L; $X_5$ is I, T, S, or F; $X_6$ is E, V, or L; $X_7$ is K or R; $X_8$ is E or Q; $X_9$ is H, P, or R; $X_{10}$ is S, T, or G; $X_{11}$ is K or R; $X_{12}$ is N, A, C, D, E, F, G, H, I, K, L, M, P, Q, R, S, T, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V.

In some aspects, the SIRP-α variant in the SIRP-α variant construct has a sequence of EEE$X_1$Q$X_2$IQPDKSVSVAAGES$X_3$ILHCT$X_4$TSL$X_5$PVGPIQWFRGAGPAR$X_6$LIYNQ$X_7$$X_8$G$X_9$FPRVTTVSE$X_{10}$T$X_{11}$RENMDFSISIS$X_{12}$IT$X_{13}$ADAGTYYC$X_{14}$K$X_{15}$RKGSPDTE$X_{16}$KSGAGTELSVRGKPS (SEQ ID NO: 19), wherein $X_1$ is L, I, or V; $X_2$ is V, L, or, I; $X_3$ is A or V; $X_4$ is V, I, or L; $X_5$ is I, T, S, or F; $X_6$ is E, V, or L; $X_7$ is K or R; $X_8$ is E or Q; $X_9$ is H, P, or R; $X_{10}$ is S, T, or G; $X_{11}$ is K or R; $X_{12}$ is N, A, C, D, E, F, G, H, I, K, L, M, P, Q, R, S, T, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V.

In some aspects, the SIRP-α variant in the SIRP-α variant construct has a sequence of EEE$X_1$Q$X_2$IQPDKSVSVAAGES$X_3$ILHCT$X_4$TSL$X_5$PVGPIQWFRGAGPAR$X_6$LIYNQ$X_7$$X_8$G$X_9$FPRVTTVSE$X_{10}$T$X_{11}$RENMDFSISIS$X_{12}$IT$X_{13}$ADAGTYYC$X_{14}$K$X_{15}$RKGSPDTE$X_{16}$KSGAGTELSVRAKPS (SEQ ID NO: 22), wherein $X_1$ is L, I, or V; $X_2$ is V, L, or, I; $X_3$ is A or V; $X_4$ is V, I, or L; $X_5$ is I, T, S, or F; $X_6$ is E, V, or L; $X_7$ is K or R; $X_8$ is E or Q; $X_9$ is H, P, or R; $X_{10}$ is S, T, or G; $X_{11}$ is K or R; $X_{12}$ is N, A, C, D, E, F, G, H, I, K, L, M, P, Q, R, S, T, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V.

In some aspects, the SIRP-α variant in the SIRP-α variant construct has a sequence of EEE$X_1$Q$X_2$IQPDKSVLVAAGET$X_3$TLRCT$X_4$TSL$X_5$PVGPIQWFRGAGPAR$X_6$LIYNQ$X_7$$X_8$G$X_9$FPRVTTVSE$X_{10}$T$X_{11}$RENMDFSISIS$X_{12}$IT$X_{13}$ADAGTYYC$X_{14}$K$X_{15}$RKGSPDTE$X_{16}$KSGAGTELSVRAKPS (SEQ ID NO: 20), wherein $X_1$ is L, I, or V; $X_2$ is V, L, or, I; $X_3$ is A or V; $X_4$ is A, I, or L; $X_5$ is I, T, S, or F; $X_6$ is E, V, or L; $X_7$ is K or R; $X_8$ is E or Q; $X_9$ is H, P, or R; $X_{10}$ is S, T, or G; $X_{11}$ is K or R; $X_{12}$ is N, A, C, D, E, F, G, H, I, K, L, M, P, Q, R, S, T, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V.

In some aspects, the SIRP-α variant in the SIRP-α variant construct has a sequence of EE$X_1$$X_2$Q$X_3$IQPDK$X_4$V$X_5$VAAGE$X_6$$X_7$$X_8$L$X_9$CT$X_{10}$TSL$X_{11}$PVGPIQWFRGAGP$X_{12}$R$X_{13}$LIYNQ$X_{14}$$X_{15}$G$X_{16}$FPRVTTVS$X_{17}$$X_{18}$T$X_{19}$R$X_{20}$NMDF$X_{21}$I$X_{22}$$X_{23}$$X_{24}$IT$X_{25}$ADAGTYYC$X_{26}$K$X_{27}$RKGSPD$X_{28}$$X_{29}$E$X_{30}$KSGAGTELSVR$X_{31}$KPS (SEQ ID NO: 23), wherein $X_1$ is E or G; $X_2$ is L, I, or V; $X_3$ is V, L, or, I; $X_4$ is S or F; $X_5$ is L or S; $X_6$ is S or T; $X_7$ is A or V; $X_8$ is I or T; $X_9$ is H or R; $X_{10}$ is A, V, I, or L; $X_{11}$ is I, T, S, or F; $X_{12}$ is A or G; $X_{13}$ is E, V, or L; $X_{14}$ is K or R; $X_{15}$ is E or Q; $X_{16}$ is H, P, or R; $X_{17}$ is D or E; $X_{18}$ is S, L, T, or G; $X_{19}$ is K or R; $X_{20}$ is E or N; $X_{21}$ is S or P; $X_{22}$ is S or R; $X_{23}$ is S or G; $X_{24}$ is N, A, C, D, E, F, G, H, I, K, L, M, P, Q, R, S, T, V, W, or Y; $X_{25}$ is P, A, C, D, E, F, G, H, I, K, L, M, N, Q, R, S, T, V, W, or Y; $X_{26}$ is V or I; $X_{27}$ is F, L, V; $X_{28}$ is D or absent; $X_{29}$ is T or V; $X_{30}$ is F or V; and $X_{31}$ is A or G.

In some aspects, the SIRP-α variant in the SIRP-α variant construct has at least 80% (e.g., at least 85%, 87%, 90%, 91 %, 92%, 93%, 94%, 95%, 96%, 97%, 98%, or 99%) sequence identity to a sequence of any one of SEQ ID NOs: 13-23.

In some embodiments, a SIRP-α variant in the SIRP-α variant construct does not include the sequence of any one of SEQ ID NOs: 3-12 and 24-34.

In some aspects, the SIRP-α variant in the SIRP-α variant construct includes one or more substitutions of amino acid residues with histidine residues. In some aspects, the one or more substitutions of amino acid residues with histidine residues are located at one or more of the following amino acid positions: 29, 30, 31, 32, 33, 34, 35, 52, 53, 54, 66, 67, 68, 69, 74, 93, 96, 97, 98, 100, 4, 6, 27, 36, 39, 47, 48, 49, 50, 57, 60, 72, 74, 76, 92, 94, 103, relative to a sequence of any one of SEQ ID NOs: 3-12.

In some embodiments, the SIRP-α variant construct binds with at least two, at least four, or at least six fold higher affinity to CD47 on diseased cells or at a diseased site than on non-diseased cells.

In some embodiments, the SIRP-α variant construct binds with at least two, at least four, or at least six fold higher affinity to CD47 under acidic pH than under neutral pH.

In some embodiments, the SIRP-α variant construct binds with at least two, at least four, or at least six fold higher affinity to CD47 under hypoxic condition than under physiological condition.

In some embodiments, the diseased cell is a cancer cell of a cancer disease.

In some embodiments, the acidic pH is a pH between about 4 to about 7.

In another aspect, the invention features a nucleic acid molecule encoding a SIRP-α variant construct described herein.

In another aspect, the invention features a vector including the nucleic acid molecule encoding a SIRP-α variant construct described herein.

In another aspect, the invention features a host cell that expresses a SIRP-α variant construct described herein, wherein the host cell includes a nucleic acid molecule encoding a SIRP-α variant construct described herein or a vector including the nucleic acid molecule, wherein the nucleic acid molecule or vector is expressed in the host cell.

In another aspect, the invention features a method of preparing a SIRP-α variant construct described herein, wherein the method includes: a) providing a host cell including a nucleic acid molecule of encoding a SIRP-α variant construct described herein or a vector including the nucleic acid molecule; b) expressing the nucleic acid molecule or vector in the host cell under conditions that allow for the formation of the SIRP-α variant construct; and c) recovering the SIRP-α variant construct.

In another aspect, the invention features a pharmaceutical composition including a therapeutically effective amount of a SIRP-α variant construct described herein, and one or more pharmaceutically acceptable carriers or excipients.

In another aspect, described herein is a method of increasing phagocytosis of a target cell in a subject including administering to the subject a SIRP-α variant construct described herein or a pharmaceutical composition including a therapeutically effective amount of a SIRP-α variant construct described herein. In some embodiments, the target cell is a cancer cell.

In another aspect, described herein is a method of eliminating regulatory T-cells in a subject including administering to the subject a SIRP-α variant construct described herein or a pharmaceutical composition including a therapeutically effective amount of a SIRP-α variant construct described herein.

In another aspect, described herein is a method for killing a cancer cell, the method includes contacting the cancer cell with a SIRP-α variant construct described herein or the pharmaceutical composition including a therapeutically effective amount of a SIRP-α variant construct described herein.

In another aspect, described herein is a method for treating a disease associated with SIRP-α and/or CD47 activity in a subject, the method includes administering to the subject a therapeutically effective amount of the SIRP-α variant construct described herein or the pharmaceutical composition including a therapeutically effective amount of a SIRP-α variant construct described herein.

In another aspect, described herein is a method of treating a disease associated with SIRP-α and/or CD47 activity in a subject, the method includes: (a) determining the amino acid sequences of SIRP-α of the subject; and (b) administering to the subject a therapeutically effective amount of a SIRP-α variant construct described herein; wherein the SIRP-α variant in the SIRP-α variant construct has the same amino acid sequence as that of a SIRP-α of the subject.

In another aspect, described herein is a method of treating a disease associated with SIRP-α and/or CD47 activity in a subject, the method includes: (a) determining the amino acid sequences of SIRP-α of the subject; and (b) administering to the subject a therapeutically effective amount of a SIRP-α variant construct described herein; wherein the SIRP-α variant in the SIRP-α variant construct has minimal immunogenicity in the subj ect.

In another aspect, described herein is a method of treating a disease associated with SIRP-α and/or CD47 activity in a subject, the method includes: administering to the subject a SIRP-α variant construct described herein, wherein the SIRP-α variant construct preferentially binds CD47 on diseased cells or at a diseased site over CD47 on non-diseased cells.

In some embodiments, the disease is cancer. In some embodiments, the cancer is selected from solid tumor cancer, hematological cancer, acute myeloid leukemia, chronic lymphocytic leukemia, chronic myeloid leukemia, acute

lymphoblastic leukemia, non-Hodgkin lymphoma, Hodgkin lymphoma, multiple myeloma, bladder cancer, pancreatic cancer, cervical cancer, endometrial cancer, lung cancer, bronchus cancer, liver cancer, ovarian cancer, colon and rectal cancer, stomach cancer, gastric cancer, gallbladder cancer, gastrointestinal stromal tumor cancer, thyroid cancer, head and neck cancer, oropharyngeal cancer, esophageal cancer, melanoma, non-melanoma skin cancer, Merkel cell carcinoma, virally induced cancer, neuroblastoma, breast cancer, prostate cancer, renal cancer, renal cell cancer, renal pelvis cancer, leukemia, lymphoma, sarcoma, glioma, brain tumor, and carcinoma. In some embodiments, the cancer is a solid tumor cancer. In some embodiments, the cancer is a hematological cancer.

In some aspects, the disease is an immunological disease. In some aspects, the immunological disease is an autoimmune disease or an inflammatory disease. In some aspects, the autoimmune or inflammatory disease is multiple sclerosis, rheumatoid arthritis, a spondyloarthropathy, systemic lupus erythematosus, an antibody-mediated inflammatory or autoimmune disease, graft versus host disease, sepsis, diabetes, psoriasis, atherosclerosis, Sjogren's syndrome, progressive systemic sclerosis, scleroderma, acute coronary syndrome, ischemic reperfusion, Crohn's Disease, endometriosis, glomerulonephritis, myasthenia gravis, idiopathic pulmonary fibrosis, asthma, acute respiratory distress syndrome (ARDS), vasculitis, or inflammatory autoimmune myositis.

In another aspect, described herein is a method of increasing hematopoietic stem cell engraftment in a subject including modulating the interaction between SIRP-$\alpha$ and CD47 in the subject by administering to the subject a SIRP-$\alpha$ variant described herein or a pharmaceutical composition including a therapeutically effective amount of a SIRP-$\alpha$ variant described herein.

In another aspect, described herein is a method of altering an immune response in a subject including administering the subject a SIRP-$\alpha$ variant construct described herein or a pharmaceutical composition including a therapeutically effective amount of a SIRP-$\alpha$ variant construct described herein, thereby altering the immune response in the subject. In some aspects, the immune response includes suppressing the immune response.

In some embodiments, the subject is a mammal, preferably, the mammal is a human.

*Definitions*

As used herein, the term "diseased cells" and "diseased tissue" refer to, for example, cancer cells and tissue. In particular, the cancer may be a solid tumor cancer or a hematological cancer. For example, if the cancer is a solid tumor cancer, the diseased cells are the cells of the solid tumor. Diseased cells are often living under conditions characteristic of a diseased site, such as acidic pH and hypoxia. "Diseased cells" and "diseased tissue" can also be associated with other diseases including, but not limited to, cancer. "Diseased cells" and "diseased tissue" can also be associated with an immunological disease or disorder, a cardiovascular disease or disorder, a metabolic disease or disorder, or a proliferative disease or disorder. An immunological disorder includes an inflammatory disease or disorder and an autoimmune disease or disorder.

As used herein, the term "non-diseased cells" refers to normal, healthy cells of the body. Non-diseased cells often live under physiological conditions, such as neutral pH and adequate oxygen concentration that maintain normal metabolism and regulatory functions of the cells.

As used herein, the term "diseased site" refers to the location or area proximal to the location of the disease in the body. For example, if the disease is solid tumor cancer located in the liver, then diseased site is the site of the tumor in the liver and areas close to the tumor in the liver. Cells at a diseased site may include diseased cells as well as cells that support the disease at the diseased site. For example, if the diseased site is the site of a tumor, cells at the site of the tumor include both diseased cells (e.g., tumor cells) and cells supporting tumor growth at the site of the tumor. Similarly, the term "cancer site" refers to the location of the cancer in the body.

As used herein, the term "SIRP-$\alpha$ D1 domain" or "D1 domain" refers to the membrane distal, extracellular domain of SIRP-$\alpha$. The SIRP-$\alpha$ D1 domain is located at the N-terminus of a full-length, wild-type SIRP-$\alpha$ and mediates binding to CD47. Amino acid sequences of D1 domains are shown in Table 1.

As used herein, the term "SIRP-$\alpha$ D2 domain" or "D2 domain" refers to the second extracellular domain of SIRP-$\alpha$. The SIRP-$\alpha$ D2 domain includes approximately amino acids 119 to 220 of a full-length, wild-type SIRP-$\alpha$.

As used herein, the term "SIRP-$\alpha$ D3 domain" or "D3 domain" refers to the third extracellular domain of SIRP-$\alpha$. The SIRP-$\alpha$ D3 domain includes approximately amino acids 221 to 320 of a full-length, wild-type SIRP-$\alpha$.

As used herein, the term "SIRP-$\alpha$ polypeptide" refers to a wild-type SIRP-$\alpha$ as well as a SIRP-$\alpha$ variant, as each term is defined and described herein.

As used herein, the term "SIRP-$\alpha$ variant" refers to a polypeptide containing a SIRP-$\alpha$ D1 domain, or a CD47-binding portion of a full-length SIRP-$\alpha$. In some embodiments, the SIRP-$\alpha$ variant has at least 80% (e.g., at least 85%, 87%, 90%, 91%, 92%, 93%, 94%, 95%, 96%, 97%, 98%, or 99%.) sequence identity to a sequence of any one of SEQ ID NOs: 3-12 and 24-34. In some embodiments, a SIRP-$\alpha$ variant has higher affinity to CD47 than a wild-type SIRP-$\alpha$. In some embodiments, a SIRP-$\alpha$ variant contains a portion of wild-type human SIRP-$\alpha$ (preferably a CD47-binding portion of the wild-type SIRP-$\alpha$) and/or has one or more amino acid substitutions. For example, a SIRP-$\alpha$ variant may

contain substitutions of one or more (e.g., one, two, three, four, five, six, seven, eight, nine, ten, etc, with a maximum of 20) amino acid residues relative to a wild-type SIRP-α. For example, a SIRP-α variant may contain substitutions of one or more (e.g., one, two, three, four, five, six, seven, eight, nine, ten, etc, with a maximum of 20) amino acid residues with histidine residues. In some embodiments, SIRP-α variants have a portion that has at least 80% (e.g., at least 85%, 87%, 90%, 91%, 92%, 93%, 94%, 95%, 96%, 97%, 98%, or 99%) amino acid sequence identity to a sequence of wild-type human SIRP-α or to any of the SIRP-α variants described herein (e.g., to a sequence of a CD47-binding portion of wild-type human SIRP-α). A CD47-binding portion of wild-type SIRP-α includes the D1 domain of the wild-type SIRP-α (a sequence of any one of SEQ ID NOs: 3-12).

As used herein, the term "SIRP-α variant construct" refers to a polypeptide containing a SIRP-α variant attached to, e.g., a blocking peptide, an Fc domain monomer, an HSA, an albumin-binding peptide, a polymer, an antibody-binding peptide, an antibody. In some embodiments, a SIRP-α variant construct has preferential activity at a diseased site. In some embodiments, SIRP-α variant constructs have preferential activity at a diseased site and include a SIRP-α variant having a portion that has at least 80% (e.g., at least 85%, 87%, 90%, 91%, 92%, 93%, 94%, 95%, 96%, 97%, 98%, or 99%) amino acid sequence identity to a sequence of wild-type human SIRP-α or to any of the SIRP-α variants described herein (e.g., to a sequence of a CD47-binding portion of wild-type human SIRP-α).

As used herein, the term "percent (%) identity" refers to the percentage of amino acid (or nucleic acid) residues of a candidate sequence, e.g., a SIRP-α variant, that are identical to the amino acid (or nucleic acid) residues of a reference sequence, e.g., a wild-type human SIRP-α or a CD47-binding portion thereof, after aligning the sequences and introducing gaps, if necessary, to achieve the maximum percent identity (i.e., gaps can be introduced in one or both of the candidate and reference sequences for optimal alignment and non-homologous sequences can be disregarded for comparison purposes). Alignment for purposes of determining percent identity can be achieved in various ways that are within the skill in the art, for instance, using publicly available computer software such as BLAST, ALIGN, or Megalign (DNASTAR) software. Those skilled in the art can determine appropriate parameters for measuring alignment, including any algorithms needed to achieve maximal alignment over the full length of the sequences being compared. In some embodiments, the percent amino acid (or nucleic acid) sequence identity of a given candidate sequence to, with, or against a given reference sequence (which can alternatively be phrased as a given candidate sequence that has or includes a certain percent amino acid (or nucleic acid) sequence identity to, with, or against a given reference sequence) is calculated as follows:

$$100 \times (\text{fraction of } A/B)$$

where A is the number of amino acid (or nucleic acid) residues scored as identical in the alignment of the candidate sequence and the reference sequence, and where B is the total number of amino acid (or nucleic acid) residues in the reference sequence. In some embodiments where the length of the candidate sequence does not equal to the length of the reference sequence, the percent amino acid (or nucleic acid) sequence identity of the candidate sequence to the reference sequence would not equal to the percent amino acid (or nucleic acid) sequence identity of the reference sequence to the candidate sequence.

In particular embodiments, a reference sequence aligned for comparison with a candidate sequence may show that the candidate sequence exhibits from 50% to 100% identity across the full length of the candidate sequence or a selected portion of contiguous amino acid (or nucleic acid) residues of the candidate sequence. The length of the candidate sequence aligned for comparison purpose is at least 30%, e.g., at least 40%, e.g., at least 50%, 60%, 70%, 80%, 90%, or 100% of the length of the reference sequence. When a position in the candidate sequence is occupied by the same amino acid (or nucleic acid) residue as the corresponding position in the reference sequence, then the molecules are identical at that position.

As used herein, the term "tumor-associated protease" or "tumor enzyme" refers to an enzyme, e.g., a protease, that is present at an increased level in a cancer, e.g., a solid tumor cancer. In some embodiments, the tumor-associated protease may cleave a cleavable linker.

As used herein, the term "blocking peptide" refers to a peptide that can bind to a SIRP-α variant and block or "mask" the CD47-binding portion of the SIRP-α variant. In a SIRP-α variant construct, the blocking peptide may be attached to a SIRP-α variant by way of a linker that is optionally cleavable, and optionally one or more spacers. The blocking peptide may be coupled via non-covalent bonds to the SIRP-α variant and cleaved at a diseased site or diseased cell. In some embodiments, the blocking peptide may bind to a wild-type SIRP-α at the diseased site or diseased cell. A blocking peptide can be used to reduce or minimize binding of the SIRP-α variant with wild-type CD47 under normal physiological conditions or at a non-diseased site. In some embodiments, the blocking peptide has higher binding affinity to a wild-type SIRP-α than a SIRP-α variant. The blocking peptide may dissociate from the SIRP-α variant to bind to a wild-type SIRP-α at, for e.g., a diseased site or under non-physiological conditions. An example of a blocking peptide

is a CD47-based blocking peptide, which is a peptide derived from CD47 or a fragment thereof. In some embodiments, a CD47-based blocking peptide is the extracellular, SIRP-α binding portion of CD47 (i.e., the IgSF domain of CD47). In some embodiments, a CD47-based blocking peptide includes one or more amino acid substitutions, additions, and/or deletions relative to the wild-type CD47.

As used herein, the term "cleavable linker" refers to a linker between two portions of a SIRP-α variant construct. In some embodiments, a cleavable linker may covalently attach a blocking peptide to a SIRP-α variant to block binding of the SIRP-α variant to CD47 under physiological conditions. In some embodiments, a cleavable linker may be installed within a blocking peptide, which may be non-covalently associated with the SIRP-α variant to block binding of the SIRP-α variant to CD47 under physiological conditions. A cleavable linker may be cleaved under certain conditions. If the cleavable linker is within a blocking peptide, cleavage of the linker would inactivate the blocking peptide. The cleavable linker contains a moiety that acts to cleave or induce cleavage of the linker under conditions characteristic of a diseased site, such as a cancer site, e.g., inside a solid tumor. The cleavable linker is stable under healthy physiological conditions (e.g., neutral pH and adequate oxygen concentration). The moiety may be a pH-sensitive chemical functional group (e.g., acetals, ketals, thiomaleamates, hydrazones, disulfide bonds) capable of being hydrolyzed under acidic pH. The moiety may also be a hypoxia-sensitive chemical functional group (e.g., quinones, N-oxides, and heteroaromatic nitro groups) or amino acid capable of being reduced under hypoxic condition. The moiety in the cleavable linker may also be a protein substrate capable of being recognized and cleaved by a tumor-associated protease, enzyme, or peptidase.

As used herein, the term "spacer" refers to a covalent or non-covalent linkage between two portions of a SIRP-α variant construct, such as the linker (e.g., cleavable linker) and the SIRP-α variant, or the antibody-binding peptide and the SIRP-α variant. Preferably, the spacer is a covalent linkage. A spacer can be a simple chemical bond, e.g., an amide bond, or an amino acid sequence (e.g., a 3-200 amino acid sequence). An amino acid spacer is part of the primary sequence of a polypeptide (e.g., joined to the spaced polypeptides or polypeptide domains via the polypeptide backbone). A spacer provides space and/or flexibility between the two portions. A spacer is stable under physiological conditions (e.g., neutral pH and adequate oxygen concentration) as well as under conditions characteristic of a diseased site, e.g., acidic pH and hypoxia. A spacer is stable at a diseased site, such as a cancer site, e.g., inside a tumor. Descriptions of spacers are provided in detail further herein.

As used herein, the term "antibody" refers to intact antibodies, antibody fragments, provided that they exhibit the desired biological activity, monoclonal antibodies, polyclonal antibodies, monospecific antibodies, and multispecific antibodies (e.g., bispecific antibodies) formed from at least two intact antibodies. Preferably, the antibody is specific to a diseased cell, e.g., a tumor cell. For example, the antibody may specifically bind to a cell surface protein on a diseased cell, e.g., a tumor cell.

As used herein, the term "albumin-binding peptide" refers to an amino acid sequence of 12 to 16 amino acids that has affinity for and functions to bind serum albumin. An albumin-binding peptide can be of different origins, e.g., human, mouse, or rat. In some embodiments of the present invention, a SIRP-α variant construct may include an albumin-binding peptide that is fused to the C-terminus of the SIRP-α variant to increase the serum half-life of the SIRP-α variant. An albumin-binding peptide can be fused, either directly or through a spacer, to the SIRP-α variant.

As used herein, the term "human serum albumin (HSA)" refers to the albumin protein present in human blood plasma. Human serum albumin is the most abundant protein in the blood. It constitutes about half of the blood serum protein. In some embodiments, a human serum albumin has the sequence of amino acids 25-609 (SEQ ID NO: 67) of UniProt ID NO: P02768. In some embodiments, a human serum albumin further contains C34S relative to the sequence of SEQ ID NO: 67.

As used herein, the term "Fc domain monomer" refers to a polypeptide chain that includes second and third antibody constant domains ($C_H2$ and $C_H3$). In some embodiment, the Fc domain monomer also includes a hinge domain. The Fc domain monomer can be any immunoglobulin antibody isotype, including IgG, IgE, IgM, IgA, or IgD. Additionally, the Fc domain monomer can be an IgG subtype (e.g., IgG1, IgG2a, IgG2b, IgG3, or IgG4). An Fc domain monomer does not include any portion of an immunoglobulin that is capable of acting as an antigen-recognition region, e.g., a variable domain or a complementarity determining region (CDR). Fc domain monomers can include as many as ten changes from a wild-type Fc domain monomer sequence (e.g., 1-10, 1-8, 1-6, 1-4 amino acid substitutions, additions, or deletions) that alter the interaction between an Fc domain and an Fc receptor. Examples of suitable changes are known in the art.

As used herein, the term "Fc domain" refers to a dimer of two Fc domain monomers. In the wild-type Fc domain, the two Fc domain monomers dimerize by the interaction between the two $C_H3$ antibody constant domains, as well as one or more disulfide bonds that form between the hinge domains of the two dimerizing Fc domain monomers. In some embodiments, an Fc domain may be mutated to lack effector functions, typical of a "dead Fc domain." In certain embodiments, each of the Fc domain monomers in an Fc domain includes amino acid substitutions in the $C_H2$ antibody constant domain to reduce the interaction or binding between the Fc domain and an Fcγ receptor.

As used herein, the term "affinity" or "binding affinity" refers to the strength of the binding interaction between two molecules. Generally, binding affinity refers to the strength of the sum total of non-covalent interactions between a

molecule and its binding partner, such as a SIRP-α variant and CD47. Unless indicated otherwise, binding affinity refers to intrinsic binding affinity, which reflects a 1:1 interaction between members of a binding pair. The binding affinity between two molecules is commonly described by the dissociation constant ($K_D$) or the affinity constant ($K_A$). Two molecules that have low binding affinity for each other generally bind slowly, tend to dissociate easily, and exhibit a large $K_D$. Two molecules that have high affinity for each other generally bind readily, tend to remain bound longer, and exhibit a small $K_D$. The $K_D$ of two interacting molecules may be determined using methods and techniques well known in the art, e.g., surface plasmon resonance. $K_D$ is calculated as the ratio of $k_{off}/k_{on}$.

As used herein, the term "host cell" refers to a vehicle that includes the necessary cellular components, e.g., organelles, needed to express proteins from their corresponding nucleic acids. The nucleic acids are typically included in nucleic acid vectors that can be introduced into the host cell by conventional techniques known in the art (e.g., transformation, transfection, electroporation, calcium phosphate precipitation, direct microinjection, etc.). A host cell may be a prokaryotic cell, e.g., a bacterial cell, or a eukaryotic cell, e.g., a mammalian cell (e.g., a CHO cell). As described herein, a host cell is used to express one or more SIRP-α variant constructs.

As used herein, the term "pharmaceutical composition" refers to a medicinal or pharmaceutical formulation that contains an active ingredient as well as excipients and diluents to enable the active ingredient suitable for the method of administration. The pharmaceutical composition of the present invention includes pharmaceutically acceptable components that are compatible with the SIRP-α variant construct. The pharmaceutical composition may be in tablet or capsule form for oral administration or in aqueous form for intravenous or subcutaneous administration.

As used herein, the term "disease associated with SIRP-α and/or CD47 activity" refers to any disease or disorder that is caused by and/or related to SIRP-α and/or CD47 activity. For example, any disease or disorder that is caused by and/or related to the increase and/or decrease of SIRP-α and/or CD47 activity. Examples of diseases associated with SIRP-α and/or CD47 activity include, but are not limited to, cancers and immunological diseases (e.g., autoimmune diseases and inflammatory diseases).

As used herein, the term "therapeutically effective amount" refers an amount of a SIRP-α variant construct of the invention or a pharmaceutical composition containing a SIRP-α variant construct of the invention effective in achieving the desired therapeutic effect in treating a patient having a disease, such as a cancer, e.g., solid tumor or hematological cancer. In particular, the therapeutic effective amount of the SIRP-α variant construct avoids adverse side effects.

As used herein, the term "optimized affinity" or "optimized binding affinity" refers to an optimized strength of the binding interaction between a SIRP-α variant and CD47. In some embodiments, the SIRP-α variant construct binds primarily or with higher affinity to CD47 on cells at a diseased site (i.e., cancer cells) and does not substantially bind or binds with lower affinity to CD47 on cells at a non-diseased site (i.e., non-cancer cells). The binding affinity between the SIRP-α variant and CD47 is optimized such that the interaction does not cause clinically relevant toxicity. In some embodiments, in order to achieve an optimized binding affinity between the SIRP-α variant and CD47, the SIRP-α variant may be developed to have a lower binding affinity to CD47 than which is maximally achievable.

As used herein, the term "immunogenicity" refers to the property of a protein (e.g., a therapeutic protein) which causes an immune response in the host as though it is a foreign antigen. The immunogenicity of a protein can be assayed in vitro in a variety of different ways, in particular through in vitro T-cell proliferation assays (see, e.g., Jawa et al., Clinical Immunology 149:534-555, 2013), some of which are commercially available (see, e.g., immunogenicity assay services offered by Proimmune).

As used herein, the term "minimal immunogenicity" refers to an immunogenicity of a protein (e.g., a therapeutic protein) that has been modified, i.e., through amino acid substitutions, to be lower (e.g., at least 10%, 25%, 50%, or 100% lower) than what could have been before the amino acid substitutions are introduced. A protein (e.g., a therapeutic protein) is modified to have minimal immunogenicity means it causes no or very little host immune response even though it is a foreign antigen.

As used herein, the term "optimized pharmacokinetics" refers to that the parameters that are generally associated with the pharmacokinetics of a protein are improved and modified to produce an optimized protein for in vitro and/or in vivo use. Parameters that are associated with the pharmacokinetics of a protein are well-known to a skilled artisan, including, for examples, $K_D$, valency, and half-life. In the present invention, the pharmacokinetics of a SIRP-α variant construct of the invention are optimized for its interaction with CD47 for use in a therapeutic context.

## DESCRIPTION OF THE DRAWINGS

FIG. 1 shows a portion of the co-crystalized structure of CD47:SIRP-α (PDB: 4KJY, 4CMM), the N-terminus of CD47 exists as a pyro-glutamate and makes hydrogen bonding interactions with Thr66 of a SIRP-α variant or Leu66 of a wild-type SIRP-α.

FIG. 2 shows computation models of the interaction site between CD47 having T102Q and a wild-type SIRP-α

having A27 (left) and the interaction site between CD47 having T102Q and a SIRP-α variant having I27.

FIG. 3 shows an SDS-PAGE of SIRP-α variant constructs (SEQ ID NOs: 48-56).

FIG. 4A shows an SDS-PAGE of SIRP-α variant construct (SEQ ID NO: 54) after in vitro cleavage with uPA and matriptase.

FIG. 4B shows an SDS-PAGE of SIRP-α variant construct (SEQ ID NO: 54) after in vitro cleavage with different amounts of matriptase.

FIG. 4C shows an SDS-PAGE of various SIRP-α variant constructs (SEQ ID NOs: 57-63) after in vitro cleavage with matriptase.

FIG. 5A shows a bar graph illustrating the different binding affinities of various SIRP-α variant constructs (SEQ ID NOs: 48-55) to CD47 before and after in vitro cleavage with matriptase.

FIG. 5B shows a bar graph illustrating the different binding affinities of various SIRP-α variant constructs (SEQ ID NOs: 52-63) and the SIRP-α variant (SEQ ID NO: 31) to CD47 before and after in vitro cleavage with matriptase.

FIG. 6 shows a sensorgram demonstrating that a SIRP-α variant construct (SEQ ID NO: 66) can bind Cetuximab and CD47 simultaneously.

FIG. 7A shows a scheme of the quaternary complex containing EGFR, Cetuximab, a SIRP-α variant construct (SEQ ID NO: 66), and CD47.

FIG. 7B shows a sensorgram demonstrating the formation of the quaternary complex shown in FIG. 7A.

FIG. 7C is an image of the sensorgram shown in FIG. 7B.

FIG. 8 is a scatter plot showing phagocytosis induced by the SIRP-α variant construct (SEQ ID NO: 66) and the SIRP-α variant (SEQ ID NO: 31).

## DETAILED DESCRIPTION OF THE INVENTION

The invention features signal-regulatory protein α(SIRP-α) variant constructs having preferential activity at a diseased site (e.g., at the site of a tumor than at a non-diseased site). In certain embodiments, the SIRP-α variant constructs have higher binding affinity to CD47 on diseased cells (e.g., tumor cells), cells. In some embodiments, the SIRP-α variants may contain one or more amino acid substitutions. In some embodiments, the amino acids may be substituted with histidine residues. In some embodiments, the amino acids may be substituted with other non-histidine amino acid residues. In some embodiments, the SIRP-α variant constructs bind with higher affinity to CD47 on diseased cells or at a diseased site than on non-diseased cells and under conditions characteristic of a diseased site, such as a cancer site, e.g., at the site of or inside a tumor. In some embodiments, the SIRP-α variant constructs bind with higher affinity to CD47 under acidic pH (e.g., less than around pH 7) and/or under hypoxic condition than under physiological conditions. In some embodiments, the SIRP-α variant constructs include a SIRP-α variant and a blocking peptide; the SIRP-α variant is prevented from binding to CD47 by the blocking peptide unless under conditions characteristic of a diseased site. In some embodiments, the SIRP-α variants are fused to an Fc domain monomer, a human serum albumin (HSA), an albumin-binding peptide, or a polymer (e.g., a polyethylene glycol (PEG) polymer). In some embodiments, the SIRP-α variant constructs have their immunogenicity, affinity, and/or pharmacokinetics optimized for use in a therapeutic context. In some embodiments, the SIRP-α variant constructs are preferentially targeted to diseased sites, e.g., a tumor, by way of a targeting moiety, e.g., a target-specific antibody. The invention features methods and pharmaceutical compositions containing SIRP-α variant constructs to treat various diseases, such as cancer, preferably solid tumor or hematological cancer, as well as methods of killing cancer cells and methods of manufacturing SIRP-α variant constructs and pharmaceutical compositions containing such SIRP-α variant constructs.

In some embodiments, a SIRP-α variant construct includes a SIRP-α variant attached to a blocking peptide. In some embodiments, the preferential binding of the SIRP-α variant in the SIRP-α variant construct to CD47 on diseased cells or diseased sites may be obtained by attaching the block peptide to the SIRP-α variant by use of a cleavable linker, which is cleaved at the diseased cells or diseased sites. In some embodiments, the preferential binding of the SIRP-α variant in the SIRP-α variant construct to CD47 on diseased cells or diseased sites may be obtained by attaching the block peptide to the SIRP-α variant, wherein the blocking peptide can be detatched or simply dissociated from the SIRP-α variant at the diseased cells or diseased sites.

### I. SIRP-α variants

There exist at least ten natural variants of wild-type human SIRP-α. The amino acid sequences of the D1 domains of the ten wild-type human SIRP-α variants are shown in SEQ ID NOs: 3-12 (see Table 1). In some embodiments, the SIRP-α variant has at least 80% (e.g., at least 85%, 87%, 90%, 91%, 92%, 93%, 94%, 95%, 96%, 97%, 98%, or 99%.) sequence identity to a sequence of any one of SEQ ID NOs: 3-12. Table 2 lists possible amino acid substitutions in each D1 domain variant (SEQ ID NOs: 13-23). In some embodiments, the SIRP-α variant binds with an optimized binding affinity to CD47. In some embodiments, the SIRP-α variant construct including a SIRP-α variant binds primarily

or with higher affinity to CD47 on cancer cells and does not substantially bind or binds with lower affinity to CD47 on non-cancer cells. In some embodiments, the binding affinity between the SIRP-α variant construct and CD47 is optimized such that the interaction does not cause clinically relevant toxicity. In some embodiments, the SIRP-α variant construct has minimal immunogenicity. In some embodiments, the SIRP-α variant has the same amino acids as that of the SIRP-α polypeptide in a biological sample of the subject, except for the amino acids changes introduced to increase affinity of the SIRP-α variant. Techniques and methods for generating SIRP-α variants and determining their binding affinities to CD47 are described in detail further herein.

Table 2 lists specific amino acid substitutions in a SIRP-α variant, relative to each D1 domain variant sequence. A SIRP-α variant may include one or more (e.g., one, two, three, four, five, six, seven, eight, nine, ten) of the substitutions listed in Table 2. In some embodiments, a SIRP-α variant includes at most ten amino acid substitutions relative to a wild-type D1 domain. In some embodiments, a SIRP-α variant includes at most seven amino acid substitutions relative to a wild-type D1 domain.

In some embodiments, a SIRP-α variant is a chimeric SIRP-α variant that includes a portion of two or more wild-type D1 domain variants (e.g., a portion of one wild-type D1 domain variant and a portion of another wild-type D1 domain variant). In some embodiments, a chimeric SIRP-α variant includes at least two portions (e.g., three, four, five, etc.) of wild-type D1 domain variants, wherein each of the portions is from a different wild-type D1 domain variant. In some embodiments, a chimeric SIRP-α variant further includes one or more amino acid substitutions listed in Table 2. In some embodiments, the SIRP-α variant has at least 80% (e.g., at least 85%, 87%, 90%, 91%, 92%, 93%, 94%, 95%, 96%, 97%, 98%, or 99%.) sequence identity to a sequence of any one of SEQ ID NOs: 24-34 in Table 3.

Table 1. Sequences of wild-type SIRP-α D1 domains

| | |
|---|---|
| Wild-type D1 domain variant 1 (SEQ ID NO: 3) | EEELQVIQPDKSVLVAAGETATLRCTATSLIPVGPIQWFRGAGPGRELIYNQKEGHFPRV TTVSDLTKRNNMDFSIRIGNITPADAGTYYCVKFRKGSPDDVEFKSGAGTELSVRAKPS |
| Wild-type D1 domain variant 2 (SEQ ID NO: 4) | EEELQVIQPDKSVSVAAGESAILHCTVTSLIPVGPIQWFRGAGPARELIYNQKEGHFPRV TTVSESTKRENMDFSISISNITPADAGTYYCVKFRKGSPDTEFKSGAGTELSVRAKPS |
| Wild-type D1 domain variant 3 (SEQ ID NO: 5) | EEELQVIQPDKSVSVAAGESAILLCTVTSLIPVGPIQWFRGAGPARELIYNQKEGHFPRV TTVSESTKRENMDFSISISNITPADAGTYYCVKFRKGSPDTEFKSGAGTELSVRAKPS |
| Wild-type D1 domain variant 4 (SEQ ID NO: 6) | EEGLQVIQPDKSVSVAAGESAILHCTATSLIPVGPIQWFRGAGPGRELIYNQKEGHFPRV TTVSDLTKRNNMDFSIRIGNITPADAGTYYCVKFRKGSPDDVEFKSGAGTELSVRAKPS |
| Wild-type D1 domain variant 5 (SEQ ID NO: 7) | EEELQVIQPDKFVLVAAGETATLRCTATSLIPVGPIQWFRGAGPGRELIYNQKEGHFPRV TTVSDLTKRNNMDFSIRIGNITPADAGTYYCVKFRKGSPDDVEFKSGAGTELSVRAKPS |
| Wild-type D1 domain variant 6 (SEQ ID NO: 8) | EEELQVIQPDKSVLVAAGETATLRCTATSLIPVGPIQWFRGAGPGRELIYNQKEGHFPRV TTVSDLTKRNNMDFPIRIGNITPADAGTYYCVKFRKGSPDDVEFKSGAGTELSVRAKPS |
| Wild-type D1 domain variant 7 (SEQ ID NO: 9) | EEELQVIQPDKSVSVAAGESAILHCTVTSLIPVGPIQWFRGAGPARELIYNQKEGHFPRV TTVSESTKRENMDFSISISNITPADAGTYYCVKFRKGSPDTEFKSGAGTELSVRGKPS |
| Wild-type D1 domain variant 8 (SEQ ID NO: 10) | EEELQVIQPDKSVLVAAGETATLRCTATSLIPVGPIQWFRGAGPARELIYNQKEGHFPRV TTVSESTKRENMDFSISISNITPADAGTYYCVKFRKGSPDTEFKSGAGTELSVRAKPS |
| Wild-type D1 domain variant 9 (SEQ ID NO: 11) | EEELQVIQPDKSVLVAAGETATLRCTATSLIPVGPIQWFRGAGPGRELIYNQKEGHFPRV TTVSDLTKRNNMDFSIRISNITPADAGTYYCVKFRKGSPDDVEFKSGAGTELSVRAKPS |
| Wild-type D1 domain variant 10 (SEQ ID NO: 12) | EEELQVIQPDKSVSVAAGESAILHCTVTSLIPVGPIQWFRGAGPARELIYNQKEGHFPRV TTVSESTKRENMDFSISISNITPADAGTYYCVKFRKGSPDTEFKSGAGTELSVRAKPS |

Table 2. Amino acid substitutions in a SIRP-α variant, relative to each D1 domain variant

| | |
|---|---|
| D1 domain v1 (SEQ ID NO: 13) | EEEX$_1$QX$_2$IQPDKSVLVAAGETX$_3$TLRCTX$_4$TSLX$_5$PVGPIQWFRGAGPGRX$_6$LIYNQX$_7$ X$_8$GX$_9$FPRVTTVSDX$_{10}$TX$_{11}$RNNMDFSIRIGX$_{12}$ITX$_{13}$ADAGTYYCX$_{14}$KX$_{15}$RKGSPDDV EX$_{16}$KSGAGTELSVRAKPS |
| Amino acid substitutions relative to SEQ ID NO: 13 | X$_1$=L, I, V; X$_2$=V, L, I; X$_3$=A, V; X$_4$=A, I, L; X$_5$=I, T, S, F; X$_6$=E, V, L; X$_7$=K, R; X$_8$=E, Q; X$_9$=H, P, R; X$_{10}$=L, T, G; X$_{11}$=K, R; X$_{12}$=N, A, C, D, E, F, G, H, I, K, L, M, P, Q, R, S, T, V, W, Y; X$_{13}$=P, A, C, D, E, F, G, H, I, K, L, M, N, Q, R, S, T, V, W, Y; X$_{14}$=V, I; X$_{15}$=F, L, V; X$_{16}$=F, V |
| D1 domain v2 (SEQ ID NO: 14) | EEEX$_1$QX$_2$IQPDKSVSVAAGESX$_3$ILHCTX$_4$TSLX$_5$PVGPIQWFRGAGPARX$_6$LIYNQX$_7$ X$_8$GX$_9$FPRVTTVSEX$_{10}$TX$_{11}$RENMDFSISISX$_{12}$ITX$_{13}$ADAGTYYCX$_{14}$KX$_{15}$RKGSPDTE X$_{16}$KSGAGTELSVRAKPS |
| Amino acid substitutions relative to SEQ ID NO: 14 | X$_1$=L, I, V; X$_2$=V, L, I; X$_3$=A, V; X$_4$=V, I, L; X$_5$=I, T, S, F; X$_6$=E, V, L; X$_7$=K, R; X$_8$=E, Q; X$_9$=H, P, R; X$_{10}$=S, T, G; X$_{11}$=K, R; X$_{12}$=N, A, C, D, E, F, G, H, I, K, L, M, P, Q, R, S, T, V, W, Y; X$_{13}$=P, A, C, D, E, F, G, H, I, K, L, M, N, Q, R, S, T, V, W, Y; X$_{14}$=V, I; X$_{15}$=F, L, V; X$_{16}$=F, V |
| D1 domain v3 (SEQ ID NO: 15) | EEEX$_1$QX$_2$IQPDKSVSVAAGESX$_3$ILLCTX4TSLX$_5$PVGPIQWFRGAGPARX$_6$LIYNQX$_7$ X$_8$GX$_9$FPRVTTVSEX$_{10}$TX$_{11}$RENMDFSISISX$_{12}$ITX$_{13}$ADAGTYYCX$_{14}$KX$_{15}$RKGSPDTE X$_{16}$KSGAGTELSVRAKPS |
| Amino acid substitutions relative to SEQ ID NO: 15 | X$_1$=L, I, V; X$_2$=V, L, I; X$_3$=A, V; X$_4$=V, I, L; X$_5$=I, T, S, F; X$_6$=E, V, L; X$_7$=K, R; X$_8$=E, Q; X$_9$=H, P, R; X$_{10}$=S, T, G; X$_{11}$=K, R; X$_{12}$=N, A, C, D, E, F, G, H, I, K, L, M, P, Q, R, S, T, V, W, Y; X$_{13}$=P, A, C, D, E, F, G, H, I, K, L, M, N, Q, R, S, T, V, W, Y; X$_{14}$=V, I; X$_{15}$=F, L, V; X$_{16}$=F, V |
| D1 domain v4 (SEQ ID NO: 16) | EEGX$_1$QX$_2$IQPDKSVSVAAGESX$_3$ILHCTX$_4$TSLX$_5$PVGPIQWFRGAGPGRX$_6$LIYNQX$_7$ X$_8$GX$_9$FPRVTTVSDX$_{10}$TX$_{11}$RNNMDFSIRIGX$_{12}$ITX$_{13}$ADAGTYYCX$_{14}$KX$_{15}$RKGSPDDV EX$_{16}$KSGAGTELSVRAKPS |
| Amino acid substitutions relative to SEQ ID NO: 16 | X$_1$=L, I, V; X$_2$=V, L, I; X$_3$=A, V; X$_4$=A, I, L; X$_5$=I, T, S, F; X$_6$=E, V, L; X$_7$=K, R; X$_8$=E, Q; X$_9$=H, P, R; X$_{10}$=L, T, G; X$_{11}$=K, R; X$_{12}$=N, A, C, D, E, F, G, H, I, K, L, M, P, Q, R, S, T, V, W, Y; X$_{13}$=P, A, C, D, E, F, G, H, I, K, L, M, N, Q, R, S, T, V, W, Y; X$_{14}$=V, I; X$_{15}$=F, L, V; X$_{16}$=F, V |
| D1 domain v5 (SEQ ID NO: 17) | EEEX$_1$QX$_2$IQPDKFVLVAAGETX$_3$TLRCTX$_4$TSLX$_5$PVGPIQWFRGAGPGRX$_6$LIYNQX$_7$ X$_8$GX$_9$FPRVTTVSDX$_{10}$TX$_{11}$RNNMDFSIRIGX$_{12}$ITX$_{13}$ADAGTYYCX$_{14}$KX$_{15}$RKGSPDDV EX$_{16}$KSGAGTELSVRAKPS |
| Amino acid substitutions relative to SEQ ID NO: 17 | X$_1$=L, I, V; X$_2$=V, L, I; X$_3$=A, V; X$_4$=A, I, L; X$_5$=I, T, S, F; X$_6$=E, V, L; X$_7$=K, R; X$_8$=E, Q; X$_9$=H, P, R; X$_{10}$=L, T, G; X$_{11}$=K, R; X$_{12}$=N, A, C, D, E, F, G, H, I, K, L, M, P, Q, R, S, T, V, W, Y; X$_{13}$=P, A, C, D, E, F, G, H, I, K, L, M, N, Q, R, S, T, V, W, Y; X$_{14}$=V, I; X$_{15}$=F, L, V; X$_{16}$=F, V |
| D1 domain v6 (SEQ ID NO: 18) | EEEX$_1$QX$_2$IQPDKSVLVAAGETX$_3$TLRCTX$_4$TSLX$_5$PVGPIQWFRGAGPGRX$_6$LIYNQX$_7$ X$_8$GX$_9$FPRVTTVSDX$_{10}$TX$_{11}$RNNMDFPIRIGX$_{12}$ITX$_{13}$ADAGTYYCX$_{14}$KX$_{15}$RKGSPDDV EX$_{16}$KSGAGTELSVRAKPS |
| Amino acid substitutions relative to SEQ ID NO: 18 | X$_1$=L, I, V; X$_2$=V, L, I; X$_3$=A, V; X$_4$=A, I, L; X$_5$=I, T, S, F; X$_6$=E, V, L; X$_7$=K, R; X$_8$=E, Q; X$_9$=H, P, R; X$_{10}$=L, T, G; X$_{11}$=K, R; X$_{12}$=N, A, C, D, E, F, G, H, I, K, L, M, P, Q, R, S, T, V, W, Y; X$_{13}$=P, A, C, D, E, F, G, H, I, K, L, M, N, Q, R, S, T, V, W, Y; X$_{14}$=V, I; X$_{15}$=F, L, V; X$_{16}$=F, V |

(continued)

| | |
|---|---|
| D1 domain v7 (SEQ ID NO: 19) | EEE$X_1$Q$X_2$IQPDKSVSVAAGES$X_3$ILHCT$X_4$TSL$X_5$PVGPIQWFRGAGPAR$X_6$LIYNQ$X_7$ $X_8$G$X_9$FPRVTTVSE$X_{10}$T$X_{11}$RENMDFSISIS$X_{12}$IT$X_{13}$ADAGTYYC$X_{14}$K$X_{15}$RKGSPDTE $X_{16}$KSGAGTELSVRGKPS |
| Amino acid substitutions relative to SEQ ID NO: 19 | $X_1$=L, I, V; $X_2$=V, L, I; $X_3$=A, V; $X_4$=V, I, L; $X_5$=I, T, S, F; $X_6$=E, V, L; $X_7$=K, R; $X_8$=E, Q; $X_9$=H, P, R; $X_{10}$=S, T, G; $X_{11}$=K, R; $X_{12}$=N, A, C, D, E, F, G, H, I, K, L, M, P, Q, R, S, T, V, W, Y; $X_{13}$=P, A, C, D, E, F, G, H, I, K, L, M, N, Q, R, S, T, V, W, Y; $X_{14}$=V, I; $X_{15}$=F, L, V; $X_{16}$=F, V |
| D1 domain v8 (SEQ ID NO: 20) | EEE$X_1$Q$X_2$IQPDKSVLVAAGET$X_3$TLRCT$X_4$TSL$X_5$PVGPIQWFRGAGPAR$X_6$LIYNQ$X_7$ $X_8$G$X_9$FPRVTTVSE$X_{10}$T$X_{11}$RENMDFSISIS$X_{12}$IT$X_{13}$ADAGTYYC$X_{14}$K$X_{15}$RKGSPDTE $X_{16}$KSGAGTELSVRAKPS |
| Amino acid substitutions relative to SEQ ID NO: 20 | $X_1$=L, I, V; $X_2$=V, L, I; $X_3$=A, V; $X_4$=A, I, L; $X_5$=I, T, S, F; $X_6$=E, V, L; $X_7$=K, R; $X_8$=E, Q; $X_9$=H, P, R; $X_{10}$=S, T, G; $X_{11}$=K, R; $X_{12}$=N, A, C, D, E, F, G, H, I, K, L, M, P, Q, R, S, T, V, W, Y; $X_{13}$=P, A, C, D, E, F, G, H, I, K, L, M, N, Q, R, S, T, V, W, Y; $X_{14}$=V, I; $X_{15}$=F, L, V; $X_{16}$=F, V |
| D1 domain v9 (SEQ ID NO: 21) | EEE$X_1$Q$X_2$IQPDKSVLVAAGET$X_3$TLRCT$X_4$TSL$X_5$PVGPIQWFRGAGPGR$X_6$LIYNQ$X_7$ $X_8$G$X_9$FPRVTTVSD$X_{10}$T$X_{11}$RNNMDFSIRIS$X_{12}$IT$X_{13}$ADAGTYYC$X_{14}$K$X_{15}$RKGSPDDV E$X_{16}$KSGAGTELSVRAKPS |
| Amino acid substitutions relative to SEQ ID NO: 21 | $X_1$=L, I, V; $X_2$=V, L, I; $X_3$=A, V; $X_4$=A, I, L; $X_5$=I, T, S, F; $X_6$=E, V, L; $X_7$=K, R; $X_8$=E, Q; $X_9$=H, P, R; $X_{10}$=L, T, G; $X_{11}$=K, R; $X_{12}$=N, A, C, D, E, F, G, H, I, K, L, M, P, Q, R, S, T, V, W, Y; $X_{13}$=P, A, C, D, E, F, G, H, I, K, L, M, N, Q, R, S, T, V, W, Y; $X_{14}$=V, I; $X_{15}$=F, L, V; $X_{16}$=F, V |
| D1 domain v10 (SEQ ID NO: 22) | EEE$X_1$Q$X_2$IQPDKSVSVAAGES$X_3$ILHCT$X_4$TSL$X_5$PVGPIQWFRGAGPAR$X_6$LIYNQ$X_7$ $X_8$G$X_9$FPRVTTVSE$X_{10}$T$X_{11}$RENMDFSISIS$X_{12}$IT$X_{13}$ADAGTYYC$X_{14}$K$X_{15}$RKGSPDTE $X_{16}$KSGAGTELSVRAKPS |
| Amino acid substitutions relative to SEQ ID NO: 22 | $X_1$=L, I, V; $X_2$=V, L, I; $X_3$=A, V; $X_4$=V, I, L; $X_5$=I, T, S, F; $X_6$=E, V, L; $X_7$=K, R; $X_8$=E, Q; $X_9$=H, P, R; $X_{10}$=S, T, G; $X_{11}$=K, R; $X_{12}$=N, A, C, D, E, F, G, H, I, K, L, M, P, Q, R, S, T, V, W, Y; $X_{13}$=P, A, C, D, E, F, G, H, I, K, L, M, N, Q, R, S, T, V, W, Y; $X_{14}$=V, I; $X_{15}$=F, L, V; $X_{16}$=F, V |
| Pan D1 domain (SEQ ID NO: 23) | EE$X_1$$X_2$Q$X_3$IQPDK$X_4$V$X_5$VAAGE$X_6$$X_7$$X_8$L$X_9$CT$X_{10}$TSL$X_{11}$PVGPIQWFRGAGP$X_{12}$R$X_{13}$ LIYNQ$X_{14}$$X_{15}$G$X_{16}$FPRVTTVS$X_{17}$$X_{18}$T$X_{19}$R$X_{20}$NMDF$X_{21}$I$X_{22}$I$X_{23}$$X_{24}$IT$X_{25}$ADAGTYY C$X_{26}$K$X_{27}$RKGSPD$X_{28}$$X_{29}$E$X_{30}$KSGAGTELSVR$X_{31}$KPS |
| Amino acid substitutions relative to SEQ ID NO: 23 | $X_1$=E, G; $X_2$=L, I, V; $X_3$=V, L, I; $X_4$=S, F; $X_5$=L, S; $X_6$=S, T; $X_7$=A, V; $X_8$=I, T; $X_9$=H, R; $X_{10}$=A, V, I, L; $X_{11}$=I, T, S, F; $X_{12}$=A, G; $X_{13}$=E, V, L; $X_{14}$=K, R; $X_{15}$=E, Q; $X_{16}$=H, P, R; $X_{17}$=D, E; $X_{18}$=S, L, T, G; $X_{19}$=K, R; $X_{20}$=E, N; $X_{21}$=S, P; $X_{22}$=S, R; $X_{23}$=S, G; $X_{24}$=N, A, C, D, E, F, G, H, I, K, L, M, P, Q, R, S, T, V, W, Y; $X_{25}$=P, A, C, D, E, F, G, H, I, K, L, M, N, Q, R, S, T, V, W, Y; $X_{26}$=V, I; $X_{27}$=F, L, V; $X_{28}$=D or absent; X29=T, V; $X_{30}$=F, V; and $X_{31}$=A, G |

Table 3. SEQ ID NOs: 24-34

| SEQ ID NO | Sequence |
|---|---|
| 24 | EEELQVIQPDKSVSVAAGESAILHCTITSLIPVGPIQWFRGAGPARELIYNQREGHFPRVTTVSET TRRENMDFSISISNITPADAGTYYCVKFRKGSPDTEVKSGAGTELSVRAKPS |
| 25 | EEEVQVIQPDKSVSVAAGESAILHCTLTSLIPVGPIQWFRGAGPARVLIYNQRQGHFPRVTTVSEG TRRENMDFSISISNITPADAGTYYCIKFRKGSPDTEFKSGAGTELSVRAKPS |

(continued)

| SEQ ID NO | Sequence |
|---|---|
| 26 | EEEVQIIQPDKSVSVAAGESVILHCTITSLTPVGPIQWFRGAGPARLLIYNQREGPFPRVTTVSET TRRENMDFSISISNITPADAGTYYCVKLRKGSPDTEFKSGAGTELSVRAKPS |
| 27 | EEELQIIQPDKSVSVAAGESAILHCTITSLSPVGPIQWFRGAGPARVLIYNQRQGPFPRVTTVSEG TKRENMDFSISISNITPADAGTYYCIKLRKGSPDTEFKSGAGTELSVRAKPS |
| 28 | EEEIQVIQPDKSVSVAAGESVIIHCTVTSLFPVGPIQWFRGAGPARVLIYNQRQGRFPRVTTVSEG TKRENMDFSISISNITPADAGTYYCVKVRKGSPDTEVKSGAGTELSVRAKPS |
| 29 | EEEVQIIQPDKSVSVAAGESIILHCTVTSLFPVGPIQWFRGAGPARVLIYNQREGRFPRVTTVSEG TRRENMDFSISISNITPADAGTYYCIKLRKGSPDTEFKSGAGTELSVRAKPS |
| 30 | EEEVQLIQPDKSVSVAAGESAILHCTVTSLFPVGPIQWFRGAGPARVLIYNQREGPFPRVTTVSEG TKRENMDFSISISNITPADAGTYYCIKFRKGSPDTEVKSGAGTELSVRAKPS |
| 31 | EEELQIIQPDKSVLVAAGETATLRCTITSLFPVGPIQWFRGAGPGRVLIYNQRQGPFPRVTTVSDT TKRNNMDFSIRIGNITPADAGTYYCIKFRKGSPDDVEFKSGAGTELSVRAKPS |
| 32 | EEELQIIQPDKSVSVAAGESAILHCTITSLFPVGPIQWFRGAGPARLLIYNQRQGPFPRVTTVSET TKRENMDFSISISNITPADAGTYYCVKFRKGSPDTEFKSGAGTELSVRAKPS |
| 33 | EEEVQIIQPDKSVSVAAGESAILHCTITSLFPVGPIQWFRGAGPARVLIYNQKQGPFPRVTTISET TRRENMDFSISISNITPADAGTYYCIKFRKGSPDTEFKSGAGTELSVRAKPS |
| 34 | EEELQIIQPDKSVSVAAGESAILHCTITSLTPVGPIQWFRGAGPARVLIYNQRQGPFPRVTTVSEG TRRENMDFSISISNITPADAGTYYCIKFRKGSPDTEVKSGAGTELSVRAKPS |

Desirably, the SIRP-α variant constructs of the invention bind with higher affinity to CD47 under conditions characteristic of a diseased site, such as a cancer site, e.g., inside a tumor, than under physiological conditions (e.g., neutral pH and adequate oxygen concentration). Conditions characteristic of a diseased site, such as a cancer site, e.g., inside a tumor, are, e.g., acidic pH and hypoxia. In some embodiments, SIRP-α variant constructs of the invention may be engineered to preferentially bind to diseased cells over non-diseased cells. In particular, the disease cells may be cancer cells of a cancer disease, e.g., solid tumor or hematological cancer. Preferably, the SIRP-α variant constructs bind with higher affinity to CD47 under acidic pH (e.g., less than around pH 7) than under neutral pH, e.g., pH 7.4. Preferably, the SIRP-α variant constructs bind with higher affinity to CD47 under hypoxic condition than under a condition with adequate oxygen concentration. In some embodiments, a SIRP-α variant construct includes a SIRP-α variant attached to a blocking peptide. In some embodiments, the preferential binding of the SIRP-α variant in the SIRP-α variant construct to CD47 on diseased cells or diseased sites may be obtained by attaching the block peptide to the SIRP-α variant by use of a cleavable linker, which is cleaved at the diseased cells or diseased sites. In some embodiments, the preferential binding of the SIRP-α variant in the SIRP-α variant construct to CD47 on diseased cells or diseased sites may be obtained by attaching the block peptide to the SIRP-α variant, wherein the blocking peptide can be detatched or simply dissociated from the SIRP-α variant at the diseased cells or diseased sites.

In some embodiments, a SIRP-α variant construct includes a SIRP-α variant and a blocking peptide. In some embodiments, SIRP-α variant may be attached to a blocking peptide through a linker (e.g., a cleavable linker). The blocking peptide serves to block the CD47 binding site of the SIRP-α variant to prevent binding of SIRP-α variant to CD47 under physiological conditions (e.g., neutral pH and adequate oxygen concentration). The cleavable linker is a linker capable of being cleaved only under conditions characteristic of a diseased site (such as a cancer site, e.g., inside a tumor), such as acidic pH and hypoxia. In some embodiments, the cleavable linker is cleaved by a tumor-associated protease at a diseased site. In some embodiments, the linker is not cleaved and the blocking peptide simply dissociates from the SIRP-α variant at a diseased site such that the SIRP-α variant is free to bind to nearby CD47 on diseased cells, e.g., tumor cells. Therefore, only when the SIRP-α variant is at a diseased site would it be released from the blocking peptide and be free to bind to nearby CD47 on diseased cells, e.g., tumor cells. Blocking peptides and linkers (e.g., cleavable linkers) are described in detail further herein.

In some embodiments, a SIRP-α variant construct includes a SIRP-α variant and a targeting moiety. In some embodiments, a SIRP-α variant may be attached to a targeting moiety, such as an antibody, e.g., a tumor-specific antibody, or another protein or peptide, e.g., an antibody-binding peptide, that exhibit binding affinity to a diseased cell. After administration, the tumor-specific antibody or antibody-binding peptide serves as a targeting moiety to bring the SIRP-α variant to the diseased site, such as a cancer site, e.g., inside a solid tumor, where the SIRP-α can interact

specifically with CD47 on diseased cells. In some embodiments, a SIRP-α variant may be fused to a protein or peptide, e.g., an antibody-binding peptide, capable of binding to an antibody (e.g., tumor-specific antibody), i.e., binding to a constant or variable region of the antibody. SIRP-α variants capable of binding to one or more antibodies are described in detail further herein. In other embodiments, other SIRP-α variants, such as the ones described in International Publication No. WO2013109752, may be attached to a tumor-specific antibody or to a protein or peptide, e.g., an antibody-binding peptide, capable of binding to a tumor-specific antibody. In some embodiments, the SIRP-α variant may be attached to the antibody either *in vitro* (prior to administration to a human) or *in vivo* (after administration).

In some embodiments, a SIRP-α variant may further include a D2 and/or D3 domain of a wild-type human SIRP-α. In some embodiments, a SIRP-α variant may be attached to an Fc domain monomer, a human serum albumin (HSA), a serum-binding protein or peptide, or an organic molecule, e.g., a polymer (e.g., a polyethylene glycol (PEG)), in order to improve the pharmacokinetic properties of the SIRP-α variant, e.g., increase serum half-life. Fc domain monomers, HSA proteins, serum-binding proteins or peptides, and organic molecules such as a PEG that serve to increase the serum half-life of the SIRP-α variants of the invention are described in detail further herein. In some embodiments, a SIRP-α variant does not include the sequence of any one of SEQ ID NOs:3-12 and 24-34.

## II. Amino acid substitutions with histidine residues in SIRP-α variants

In some embodiments, in addition to the amino acid substitutions in a SIRP-α variant listed in Table 2, the SIRP-α variant may include one or more amino acid substitutions with histidine residues. The SIRP-α variant constructs including a SIRP-α variant bind with higher affinity to CD47 on diseased cells or at a diseased site than on non-diseased cells and under conditions characteristic of a diseased site (e.g., acidic pH, hypoxia) than under physiological conditions. Amino acid residues to be substituted with histidine residues may be identified using histidine scanning mutagenesis, protein crystal structures, and computational design and modeling methods. Techniques and methods that may be used to generate SIRP-α variants and ways to determine their binding affinities to CD47 on diseased and non-diseased cells are described in detail further herein. The histidine residue substitutions may be located at the interface of a SIRP-α variant and CD47 or may be at internal regions of a SIRP-α variant. Preferentially, histidine residue substitutions are located at the interface of a SIRP-α variant and CD47. Table 4 lists specific SIRP-α amino acids that may be substituted with histidine residues. The amino acid numbering in Table 4 is relative to the sequence of SEQ ID NO: 3; one or more amino acids at the corresponding positions in any one of the sequences of SEQ ID NOs: 4-12 may also be substituted with histidine residues. Contact residues are the amino acids located at the interface of a SIRP-α variant and CD47. Core residues are the internal amino acids not directly involved in the binding between a SIRP-α variant and CD47. The SIRP-α variants may include one or more (e.g., one, two, three, four, five, six, seven, eight, nine, ten, etc, or all) of the substitutions listed in Table 4. The SIRP-α variants may contain a maximum of 20 histidine substitutions.

Table 4. SIRP-α amino acid substitutions (amino acid numbering is relative to the sequence of SEQ ID NO: 3)

| | |
|---|---|
| Contact residues | S29H, L30H, I31H, P32H, V33H, G34H, P35H, Q52H, K53H, E54H, L66H, T67H, K68H, R69H, F74H, K93H, K96H, G97H, S98H, D100H |
| Core residues | L4H, V6H, A27H, I36H, F39H, E47H, L48H, I49H, Y50H, F57H, V60H, M72H, F74H, I76H, V92H, F94H, E103H |

## III. pH-dependent binding

Studies have shown that tumor cell mediated oncogenic metabolism generates a large amount of lactic acid and protons, leading to the reduction in the extracellular pH values to as low as 6 in tumor tissue (Icard et al., Biochim. Biophys. Acta. 1826:423-433, 2012). In some embodiments, the SIRP-α variant constructs including a SIRP-α variant are engineered to bind with high affinity to CD47 under acidic pH than under neutral pH (e.g., around pH 7.4). Thus, the SIRP-α variant constructs of the invention are engineered to selectively bind to CD47 on diseased cells (e.g., tumor cells) or on cells at a diseased site (e.g., cells in the tumor micro-environment supporting tumor growth), over CD47 on non-diseased cells.

In one embodiment, to engineer pH-dependent binding of a SIRP-α variant construct of the invention, histidine mutagenesis may be performed on the SIRP-α variant, especially on the region of SIRP-α that interacts with CD47. Crystal structures of a SIRP-α and CD47 complex (see, e.g., PDB ID No. 2JJS) and computer modeling may be used to visualize the three-dimensional binding site of SIRP-α and CD47. Computational design and modeling methods useful in designing a protein with pH-sensitive binding properties are known in the literature and described in, e.g., Strauch et al., Proc Natl Acad Sci 111:675-80, 2014. In some embodiments, computer modeling may be used to identify key contact residues at the interface of SIRP-α and CD47. Identified key contact residues may be substituted with histidine residues

using available protein design software (e.g., RosettaDesign), which can generate various protein designs that can be optimized, filtered, and ranked based on computed binding energy and shape complementarity. Therefore, energetically favorable histidine substitutions at certain amino acid positions may be identified using computational design methods. Computer modeling may be also be used to predict the change in the three-dimensional structure of SIRP-α. Histidine substitutions that generate a significant change in the three-dimensional structure of SIRP-α may be avoided.

Once energetically and structurally optimal amino acid substitutions are identified, the amino acids may be systematically substituted with histidine residues. In some embodiments, one or more (e.g., one, two, three, four, five, six, seven, eight, nine, ten, etc, with a maximum of 20) amino acids of SIRP-α may be substituted with histidine residues. In particular, amino acids located at the interface of SIRP-α and CD47, preferably, amino acids directly involved in the binding of SIRP-α to CD47, may be substituted with histidine residues. The SIRP-α variant may include one or more (e.g., one, two, three, four, five, six, seven, eight, nine, ten, etc, with a maximum of 20) histidine residue substitutions. In other embodiments, naturally occurring histidine residues of SIRP-α may be substituted with other amino acid residues. In yet other embodiments, one or more amino acids of SIRP-α may be substituted with non-histidine residues in order to affect the binding of naturally occurring or substituted histidine residues with CD47. For example, substituting amino acids surrounding a naturally occurring histidine residue with other amino acids may "bury" the naturally occurring histidine residue. In some embodiments, amino acids not directly involved in binding with CD47, i.e., internal amino acids (e.g., amino acids located at the core of SIRP-α) may also be substituted with histidine residues. Table 4 lists specific SIRP-α amino acids that may be substituted with histidine or non-histidine residues. Contact residues are the amino acids located at the interface of SIRP-α and CD47. Core residues are the internal amino acids not directly involved in the binding between SIRP-α and CD47. The SIRP-α variants may include one or more (e.g., one, two, three, four, five, six, seven, eight, nine, ten, etc, or all) of the substitutions listed in Table 4.

SIRP-α variants containing one or more (e.g., one, two, three, four, five, six, seven, eight, nine, ten, etc, with a maximum number of 20) histidine residue substitutions may be tested for their binding to CD47 under different pH conditions (e.g., at pH 5, 5.5, 6, 6.5, 7, 7.4, 8). In some embodiments, purified CD47 protein may be used to test binding. Various techniques known to those skilled in the art may be used to measure the affinity constant ($K_A$) or dissociation constant ($K_D$) of a SIRP-α variant/CD47 complex under different pH conditions (e.g., at pH 5, 5.5, 6, 6.5, 7, 7.4, 8). In a preferred embodiment, the binding affinity of a SIRP-α variant to a CD47 may be determined using surface plasmon resonance (e.g., Biacore3000™ surface plasmon resonance (SPR) system, Biacore, INC, Piscataway N.J.). In an exemplary embodiment, a SIRP-α variant with pH-dependent binding, which specifically binds a CD47 with higher affinity at pH 6 than at pH 7.4, exhibits a lower $K_D$ at pH 6 than at pH 7.4.

### IV. Hypoxia-dependent binding

Tumor hypoxia is the condition in which tumor cells have been deprived of oxygen. As a tumor grows, its blood supply is constantly redirect to the most fast growing parts of the tumor, leaving portions of the tumor with oxygen concentration significantly lower than in healthy tissues.

In some embodiments, a SIRP-α variant may be attached to a hypoxia-activated prodrug, which may act to increase the efficacy of a SIRP-alpha variant against the relevant diseased cells under specifically hypoxic conditions. Hypoxia-activated prodrugs are known in the literature, such as those described by Kling et al. (Nature Biotechnology, 30:381, 2012).

### V. Antibody binding

Another strategy to provide selective SIRP-α activity at a diseased site than at a non-diseased site is to attach the SIRP-α protein to a protein or peptide that can bind to a region of an antibody. Preferably, the antibody is specific to a diseased cell, e.g., a tumor cell. For example, the antibody may specifically bind to a cell surface protein on a diseased cell, e.g., a tumor cell. The SIRP-α protein may bind to the antibody reversibly or irreversibly.

*General antibody binding*

In some embodiments, to engineer a SIRP-α protein that can bind to different antibodies regardless of antibody specificity, the SIRP-α protein may be fused to a protein or peptide that recognizes the constant region of an antibody, e.g., the CH2 or CH3 constant domain of the Fc domain of an antibody. A SIRP-α protein is capable of binding CD47 and has at least 50% amino acid sequence identity to a sequence of a wild-type SIRP-α (e.g., variant 1 (SEQ ID NO: 1, shown below)) or to a sequence of a CD47-binding portion of a wild-type SIRP-α (e.g., a sequence of any one of SEQ ID NOs: 3-12 listed in Table 1).

SEQ ID NO: 1

```
  1 MEPAGPAPGR LGPLLCLLLA ASCAWSGVAG EEELQVIQPD KSVLVAAGET ATLRCTATSL
 61 IPVGPIQWFR GAGPGRELIY NQKEGHFPRV TTVSDLTKRN NMDFSIRIGN ITPADAGTYY
121 CVKFRKGSPD DVEFKSGAGT ELSVRAKPSA PVVSGPAARA TPQHTVSFTC ESHGFSPRDI
181 TLKWFKNGNE LSDFQTNVDP VGESVSYSIH STAKVVLTRE DVHSQVICEV AHVTLQGDPL
241 RGTANLSETI RVPPTLEVTQ QPVRAENQVN VTCQVRKFYP QRLQLTWLEN GNVSRTETAS
301 TVTENKDGTY NWMSWLLVNV SAHRDDVKLT CQVEHDGQPA VSKSHDLKVS AHPKEQGSNT
361 AAENTGSNER NIYIVVGVVC TLLVALLMAA LYLVRIRQKK AQGSTSSTRL HEPEKNAREI
421 TQDTNDITYA DLNLPKGKKP APQAAEPNNH TEYASIQTSP QPASEDTLTY ADLDMVHLNR
481 TPKQPAPKPE PSFSEYASVQ VPRK
```

A CD47-binding portion of a wild-type SIRP-α includes the D1 domain of a wild-type SIRP-α (e.g., a sequence of any one of SEQ ID NOs: 3-12 listed in Table 1). Proteins and peptides exhibiting general binding to the constant region of an antibody are known in the art. For example, the bacterial antibody-binding proteins, e.g., Proteins A, G, and L, bind to the constant regions of an antibody. Proteins A and G bind to the Fc domains, while Protein L binds to the constant region of the light chain. In an exemplary embodiment, Protein A, G, or L may be fused to the N- or C-terminus of a SIRP-α protein. Preferentially, in this embodiment, the fusion protein of Protein A, G, or L and the SIRP-α protein may be attached, i.e., through chemical conjugation, to an antibody prior to administration to prevent the fusion protein from binding to various other antibodies in serum. Protein A, G, or L may also be evolved and screened using conventional techniques in the field (i.e., directed evolution and display libraries) for higher binding affinity to the constant regions of an antibody. In some embodiments, a SIRP-α protein may be directly attached to an antibody using conventional genetic or chemical conjugation techniques in the art. In other embodiments, a SIRP-α protein may also be attached to an antibody by way of a spacer, which allows for additional structural and spatial flexibility of the protein. Various spacers are described in detail further herein. In some embodiments, the SIRP-α protein may bind, either directly or through an antibody-binding protein or peptide, to the antibody reversibly or irreversibly. Further, screening of modified antibodies which can be utilized in accordance with the embodiments of the invention described herein can be carried out as described in, e.g., US Patent Publication No. US 20100189651 .

Other proteins or peptides capable of binding to a constant region of an antibody and methods of screening for such proteins or peptides are described in US Patent Publication No. US20120283408.

*Specific antibody binding*

In some embodiments, to provide selective targeting of SIRP-α variants at a diseased site and to engineer a SIRP-α variant capable of binding to a specific antibody, e.g., a tumor-specific antibody, the SIRP-α variant construct may include a SIRP-α variant and an antibody-specific protein or peptide. The SIRP-α variant may be fused to an antibody-specific protein or peptide (e.g., an antibody-binding peptide). Preferably, the protein or peptide specifically binds to a tumor-specific antibody. In some embodiments, the fusion protein of the SIRP-α variant and the antibody-binding protein or peptide may be co-administered with the tumor-specific antibody in a combination therapy. In other embodiments, the fusion protein and the tumor-specific antibody may be administrated separately, i.e., within hours of each other, preferably, the antibody is administered first. In yet other embodiments, prior to administration, the fusion protein may be covalently attached to the tumor-specific antibody using genetic or chemical methods commonly known in the art.

Examples of antibody-binding peptides include a disease localization peptide (DLP) (SEQ ID NO: 64 or 65), a small peptide that can bind to the center of the fragment antigen-binding (Fab) region of Cetuximab (see, e.g., Donaldson et al., Proc Natl Acad Sci USA. 110: 17456-17461, 2013). Cetuximab is an anti-epidermal growth factor receptor (EGFR) IgG1 antibody. Antibody-binding peptides that can be fused to a SIRP-α variant also include, but are not limited to, peptides having at least 75% amino acid sequence identity to the sequence of the DLP (SEQ ID NO: 64 or 65) or a fragment thereof. In some embodiments, the antibody-binding peptide has the sequence of SEQ ID NO: 64.

In a recent study, SIRP-α has been shown to enhance in vitro phagocytosis of DLD-1 cells in combination with the antibody Cetuximab (Weiskopf et al., Science 341: 88-91, 2013). In some embodiments, a SIRP-α variant may be fused to a specific antibody-binding peptide, e.g., a DLP having the sequence of SEQ ID NO: 64. In these embodiments, the SIRP-α variant construct including a SIRP-α variant and a DLP may target its activity in Cetuximab-bound, EGFR expressing tumors. This in turn may further improve the delivery of the SIRP-α variant construct including a SIRP-α variant and DLP and Cetuximab to anti-EGFR responsive patients. An example of a SIRP-α variant construct including a SIRP-α variant and DLP is shown in SEQ ID NO: 66, in which single-underlined portion indicates the DLP and bold portion indicates the SIRP-α variant. The sequence of the SIRP-α variant (bold portion) in SEQ ID NO: 66 may be

replaced by a sequence of any SIRP-α variant described herein. Other antibody-binding peptides may also be fused to a SIRP-α variant. Such antibody-binding peptides include, but are not limited to, peptides that can specifically bind to antibodies such as cetuximab, pembrolizumab, nivolumab, pidilizumab, MEDI0680, MEDI6469, Ipilimumab, tremelimumab, urelumab, vantictumab, varlilumab, mogamalizumab, anti-CD20 antibody, anti-CD19 antibody, anti-CSl antibody, herceptin, trastuzumab, and/or pertuzumab.

SEQ ID NO: 66

CQFDLSTRRLKCGGGGSGGGGSGGGGSGGGGS**EEELQIIQPDKSVLVAAGETATLRCTITSLFPVGPIQWF**
**RGAGPGRVLIYNQRQGPFPRVTTVSDTTKRNNMDFSIRIGNITPADAGTYYCIKFRKGSPDDVEFKSGAGT**
**ELSVRAKPS**GGGGSGGGGSGGGGSGGGGSCQFDLSTRRLKC

In some embodiments, a SIRP-α variant construct including a SIRP-α variant and a DLP may be further combined with a CD47-based blocking peptide described herein to block the binding of the SIRP-α variant in the construct before the construct reaches the diseased site where the cleavable linker may be cleaved. In these embodiments, the therapeutic window can be expanded as the SIRP-α variant construct containing a SIRP-α variant, a CD47-based blocking peptide, and a DLP accumulates at the diseased site and is only active at the diseased site after linker cleavage induced by a protease (e.g., a tumor-specific protease) or other characteristics of the diseased site (e.g., acidic pH, hypoxia).

In some embodiments, proteins or peptides capable of binding to tumor-specific antibodies may be identified using techniques commonly used in the art, such as directed evolution and display libraries, e.g., a phage display library. Methods and techniques directed to identifying proteins and peptides capable of binding to tumor-specific antibodies are known in the art, such as those described by Donaldson et al. (Proc Natl Acad Sci 110:17456-61, 2013). In a phage display library, a potential antibody-specific protein or peptide is typically covalently linked to a bacteriophage coat protein. The linkage results from translation of a nucleic acid encoding the protein or peptide fused to the coat protein. Bacteriophage displaying the peptide can be grown and harvested using standard phage preparatory methods, e.g. PEG precipitation from growth media. These displaying phages can then be screened against other proteins, e.g., tumor-specific antibodies, in order to detect interaction between the displayed protein and the tumor-specific antibodies. Once the tumor-specific protein or peptide is identified, the nucleic acid encoding the selected tumor-specific protein or peptide can be isolated from cells infected with the selected phages or from the phage themselves, after amplification. Individual colonies or plaques can be picked and the nucleic acid can be isolated and sequenced. After identifying and isolating the antibody-specific protein or peptide, the protein or peptide may be fused to the N- or C-terminus of a SIRP-α variant. In some embodiments, a SIRP-α variant may be directly attached to a tumor-specific antibody using conventional genetic or chemical conjugation techniques in the art. In other embodiments, a SIRP-α variant may also be attached to a tumor-specific antibody by way of a spacer, which allows for additional structural and spatial flexibility of the protein. Various spacers are described in detail further herein. In some embodiments, the SIRP-α variant may bind, either directly or through an antibody-binding protein or peptide, to the antibody reversibly or irreversibly.

In other embodiments, a wild-type SIRP-α or the extracellular D1 domain of the wild-type SIRP-α (e.g., a sequence of any one of SEQ ID NOs: 3-12 listed in Table 1) may be attached to a tumor-specific antibody. Preferably, the D1 domain of SIRP-α is attached to the tumor-specific antibody. The tumor-specific antibody serves as a targeting moiety to bring the wild-type SIRP-α or the D1 domain to the diseased site, e.g., a cancer site, e.g., inside a solid tumor, where the wild-type SIRP-α or the D1 domain can interact with CD47 on diseased cells. In some embodiments, a wild-type SIRP-α or the extracellular D1 domain of the wild-type SIRP-α may be directly attached to a tumor-specific antibody using conventional genetic or chemical conjugation techniques in the art. In other embodiments, a wild-type SIRP-α or the extracellular D1 domain of the wild-type SIRP-α may also be attached to a tumor-specific antibody by way of a spacer, which allows for additional structural and spatial flexibility of the protein. Various spacers are described in detail further herein. In other embodiments, a wild-type SIRP-α or the extracellular D1 domain of the wild-type SIRP-α may be fused to the aforementioned protein or peptide capable of binding to a tumor-specific antibody. In yet other embodiments, other SIRP-α polypeptides, such as the ones described in International Publication No. WO2013109752, may be attached to a tumor-specific antibody or to a protein or peptide capable of binding to a tumor-specific antibody. In some embodiments, the wild-type SIRP-α or the D1 domain may bind, either directly or through an antibody-binding protein or peptide, to the antibody reversibly or irreversibly.

## VI. Blocking peptides

A blocking peptide may be attached a SIRP-α variant by way of a cleavable linker. In some embodiments, a blocking peptide may also be non-covalently attached to a SIRP-α variant. The blocking peptide acts to block the CD47

binding site of the SIRP-α variant such that the SIRP-α variant cannot bind to CD47 on the cell surface of non-diseased cells under physiological conditions (e.g., neutral pH and adequate oxygen concentration). Under abnormal conditions (i.e., an acidic and/or hypoxic environment or an environment with increased protease expression) at a diseased site, such as a cancer site, e.g., inside a tumor, the cleavable linker may be cleaved to release the SIRP-α variant from the blocking peptide. The SIRP-α variant would then be free to bind to CD47 on nearby tumor cells. Examples of cleavable linkers are described in detail further herein.

In some embodiments, the blocking peptide has higher affinity towards wild-type SIRP-α than engineered SIRP-α variant. Once the linker is cleaved, the blocking peptide dissociates from the SIRP-α variant and may bind to a wild-type SIRP-α. A blocking peptide with different binding affinities to wild-type SIRP-α and SIRP-α variant may be identified using methods and techniques commonly known in the art, e.g., directed evolution and display libraries (e.g., phage or yeast display). In one exemplary embodiment, a nucleotide encoding the SIRP-α binding region of CD47 or a nucleotide encoding the variable region of an anti-SIRP-a antibody may be mutated and/or recombined at random to create a large library of gene variants using techniques such as, e.g., error-prone PCR and DNA shuffling. Once a genetic library is created, the mutant peptides encoded by the nucleotides may be screened for their ability to bind to wild-type SIRP-α and SIRP-α variant using, e.g., phage or yeast display. Identified peptides that can bind to both wild-type SIRP-α and SIRP-α variant may undergo a second screening process such that the proteins that bind with higher affinity to wild-type SIRP-α than to SIRP-α variant may be isolated. The identified peptides, once bound to wild-type SIRP-α or SIRP-α variant should prevent the binding of CD47 to wild-type SIRP-α or SIRP-α variant. Various techniques known to those skilled in the art may be used to measure the affinity constant ($K_A$) or dissociation constant ($K_D$) of a SIRP-α variant/blocking peptide complex or a wild-type SIRP-a/blocking peptide complex. A blocking peptide may bind with at least three fold higher affinity to a wild-type SIRP-α than a SIRP-α variant.

*CD47-based blocking peptides*

A blocking peptide may be a CD47 mimic polypeptide, or a CD47 fragment that can bind a SIRP-α variant described herein. Some blocking peptides may bind a SIRP-α variant at a site that is different from the CD47 binding site. Some blocking peptides may bind a SIRP-α variant in a manner that is different from CD47. In some cases, the blocking peptide may comprise at least one stabilizing disulfide bond. A blocking peptide may comprise a polypeptide sequence of CERVIGTGWVRC, or a fragment or variant thereof. A variant blocking peptide may contain one or more conservative or non-conservative modification. In some cases a variant blocking peptide may contain modifications of a cysteine to a serine and/or one or more modifications of an asparagine to a glutamine. A blocking peptide may bind to the SIRP-α variant at the same site as a peptide that comprises a polypeptide sequence of CERVIGTGWVRC, or a variant or fragment thereof. A blocking peptide may comprise a polypeptide sequence of GNYTCEVTELTREGETIIELK, or a fragment or variant thereof. A blocking peptide may bind to the SIRP-α variant at the same site as a peptide that comprises a polypeptide sequence of GNYTCEVTELTREGETIIELK, or a variant or fragment thereof. In some cases a blocking peptide may comprise a polypeptide sequence of EVTELTREGE, or a fragment or variant thereof. A blocking peptide may bind to the SIRP-α variant at the same site as a peptide that comprises a polypeptide sequence of EVTELTREGE, or a variant or fragment thereof. In some cases a blocking peptide may comprise a polypeptide sequence of CEVTELTREGEC, or a fragment or variant thereof. A blocking peptide may bind to the SIRP-α variant at the same site as a peptide that comprises a polypeptide sequence of CEVTELTREGEC, or a variant or fragment thereof.

Provided herein are SIRP-α variant constructs comprising a SIRP-α variant and a blocking peptide, wherein the blocking peptide may comprise a polypeptide sequence of SEVTELTREGET, or a fragment or variant thereof. A blocking peptide may bind to the SIRP-α variant at the same site as a peptide that comprises a polypeptide sequence of SEVTELTREGET, or a variant or fragment thereof. In some cases, the blocking peptide may comprise a polypeptide sequence of GQYTSEVTELTREGETIIELK, or a fragment or variant thereof. A blocking peptide may bind to the SIRP-α variant at the same site as a peptide that comprises a polypeptide sequence of GQYTSEVTELTREGETIIELK, or a variant or fragment thereof.

In some cases, the blocking peptide may be a CD47 variant polypeptide, that exhibits a higher affinity for wild-type SIRP-α, as compared to the SIRP-α variant. As compared to wild-type CD47, the blocking polypeptide may comprise at least one of the following mutations: T102Q, T102H, L101Q, L101H, and L101Y. As compared to wild-type CD47, the blocking polypeptide may comprise an introduction of an additional glycine residue at or near the N-terminus. The glycine may be introduced adjacent to a glutamine and/or a leucine at or near the N-terminus of CD47. In some cases, a blocking peptide may be a CD47 variant polypeptide that demonstrates a lower affinity for a SIRP-α variant as compared to a wild-type CD47. Such CD47 variant polypeptides are easily identified and tested using methods described herein.

Provided herein are SIRP-α variant constructs comprising a SIRP-α variant described herein, wherein said SIRP-α variant is connected to a blocking peptide described herein by use of at least one linker. The SIRP-α variant may comprise the same CD47 binding site as a wild type SIRP-α. The SIRP-α variant may comprise one or more mutations, or insertions as compared to a wild type SIRP-α. The SIRP-α variant may be a truncated form of the wild

type SIRP-α. The blocking peptide maybe a CD47 mimic, variant, or fragment described herein. The blocking peptide may exhibit a higher affinity for wild-type SIRP-α, as compared to the SIRP-α variant in the SIRP-α variant construct. The blocking peptide may be a CD47 variant polypeptide that demonstrates a lower affinity for a SIRP-α variant as compared to wild-type CD47. The linker may be at least one linker that is optionally cleavable by one or more proteases, and optionally also comprises one or more spacers. The cleavable linker may comprise the sequence LSGRSDNH. The spacers may comprise one or more units of glycine-serine spacers, each unit of which may comprise the sequence GGGGS.

In some embodiments, the blocking peptide that is attached to a SIRP-α variant by way of a cleavable linker is a SIRP-α-binding peptide derived from CD47 (i.e., a CD47-based blocking peptide). In some embodiments, the CD47-based blocking peptide is derived from the SIRP-α binding portion of CD47. The SIRP-α binding portion of CD47 is often referred to as the immunoglobulin superfamily (IgSF) domain of CD47, the sequence of which is shown below (SEQ ID NO: 35; Ref. NP_0017681).

SEQ ID NO: 35: wild-type, IgSF domain of human CD47

```
   1-50 QLLFNKTKSV EFTFCNDTVV IPCFVTNMEA QNTTEVYVKW KFKGRDIYTF
 51-100 DGALNKSTVP TDFSSAKIEV SQLLKGDASL KMDKSDAVSH TGNYTCEVTE
101-123 LTREGETIIE LKYRVVSWFS PNE
```

In some embodiments, the CD47-based blocking peptide contains the full-length, IgSF domain of CD47 (SEQ ID NO: 35) or a fragment thereof. In some embodiments, the CD47-based blocking peptide contains one or more amino acid substitutions, deletions, and/or additions relative to the wild-type, IgSF domain of CD47 (SEQ ID NO: 35) or a fragment thereof. In some embodiments, a CD47-based blocking peptide has at least 80% (e.g., 83%, 86%, 90%, 93%, 96%, etc) amino acid sequence identity to the sequence of the wild-type, IgSF domain of CD47 (SEQ ID NO: 35) or a fragment thereof.

In some embodiments, the amino acid substitutions, deletions, and/or additions in the CD47-based blocking peptide results in the CD47-based blocking peptide having low binding affinity for a SIRP-α variant and relatively higher binding affinity for the wild-type SIRP-α. In some embodiments, the amino acid substitutions in the CD47-based blocking peptide are located at the interface of CD47 and SIRP-α. For example, amino acid substitution T102Q in the CD47 IgSF domain sterically clashes with amino acid substitution A27I in a SIRP-α variant, while a wild-type SIRP-α having A27 would not sterically clash with the amino acid substitution T102Q (see FIG. 2). Thus, a CD47-based blocking peptide having T102Q would bind with higher affinity to a wild-type SIRP-α having A27 than to a SIRP-α variant having A27I. Examples of amino acid substitutions in a CD47-based blocking peptide that may create steric clashes with specific amino acids in a SIRP-α variant are listed in Table 5. Each of these amino acid substitutions in a CD47-based blocking peptide may reduce the binding affinity of the CD47-based blocking peptide to a SIRP-α variant, depending on the specific amino acid in the SIRP-α variant at the SIRP-α-CD47 interaction site.

Table 5: Examples of amino acid substitutions in a CD47-based blocking peptide that may create steric clashes with specific amino acids in a SIRP-α variant

| Amino acid substitution in a CD47-based blocking peptide (amino acid numbering is relative to SEQ ID NO: 35) | Amino acid in a SIRP-α variant (amino acid numbering is relative to any one of SEQ ID NOs: 13-23) |
|---|---|
| T102Q | 27I |
| T102H | 27I |
| L101Q | 31F |
| L101H | 31F |
| L101Y | 31F |

In addition to creating steric clashes between a CD47-based blocking peptide and a SIRP-α variant, amino acid substitutions, additions, and/or deletions can also be used to break specific non-covalent interactions between a CD47-based blocking peptide and a SIRP-α variant, thus, reducing the binding affinity of the CD47-based blocking peptide to the SIRP-α variant. In some embodiments, extending the N-terminus of the CD47-based blocking peptide by one or more amino acids (e.g., one amino acid), either by adding the one or more amino acids directly to the N-terminus and/or by inserting the one or more amino acids between other amino acids at the N-terminus, breaks non-covalent

interactions (e.g., hydrogen bonding interactions) between the N-terminus of the CD47-based blocking peptide and a SIRP-α variant. For example, an amino acid addition, e.g., a glycine addition, at the N-terminus of the CD47-based blocking peptide will prevent cyclization of glutamine to pyroglutamate at the N-terminus and also create unwanted contacts and interactions that will likely will disrupt the hydrogen bonding interactions between the N-terminal pyroglutamate of the CD47-based blocking peptide and the amino acid L66 in a wild-type SIRP-α or amino acid substitution L66T in a SIRP-α variant (see also Example 5). In some embodiments, an amino acid residue, e.g., glycine, is added at the N-terminus of the CD47-based blocking peptide such that the N-terminus of CD47 is changed from QLLFNK to GQLLFNK or QGLLFNK. The choice of the amino acid substitutions, deletions, and/or additions in a CD47-based blocking peptide would depend on the specific amino acid substitutions in a SIRP-α variant.

Furthermore, fusing the N-terminus of the CD47-based blocking peptide to the C-terminus of a SIRP-α variant through a cleavable linker and optionally one or more spacers also affects the binding interactions between the CD47-based blocking peptide and the SIRP-α variant and reduces the binding affinity of the CD47-based blocking peptide to the SIRP-α variant. In some embodiments, in a SIRP-α variant construct, the N-terminus of a CD47-based blocking peptide is fused to the C-terminus of a SIRP-α variant by way of a cleavable linker and optionally one or more spacers. In some embodiments, in a SIRP-α variant construct, the C-terminus of a CD47-based blocking peptide is fused to the N-terminus of a SIRP-α variant by way of a cleavable linker and optionally one or more spacers. Examples of cleavable linkers and spacers are described in detail further herein.

Exemplary CD47-based blocking peptides are shown in Table 6. In some embodiments, the CD47-based blocking peptide has or includes the sequence SEVTELTREGET (SEQ ID NO: 38). In some embodiments, the CD47-based blocking peptide has or includes the sequence GQYTSEVTELTREGETIIELK (SEQ ID NO: 40).

Table 6

| SEQ ID NO | CD47-based blocking peptides | Portion of the CD47 IgSF domain |
|---|---|---|
| 36 | EVTELTREGE | Amino acids 97-106 of SEQ ID NO: 35 |
| 37 | CEVTELTREGE**C** | Amino acids 96-107 of SEQ ID NO: 35 with T125C |
| 38 | **S**EVTELTREGET | Amino acids 96-107 of SEQ ID NO: 35 with C96S |
| 39 | GNYTCEVTELTREGETIIELK | Amino acids 92-112 of SEQ ID NO: 35 |
| 40 | G**Q**YT**S**EVTELTREGETIIELK | Amino acids 92-112 of SEQ ID NO: 35 with N93Q and C96S |
| 41 | QLLFNKTKSV EFTFCNDTVV IPCFVTNMEA QNTTEVYVKW KFKGRDIYTF DGALNKSTVP TDFSSAKIEV SQLLKGDASL KMDKSDAVSH TGNYTCEVTE **Q**TREGETIIE LKYRVVSWFS PNE | CD47 IgSF domain with L101Q |
| 42 | QLLFNKTKSV EFTFCNDTVV IPCFVTNMEA QNTTEVYVKW KFKGRDIYTF DGALNKSTVP TDFSSAKIEV SQLLKGDASL KMDKSDAVSH TGNYTCEVTE **Y**TREGETIIE LKYRVVSWFS PNE | CD47 IgSF domain with L101Y |
| 43 | QLLFNKTKSV EFTFCNDTVV IPCFVTNMEA QNTTEVYVKW KFKGRDIYTF DGALNKSTVP TDFSSAKIEV SQLLKGDASL KMDKSDAVSH TGNYTCEVTE **H**TREGETIIE LKYRVVSWFS PNE | CD47 IgSF domain with L101H |
| 44 | QLLFNKTKSV EFTFCNDTVV IPCFVTNMEA QNTTEVYVKW KFKGRDIYTF DGALNKSTVP TDFSSAKIEV SQLLKGDASL KMDKSDAVSH TGNYTCEVTE L**Q**REGETIIE LKYRVVSWFS PNE | CD47 IgSF domain with T102Q |
| 45 | QLLFNKTKSV EFTFCNDTVV IPCFVTNMEA QNTTEVYVKW KFKGRDIYTF DGALNKSTVP TDFSSAKIEV SQLLKGDASL KMDKSDAVSH TGNYTCEVTE L**H**REGETIIE LKYRVVSWFS PNE | CD47 IgSF domain with T102H |

(continued)

| SEQ ID NO | CD47-based blocking peptides | Portion of the CD47 IgSF domain |
|---|---|---|
| 46 | GQLLFNKTKSV EFTFCNDTVV IPCFVTNMEA QNTTEVYVKW KFKGRDIYTF DGALNKSTVP TDFSSAKIEV SQLLKGDASL KMDKSDAVSH TGNYTCEVTE LTREGETIIE LKYRVVSWFS PNE | CD47 IgSF domain with N-terminal glycine addition |

## VII. Cleavable linkers

In some embodiments, a SIRP-α variant construct includes a SIRP-α variant attached to a blocking peptide. In some embodiments, a SIRP-α variant construct includes a wild-type SIRP-α attached to a blocking peptide. A linker used to fuse a SIRP-α variant or a wild-type SIRP-α and a blocking peptide can be a cleavable linker or a non-cleavable linker. In some embodiments, the preferential binding of the SIRP-α variant in the SIRP-α variant construct to CD47 on diseased cells or diseased sites may be obtained by attaching the block peptide to the SIRP-α variant by use of a cleavable linker, which is cleaved at the diseased cells or diseased sites.

In some embodiments, a cleavable linker is used between a SIRP-α variant and a blocking peptide. In some embodiments, a cleavable linker may be installed within a blocking peptide, which may be non-covalently associated with the SIRP-α variant to block binding of the SIRP-α variant to CD47 under physiological conditions. A cleavable linker may be cleaved under certain conditions. If the cleavable linker is within a blocking peptide, cleavage of the linker would inactivate the blocking peptide. Under conditions characteristic of a diseased site, such as a cancer site, e.g., inside a tumor, the linker is cleaved to release the SIRP-α variant from the blocking peptide such that the SIRP-α variant can bind to nearby CD47 on the cell surface of diseased cells, e.g., tumor cells. In this manner, in a SIRP-α construct that includes a SIRP-α variant and a blocking peptide, the SIRP-α variant can only bind to CD47 on diseased cells (e.g., tumor cells) or cells at a diseased site (e.g., cells in the tumor micro-environment supporting tumor growth), and is unable to bind to CD47 on non-diseased cells under physiological conditions, since the cleavable linker remains stable under physiological conditions and the CD47-binding site of the SIRP-α variant would be blocked by the blocking peptide. A cleavable linker may include amino acids, organic small molecules, or a combination of amino acids and organic small molecules that cleave or induce cleavage of the linker under conditions characteristic of a diseased site, such as acidic pH, hypoxia, and increased protease expression. Cleavable linkers are stable at physiological conditions (e.g., neutral pH and adequate oxygen concentration). In some embodiments, a cleavable linker may not be cleaved and the blocking peptide may simply dissociate from the SIRP-α variant at a diseased site such that the SIRP-α variant is free to bind to nearby CD47 on diseased cells, e.g., tumor cells. In these embodiments, the SIRP-α variants may be engineered to have pH-dependent binding to CD47, the details of which are described previously. The SIRP-α variants may be engineered to bind with high affinity to CD47 under acidic pH of a diseased site than under neutral pH (e.g., around pH 7.4) of a non-diseased site. Thus, the blocking peptide (e.g.., a CD-47 based blocking peptide or a CD47 IgSF domain blocking protein) may dissociate away from the SIRP-α variant under the acidic pH of a diseased site. In some embodiments, to engineer pH-dependent binding of a SIRP-α variant to CD47 at a diseased site, histidine mutagenesis may be performed on the SIRP-α, especially on the region of SIRP-α that interacts with CD47.

*pH-dependent cleavable linkers*

One of the characteristics of a cancer site, e.g., inside a tumor, is acidic pH. In some embodiments, a linker may be cleaved under acidic pH (e.g., less than around pH 7). An acid-sensitive linker is stable at physiological pH (e.g., around pH 7.4). The cleavage at acidic pH may be through acid hydrolysis or by proteins present and active at acidic pH of a diseased site, such as a cancer site, e.g., inside a tumor. Acid-sensitive linkers may include a moiety, such as a chemical functional group or compound, capable of being hydrolyzed under acidic pH. Acid-sensitive chemical functional groups and compounds include, but are not limited to, e.g., acetals, ketals, thiomaleamates, hydrazones, and disulfide bonds. Acid-sensitive linkers, as well as acid-sensitive chemical groups and compounds, which may be used in the construction of acid-sensitive linkers, are well known in the art and described in US Patent Nos. 8,748,399 , 5,306,809 , and 5,505,931 , Laurent et al., (Bioconjugate Chem. 21:5-13, 2010), Castaneda et al. (Chem. Commun. 49:8187-8189, 2013), and Ducry et al. (Bioconjug. Chem. 21:5-13, 2010).. In one embodiment, a disulfide bond may be installed in a cleavable linker using a peptide synthesizer and/or conventional chemical synthesis techniques. In another embodiment, a thiomaleamic acid linker (Castaneda et al. Chem. Commun. 49:8187-8189, 2013) may be used as the cleavable linker. In this embodiment, to insert a thiomalemic acid linker between a SIRP-α variant and a blocking peptide, one of the two thiol groups of the thiomalemic acid linker (see, e.g., Scheme 2, Castaneda et al.) may be attached to the C-terminus of a SIRP-α variant, while the ester group of the thiomalemic linker may be attached to the N-terminus of the blocking

peptide.

*Hypoxia-dependent cleavable linkers*

In some embodiments, a linker may be cleaved under hypoxic condition, which is another characteristic of a cancer site, e.g., inside a tumor. A SIRP-α variant attached to a blocking peptide by way of a hypoxia-sensitive linker is prevented from binding to CD47 on non-diseased cells while the linker remains stable under physiological conditions (e.g., neutral pH and adequate oxygen concentration). Once the fusion protein is at the site of cancer, e.g., inside a tumor, where oxygen concentration is significantly lower than in healthy tissues, the linker is cleaved to release the SIRP-α variant from the blocking peptide, which can then bind to cell surface CD47 on tumor cells. The hypoxia-sensitive linker may include a moiety, e.g., an amino acid or a chemical functional group, capable of being cleaved under hypoxic condition. Some examples of chemical moieties that may be cleaved, i.e., cleaved through reduction, under hypoxic condition include, but are not limited to, quinones, N-oxides, and heteroaromatic nitro groups. These chemical moieties may be installed in the cleavable linker using conventional chemical and peptide synthesis techniques. Examples of hypoxia-sensitive amino acids are also known in the art, such as those described by Shigenaga et al. (European Journal of Chemical Biology 13:968-971, 2012).

In a preferred embodiment, the hypoxia-sensitive amino acid described by Shigenaga et al. (European J. Chem, Biol. 13:968-971, 2012) may be inserted between a SIRP-α variant and a blocking peptide. For example, the amino group of the hypoxia-sensitive amino acid may be attached to the C-terminus of the SIRP-α variant through a peptide bond, and similarly, the carboxylic acid group of the hypoxia-sensitive amino acid may be attached to the N-terminus of the blocking peptide through a peptide bond. Under hypoxic condition, the reduction of the nitro group induces the cleavage of the peptide bond between the hypoxia-sensitive amino acid and the N-terminus of the blocking peptide, thus, successfully releasing the SIRP-α variant from the blocking peptide. The SIRP-α variant can then bind to CD47 on tumor cells.

In another embodiment, the hypoxia-sensitive 2-nitroimidazole group described by Duan et al. (J. Med. Chem. 51:2412-2420, 2008) may be inserted between a SIRP-α variant and a blocking peptide or installed in a cleavable linker inserted between a SIRP-α variant and a blocking peptide. Under hypoxic condition, the reduction of the nitro group induces further reduction, which eventually leads to elimination of the 2-nitroimidazole group from its attachment, e.g., the SIRP-α variant, the blocking peptide, or the cleavable linker.

*Tumor-associated enzyme-dependent cleavable linkers*

In other embodiments, a SIRP-α variant construct may include a SIRP-α variant attached to a blocking peptide by way of a linker (e.g., a cleavable linker) and optionally one or more spacers (examples of spacers are described in detail further herein). In some embodiments, the linker (e.g., a cleavable linker) may be cleaved by a tumor-associated enzyme. In some embodiments, a linker, which can be cleaved by a tumor-associated enzyme, may be contained within a blocking peptide, which may be non-covalently attached to a SIRP-α variant. Once the fusion protein is at the site of cancer, e.g., inside a tumor, the linker is cleaved by a tumor-associated enzyme to release the SIRP-α variant from the blocking peptide, which can then bind to cell surface CD47 on tumor cells. A linker sensitive to a tumor-associated enzyme may contain a moiety, e.g., a protein substrate, capable of being specifically cleaved by an enzyme, e.g., a protease, that is only present at the cancer site, e.g., inside a tumor. The moiety may be selected based on the type of enzyme, e.g., a protease, present at the cancer site, e.g., inside a tumor. An exemplary cleavable linker that can be cleaved by a tumor-associated enzyme is LSGRSDNH (SEQ ID NO: 47), which can be cleaved by multiple proteases, e.g., matriptase (MTSP1), urinary-type plasminogen activator (uPA), legumain, PSA (also called KLK3, kallikrein-related peptidase-3), matrix metalloproteinase-2 (MMP-2), MMP9, human neutrophil elastase (HNE), and proteinase 3 (Pr3).. Other cleavable linkers that are susceptible to cleavage by enzymes (e.g., proteases) are also available. In addition to the aforementioned proteases, other enzymes (e.g., proteases) that can cleave a cleavable linker include, but are not limited to, urokinase, tissue plasminogen activator, trypsin, plasmin, and another enzyme having proteolytic activities. According to some embodiments of the present invention, a SIRP-α variant or a wild-type SIRP-α is attached to a blocking peptide by way of a linker (e.g., a cleavable linker) susceptible to cleavage by enzymes having proteolytic activities, such as a urokinase, a tissue plasminogen activator, plasmin, or trypsin.

In some embodiments, sequences of cleavable linkers can be derived and selected by putting together several sequences based on different enzyme preferences. Non-limited examples of several potential proteases and their corresponding protease sites are shown in Table 7. In Table 7, "-" means any amino acid (i.e., any naturally occurring amino acid), capital case indicates an strong preference for that amino acid, lower case indicates a minor preference for that amino acid, and "/" separates amino acid positions in cases where more than one amino acid at a position adjacent to the "/"is possible. Other cleavable sequences include, but are not limited to, a sequence from a human liver collagen (α1(III) chain (e.g., GPLGIAGI (SEQ ID NO: 100))), a sequence from a human PZP (e.g., YGAGLGVV (SEQ ID NO:

101), AGLGVVER (SEQ ID NO: 102), or AGLGISST (SEQ ID NO: 103)), and other sequences that are autolytic (e.g., VAQFVLTE (SEQ ID NO: 104), AQFVLTEG (SEQ ID NO: 105), or PVQPIGPQ (SEQ ID NO: 106)).

Table 7

| Protease | Potential protease sites |
|---|---|
| uPA: | L/S/G-/R--/S-/D/N/H (SEQ ID NO: 69); -/s/gs/Rk-/rv/-/-/- (SEQ ID NO: 70); SGR-SA (SEQ ID NO: 71) |
| Matriptase: | L/S/G-/R-/S-/D/N/H (SEQ ID NO: 72); r/-/--/Rk-/v-/-/g/- (SEQ ID NO: 73); RQAR-VV (SEQ ID NO; 74); r/-/-/Rk/v/-/g (SEQ ID NO: 75); /Kr/RKQ/gAS/RK/A (SEQ ID NO: 76) |
| Legumain: | L/S/G-/R--/S-/D/N/H (SEQ ID NO: 77); ---/--/-/N/-/-/- (SEQ ID NO: 78); AAN-L (SEQ ID NO: 79); ATN-L (SEQ ID NO: 80) |
| PSA | si/sq/-/yqr s/s/-/- (SEQ ID NO: 81); S/S/K/L/Q (SEQ ID NO: 82) |
| MMP2 | -/p/-/- /li/-/-/- (SEQ ID NO: 83) |
| MMP9 | g/pa/-/gl/-/g/- (SEQ ID NO: 84); G/P/L/G/I/A/G/Q (SEQ ID NO: 85); |
| | |
| Protease | Potential protease sites |
| | P/V/G/L/I/G (SEQ ID NO: 86); H/P/V/G/L/L/A/R (SEQ ID NO: 87) |
| HNE | -/-/-/viat-/-/-/- (SEQ ID NO: 88) |
| Pr3 | -/y/y/vta -/-/-/- (SEQ ID NO: 89) |
| Pro-urokinase | PRFKIIGG (SEQ ID NO: 90); PRFRIIGG (SEQ ID NO: 91) |
| TGFβ | SSRHRRALD (SEQ ID NO: 92) |
| Plasminogen | RKSSIIIRMRDVVL (SEQ ID NO: 93) |
| Staphylokinase | SSSFDKGKYKKGDDA (SEQ ID NO: 94); SSSFDKGKYKRGDDA (SEQ ID NO: 95) |
| Factor Xa | IEGR; IDGR (SEQ ID NO: 96); GGSIDGR (SEQ ID NO: 97) |
| Gelatinase | PLGLWA (SEQ ID NO: 98) |
| Human fibroblast collagenase | DVAQFVLT (SEQ ID NO: 99) |

There are reports in the literature of increased levels of enzymes having known substrates in various types of cancers, e.g., solid tumors. See, e.g., La Rocca et al., Brit. J. Cancer 90:1414-1421 and Ducry et al., Bioconjug. Chem. 21:5-13, 2010. Tumor-associated enzymes may also be identified using conventional techniques known in the art, e.g., immunohistochemistry of tumor cells. In one exemplary embodiment, the enzyme-sensitive moiety in a linker may be a matrix metalloproteinase (MMP) substrate, which may be cleaved by an MMP present at the cancer site, e.g., inside a tumor. In another exemplary embodiment, the enzyme-sensitive moiety in a linker may be a maleimido-containing dipeptide linker (see, e.g., Table 1 in Ducry et al.), which may be cleaved through proteolysis by proteases (e.g., cathepsin or plasmin) present at elevated levels in certain tumors (Koblinski et al., Chim. Acta 291:113-135, 2000). In this embodiment, the maleimide group of the maleimido-containing dipeptide linker may be conjugated to a cysteine residue of the SIRP-α variant and the carboxylic acid group at the C-terminus of the maleimido-containing dipeptide linker may be conjugated to the amino group at the N-terminus of the blocking peptide. Similarly, the maleimide group of the maleimido-containing dipeptide linker may be conjugated to a cysteine residue of the blocking peptide and the carboxylic acid group at the C-terminus of the maleimido-containing dipeptide linker may be conjugated to the amino group at the N-terminus of the SIRP-α variant. Mass-spectrometry and other available techniques in the field of proteomics may be used to confirm the cleavage of the tumor-associated enzyme-dependent cleavable linkers. Other enzyme-sensitive moieties are described in US Patent No. 8,399,219. In some embodiments, the moiety sensitive to a tumor-associated enzyme, e.g., a protein substrate, may be inserted between a SIRP-α variant and a blocking peptide using conventional molecule cell biology and chemical conjugation techniques well known in the art.

## VIII. Serum albumin

Fusion to serum albumins can improve the pharmacokinetics of protein pharmaceuticals, and in particular, a SIRP-α variant described here may be joined with a serum albumin. Serum albumin is a globular protein that is the most abundant blood protein in mammals. Serum albumin is produced in the liver and constitutes about half of the blood serum proteins. It is monomeric and soluble in the blood. Some of the most crucial functions of serum albumin include transporting hormones, fatty acids, and other proteins in the body, buffering pH, and maintaining osmotic pressure needed for proper distribution of bodily fluids between blood vessels and body tissues. In some embodiments, a SIRP-α variant may be fused to a serum albumin. In preferred embodiments, serum albumin is human serum albumin (HSA). In some embodiments of the present invention, the N-terminus of an HSA is joined to the C-terminus of the SIRP-α variant to increase the serum half-life of the SIRP-α variant. An HSA can be joined, either directly or through a linker, to the C-terminus of the SIRP-α variant. Joining the N-terminus of an HSA to the C-terminus of the SIRP-α variant keeps the N-terminus of the SIRP-α variant free to interact with CD47 and the proximal end of the C-terminus of the HSA to interact with FcRn. An HSA that can be used in the methods and compositions described here are generally known in the art. In some embodiments, the HSA includes amino acids 25-609 (SEQ ID NO: 67) of the sequence of UniProt ID NO: P02768. In some embodiments, the HSA includes one or more amino acid substitutions (e.g., C34S and/or K573P), relative to SEQ ID NO: 67. In some embodiments, the HSA has the sequence of SEQ ID NO: 68.

## IX. Albumin-binding peptides

Binding to serum proteins can improve the pharmacokinetics of protein pharmaceuticals, and in particular the SIRP-α variants described here may be fused with serum protein-binding peptides or proteins. In some embodiments, a SIRP-α variant may be fused to an albumin-binding peptide that displays binding activity to serum albumin to increase the half-life of the SIRP-α variant. Albumin-binding peptides that can be used in the methods and compositions described here are generally known in the art. See, e.g., Dennis et al., J. Biol. Chem. 277:35035-35043, 2002 and Miyakawa et al., J. Pharm. Sci. 102:3110-3118, 2013. In one embodiment, the albumin binding peptide includes the sequence DICLPRWGCLW (SEQ ID NO: 2). An albumin-binding peptide can be fused genetically to a SIRP-α variant or attached to a SIRP-α variant through chemical means, e.g., chemical conjugation. If desired, a spacer can be inserted between the SIRP-α variant and the albumin-binding peptide to allow for additional structural and spatial flexibility of the fusion protein. Specific spacers and their amino acid sequences are described in detail further herein. In some embodiments, an albumin-binding peptide may be fused to the N- or C-terminus of a SIRP-α variant. In one example, the C-terminus of the albumin-binding peptide may be directly fused to the N-terminus of the SIRP-α variant through a peptide bond. In another example, the N-terminus of the albumin-binding peptide may be directly fused to the C-terminus of the SIRP-α variant through a peptide bond. In yet another example, the carboxylic acid at the C-terminus of the albumin-binding peptide may be fused to an internal amino acid residue, i.e., the side-chain amino group of a lysine residue of the SIRP-α variant using conventional chemical conjugation techniques. Without being bound to a theory, it is expected that fusion of an albumin-binding peptide to a SIRP-α variant may lead to prolonged retention of the therapeutic protein through its binding to serum albumin.

## X. Fc domains

In some embodiments, a SIRP-α variant construct may include a SIRP-α variant and an Fc domain monomer. In some embodiments, a SIRP-α variant may be fused to an Fc domain monomer of an immunoglobulin or a fragment of an Fc domain monomer. As conventionally known in the art, an Fc domain is the protein structure that is found at the C-terminus of an immunoglobulin. An Fc domain includes two Fc domain monomers that are dimerized by the interaction between the $C_H3$ antibody constant domains. A wild-type Fc domain forms the minimum structure that binds to an Fc receptor, e.g., FcγRI, FcγRIIa, FcγRIIb, FcγRIIIa, FcγRIIIb, FcγRIV. In the present invention, an Fc domain monomer or a fragment of an Fc domain fused to a SIRP-α variant to increase serum half-life of the SIRP-α variant may include a dimer of two Fc domain monomers or an Fc domain monomer, provided that the Fc domain monomer can bind to the Fc receptor (e.g., an FcRn receptor). Furthermore, an Fc domain or a fragment of the Fc domain fused to a SIRP-α variant to increase serum half-life of the SIRP-α variant does not induce any immune system-related response. In some embodiments, an Fc domain may be mutated to lack effector functions, typical of a "dead" Fc domain. For example, an Fc domain may include specific amino acid substitutions that are known to minimize the interaction between the Fc domain and an Fcγ receptor. In some embodiments, an Fc domain monomer or a fragment of the Fc domain may be fused to the N- or C-terminus of a SIRP-α variant through conventional genetic or chemical means, e.g., chemical conjugation. If desired, a linker (e.g., a spacer) can be inserted between the SIRP-α variant and the Fc domain monomer.

*Heterodimerization of Fc domain monomers*

In some embodiments, each of the two Fc domain monomers in an Fc domain includes amino acid substitutions that promote the heterodimerization of the two monomers. Heterodimerization of Fc domain monomers can be promoted by introducing different, but compatible, substitutions in the two Fc domain monomers, such as "knob-into-hole" residue pairs and charge residue pairs. The use of "knob-into-hole" residue pairs is described by Carter and co-workers (Ridgway et al., Protein Eng. 9:617-612, 1996 ; Atwell et al., J Mol Biol. 270:26-35, 1997 ; Merchant et al., Nat Biotechnol. 16:677-681, 1998). The knob and hole interaction favors heterodimer formation, whereas the knob-knob and the hole-hole interaction hinder homodimer formation due to steric clash and deletion of favorable interactions. The "knob-into-hole" technique is also disclosed in U.S. Patent Application Publication No. 8,216,805 , Merchant et al., Nature Biotechnology 16:677-681, 1998, and Merchant et al., Proc Natl Acad Sci USA. 110:E2987-E2996, 2013. A hole is a void that is created when an original amino acid in a protein is replaced with a different amino acid having a smaller side-chain volume. A knob is a bump that is created when an original amino acid in a protein is replaced with a different amino acid having a larger side-chain volume. Specifically, the amino acid being replaced is in the $C_H3$ antibody constant domain of an Fc domain monomer and is involved in the dimerization of two Fc domain monomers. In some embodiments, a hole in one $C_H3$ antibody constant domain is created to accommodate a knob in another $C_H3$ antibody constant domain, such that the knob and hole amino acids act to promote or favor the heterodimerization of the two Fc domain monomers. In some embodiments, a hole in one $C_H3$ antibody constant domain is created to better accommodate an original amino acid in another $C_H3$ antibody constant domain. In some embodiments, a knob in one $C_H3$ antibody constant domain is created to form additional interactions with original amino acids in another $C_H3$ antibody constant domain.

A hole can be constructed by replacing amino acids having larger side chains such as tyrosine or tryptophan with amino acids having smaller side chains such as alanine, valine, or threonine, such as Y407V mutation in the $C_H3$ antibody constant domain. Similarly, a knob can be constructed by replacing amino acids having smaller side chains with amino acids having larger side chains, such as T366W mutation in the $C_H3$ antibody constant domain. In a preferred embodiment, one Fc domain monomer includes the knob mutation T366W and the other Fc domain monomer includes hole mutations T366S, L358A, and Y407V. A SIRP-α D1 variant of the invention may be fused to an Fc domain monomer including the knob mutation T366W to limit unwanted knob-knob homodimer formation. Examples of knob-into-hole amino acid pairs are included, without limitation, in Table 8.

Table 8

| | | | | | | | | |
|---|---|---|---|---|---|---|---|---|
| Fc domain monomer 1 | Y407T | Y407A | F405A | T394S | T366S L358A Y407V | T394W Y407T | T394S Y407A | T366W T394S |
| Fc domain monomer 2 | T366Y | T366W | T394W | F405W | T366W | T366Y F405A | T366W F405W | F405W Y407A |

In addition to the knob-into-hole strategy, electrostatic steering strategy may also be used to control the dimerization of Fc domain monomers. Electrostatic steering is the utilization of favorable electrostatic interactions between oppositely charged amino acids in peptides, protein domains, and proteins to control the formation of higher ordered protein molecules. In particular, to control the dimerization of Fc domain monomers using electrostatic steering, one or more amino acid residues that make up the $C_H3$-$C_H3$ interface are replaced with positively- or negatively-charged amino acid residues such that the interaction becomes electrostatically favorable or unfavorable depending on the specific charged amino acids introduced. In some embodiments, a positively-charged amino acid in the interface, such as lysine, arginine, or histidine, is replaced with a negatively-charged amino acid such as aspartic acid or glutamic acid. In some embodiments, a negatively-charged amino acid in the interface is replaced with a positively-charged amino acid. The charged amino acids may be introduced to one of the interacting $C_H3$ antibody constant domains, or both. Introducing charged amino acids to the interacting $C_H3$ antibody constant domains of the two Fc domain monomers can promote the selective formation of heterodimers of Fc domain monomers as controlled by the electrostatic steering effects resulting from the interaction between charged amino acids. The electrostatic steering technique is also disclosed in U.S. Patent Application Publication No. 20140024111 , Gunasekaran et al., J Biol Chem. 285:19637-46, 2010 , and Martens et al., Clin Cancer Res. 12:6144-52, 2006. Examples of electrostatic steering amino acid pairs are included, without limitation, in Table 9.

Table 9

| Fc domain monomer 1 | K409D | K409D | K409E | K409E | K392D | K392D | K392E | K392E | K409D K392D | K370E K409D K439E |
|---|---|---|---|---|---|---|---|---|---|---|
| Fc domain monomer 2 | D399K | D399 R | D399K | D399R | D399K | D399R | D399K | D399R | D399K D356K | D356K E357K D399K |

**XI. Polyethylene glycol (PEG) polymer**

In some embodiments, a SIRP-α variant may also be fused to a polymer, e.g., polyethylene glycol (PEG). The attachment of a polymer to a protein pharmaceutical can "mask" the protein pharmaceutical from the host's immune system (Milla et al., Curr Drug Metab. 13:105-119, 2012). In addition, certain polymers, e.g., hydrophilic polymers, can also provide water solubility to hydrophobic proteins and drugs (Gregoriadis et al., Cell Mol. Life Sci. 57:1964-1969, 2000; Constantinou et al., Bioconjug. Chem. 19:643-650, 2008). Various polymers, such as PEG, polysialic acid chain (Constantinou et al., Bioconjug. Chem. 19:643-650, 2008), and PAS chain (Schlapschy et al., Protein Eng. Des. Sel. 26:489-501, 2013), are known in the art and can be used in the present invention. In some embodiments, a polymer, e.g., PEG, may be covalently attached to a SIRP-α variant, either at the N- or C-terminus or at an internal location, using conventional chemical methods, e.g., chemical conjugation. In some embodiments, a polymer, e.g., PEG, may be covalently attached to a cysteine substitution or addition in the SIRP-α variant. The cysteine substitution in the SIRP-α variant may be I7C, A16C, S20C, T20C, A45C, G45C, G79C, S79C, or A84C, relative to the sequence of any one of SEQ ID NOs: 13-23. The addition of a cysteine residue in the SIRP-α variant may be introduced using conventional techniques in the art, e.g., peptide synthesis, genetic modification, and/or molecular cloning. The polymer, e.g., PEG, may be attached to the cysteine residue using cysteine-maleimide conjugation well-known to one of skill in the art.

In addition to the embodiments described above, other half-life extension technologies are also available and may be used in the present invention to increase the serum half-life of SIRP-α variants. Half-life extension technologies include, but are not limited to, and EXTEN (Schellenberger et al., Nat. Biotechnol. 27:1186-1192, 2009) and Albu tag (Trussel et al., Bioconjug Chem. 20:2286-2292, 2009).

**XII. Spacers**

In some embodiments, spacers may be used in the SIRP-α variant construct. For examples, a SIRP-α variant construct may include a SIRP-α variant attached to a blocking peptide by way of a linker (e.g., a cleavable linker). In such SIRP-α constructs, a spacer may be inserted between the SIRP-α variant and the linker (e.g., a cleavable linker), and/or between the linker (e.g., a cleavable linker) and the blocking peptide. To optimized the spacing between the SIRP-α variant and the linker, and/or the spacing between the linker and the blocking peptide, any one or more of the spacers described below may be used. In some embodiments of a SIRP-α variant construct including a SIRP-α variant attached to a blocking peptide by way of a linker (e.g., a cleavable linker), the spacer serves to position the cleavable linker away from the core of the SIRP-α variant and the blocking peptide such that the cleavable linker is more accessible to the enzyme responsible for cleavage. It should be understood that the attachment of two elements in a SIRP-α variant construct, for example, a SIRP-α variant and a linker (e.g., a cleavable linker) in a SIRP-α variant construct including (e.g., in this order) a SIRP-α variant, a linker, and a blocking peptide, need not be of particular mode of attachment or through a particular reaction. Any reaction providing a SIRP-α variant construct of suitable stability and biological compatibility is acceptable.

A spacer refers to a linkage between two elements in a SIRP-α variant construct, e.g., a SIRP-α variant and a linker (e.g., a cleavable linker) in a SIRP-α variant construct including (e.g., in this order) a SIRP-α variant, a linker, and a blocking peptide, a linker (e.g., a cleavable linker) and a blocking peptide in a SIRP-α variant construct including (e.g., in this order) a SIRP-α variant, a linker, and a blocking peptide, a SIRP-α variant and a serum protein-binding peptide or protein, e.g., an albumin-binding peptide. A spacer may also refer to a linkage that can be inserted between a SIRP-α variant or a wild-type SIRP-α and an antibody, e.g., a tumor-specific antibody, or an antibody-binding peptide. A spacer can provide additional structural and/or spatial flexibility of the SIRP-α variant construct. A spacer can be a simple chemical bond, e.g., an amide bond, a small, organic molecule (e.g., a hydrocarbon chain), an amino acid sequence (e.g., a 3-200 amino acid sequence), or a combination of a small, organic molecule (e.g., a hydrocarbon chain) and an amino acid sequence (e.g., a 3-200 amino acid sequence). A spacer is stable under physiological conditions (e.g., neutral pH and adequate oxygen concentration) as well as under conditions characteristic of a diseased site, e.g., acidic pH and hypoxia. A spacer is stable at a diseased site, such as a cancer site, e.g., inside a tumor.

A spacer may include 3-200 amino acids. Suitable peptide spacers are known in the art, and include, for example, peptide linkers containing flexible amino acid residues, such as glycine and serine. In certain embodiments, a spacer can contain motifs, e.g., multiple or repeating motifs, of GS, GGS, GGGGS, GGSG, or SGGG. In certain embodiments, a spacer can contain 2 to 12 amino acids including motifs of GS, e.g., GS, GSGS, GSGSGS, GSGSGSGS, GSGSGSGSGS, or GSGSGSGSGSGS. In certain other embodiments, a spacer can contain 3 to 12 amino acids including motifs of GGS, e.g., GGS, GGSGGS, GGSGGSGGS, and GGSGGSGGSGGS. In yet other embodiments, a spacer can contain 4 to 12 amino acids including motifs of GGSG, e.g., GGSG, GGSGGGSG, or GGSGGGSGGGSG. In other embodiments, a spacer can contain motifs of $(GGGGS)_n$, wherein n is an integer from 1 to 10. In other embodiments, a spacer can also contain amino acids other than glycine and serine, e.g., GENLYFQSGG, SACYCELS, RSIAT, RPACKIPNDLKQKVMNH, GGSAGGSGSGSSGGSSGASGTGTAGGTGSGSGTGSG, AAANSSIDLISVPVDSR, or GGSGGGSEGGGSEGGGSEGGGSEGGGSEGGGSGGGS. In some embodiments in the present invention, one or more 12- or 20-amino acid peptide spacers may be used in a SIRP-α variant construct. The 12- and 20-amino acid peptide spacers may contain sequences GGGSGGGSGGGS and SGGGSGGGSGGGSGGGSGGG, respectively. In some embodiments, one or more 18-amino acid peptide spacers containing sequence GGSGGGSGGGSGGGSGGS may be used in a SIRP-α variant construct.

In some embodiments, a spacer may also have the general structure

$$\text{-}W\text{-}(CH_2)_n\text{-}Q\text{-},$$

wherein W is NH or $CH_2$, Q is an amino acid or a peptide, and n is an integer from 0 to 20.

### XIII. Fusion of a blocking peptide to a SIRP-α variant

A blocking peptide (e.g., a CD47-based blocking peptide having a sequence of any one of SEQ ID NOs: 36-46 in Table 6) may be fused to the N- or C-terminus of a SIRP-α variant by way of a linker, e.g., a cleavable linker (e.g., LSGRSDNH (SEQ ID NO: 47)), and optionally one or more spacers (e.g., $(GGGGS)_n$, fused genetically to either N- or C-terminus of the linker, wherein n is an integer from 1 to 10). Exemplary sequences of SIRP-α variant constructs including a CD47-based blocking peptide fused to a SIRP-α variant by way of a cleavable linker and one or more spacers are shown in sequences of SEQ ID NOs: 48-63. The length of the spacers may be changed to achieve the most optimized binding between the CD47-based blocking peptide and the SIRP-α variant.

### XIV. Methods of producing SIRP-α variant constructs

The SIRP-α variant constructs of the invention can be produced from a host cell. A host cell refers to a vehicle that includes the necessary cellular components, e.g., organelles, needed to express the polypeptides and constructs described herein from their corresponding nucleic acids. The nucleic acids may be included in nucleic acid vectors that can be introduced into the host cell by conventional techniques known in the art (e.g., transformation, transfection, electroporation, calcium phosphate precipitation, direct microinjection, infection, etc). The choice of nucleic acid vectors depends in part on the host cells to be used. Generally, preferred host cells are of either prokaryotic (e.g., bacterial) or eukaryotic (e.g., mammalian) origin.

*Nucleic Acid Vector Construction and Host Cells*

A polynucleotide sequence encoding the amino acid sequence of a SIRP-α variant construct may be prepared by a variety of methods known in the art. These methods include, but are not limited to, oligonucleotide-mediated (or site-directed) mutagenesis and PCR mutagenesis. A polynucleotide molecule encoding a SIRP-α variant construct of the invention may be obtained using standard techniques, e.g., gene synthesis. Alternatively, a polynucleotide molecule encoding a wild-type SIRP-α may be mutated to contain specific histidine substitutions using standard techniques in the art, e.g., QuikChange™ mutagenesis. Polynucleotides can be synthesized using nucleotide synthesizer or PCR techniques.

Polynucleotide sequences encoding SIRP-α variant constructs may be inserted into a vector capable of replicating and expressing the polynucleotides in prokaryotic or eukaryotic host cells. Many vectors are available in the art and can be used for the purpose of the invention. Each vector may contain various components that may be adjusted and optimized for compatibility with the particular host cell. For example, the vector components may include, but are not limited to, an origin of replication, a selection marker gene, a promoter, a ribosome binding site, a signal sequence, a polynucleotide sequence encoding a SIRP-α variant construct of the invention, and a transcription termination sequence. In some embodiments, a vector can include internal ribosome entry site (IRES) that allows the expression of multiple SIRP-α variant constructs. Some examples of bacterial expression vectors include, but are not limited to, pGEX series of vectors (e.g., pGEX-2T, pGEX-3X, pGEX-4T, pGEX-5X, pGEX-6P), pET series of vectors (e.g., pET-21, pET-21a, pET-21b, pET-23, pET-24), pACYC series of vectors (e.g., pACYDuet-1), pDEST series of vectors (e.g., pDEST14, pDEST15, pDEST24, pDEST42), and pBR322 and its derivatives (see, e.g., U.S. Patent No. 5,648,237). Some examples of mammalian expression vectors include, but are not limited to, pCDNA3, pCDNA4, pNICE, pSELECT, and pFLAG-CMV. Other types of nucleic acid vectors include viral vectors for expressing a protein in a cell (e.g., a cell of a subject). Such viral vectors include, but are not limited to, retroviral vectors, adenoviral vectors, poxviral vectors (e.g., vaccinia viral vectors, such as Modified Vaccinia Ankara (MVA)), adeno-associated viral vectors, and alphaviral vectors.

In some embodiments, *E. coli* cells are used as host cells for the invention. Examples of *E. coli* strains include, but are not limited to, *E. coli* 294 (ATCC® 31,446), *E. coli* λ 1776 (ATCC® 31,537, *E. coli* BL21 (DE3) (ATCC® BAA-1025), and *E. coli* RV308 (ATCC®31,608). In other embodiments, mammalian cells are used as host cells for the invention. Examples of mammalian cell types include, but are not limited to, human embryonic kidney (HEK) cells, Chinese hamster ovary (CHO) cells, HeLa cells, PC3 cells, Vero cells, and MC3T3 cells. Different host cells have characteristic and specific mechanisms for the posttranslational processing and modification of protein products. Appropriate cell lines or host systems may be chosen to ensure the correct modification and processing of the protein expressed. The above-described expression vectors may be introduced into appropriate host cells using conventional techniques in the art, e.g., transformation, transfection, electroporation, calcium phosphate precipitation, and direct microinjection. Once the vectors are introduced into host cells for protein production, host cells are cultured in conventional nutrient media modified as appropriate for inducing promoters, selecting transformants, or amplifying the genes encoding the desired sequences.

*Protein Production, Recovery, and Purification*

Host cells used to produce the SIRP-α variant constructs of the invention may be grown in media known in the art and suitable for culturing of the selected host cells. Examples of suitable media for bacterial host cells include Luria broth (LB) plus necessary supplements, such as a selection agent, e.g., ampicillin. Examples of suitable media for mammalian host cells include Minimal Essential Medium (MEM), Dulbecco's Modified Eagle's Medium (DMEM), DMEM with supplemented fetal bovine serum (FBS), and RPMI-1640.

Host cells are cultured at suitable temperatures, such as from about 20 °C to about 39 °C, e.g., from 25 °C to about 37 °C. The pH of the medium is generally from about 6.8 to 7.4, e.g., 7.0, depending mainly on the host organism. If an inducible promoter is used in the expression vector of the invention, protein expression is induced under conditions suitable for the activation of the promoter.

Protein recovery typically involves disrupting the host cell, generally by such means as osmotic shock, sonication, or lysis. Once the cells are disrupted, cell debris may be removed by centrifugation or filtration. The proteins may be further purified, for example, by affinity resin chromatography. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin, SDS-PAGE, and gel filtration.

Alternatively, SIRP-α variant constructs can be produced by the cells of a subject (e.g., a human), e.g., in the context of therapy, by administrating a vector (e.g., a retroviral vector, adenoviral vector, poxviral vector (e.g., vaccinia viral vector, such as Modified Vaccinia Ankara (MVA)), adeno-associated viral vector, and alphaviral vector) containing a nucleic acid molecule encoding the SIRP-α variant construct. The vector, once inside a cell of the subject (e.g., by transformation, transfection, electroporation, calcium phosphate precipitation, direct microinjection, infection, etc) will promote expression of the SIRP-α variant construct, which is then secreted from the cell.

## XV. Pharmaceutical compositions and preparations

In some embodiments, pharmaceutical compositions of the invention may contain one or more SIRP-α variant constructs of the invention as the therapeutic proteins. In addition to a therapeutic amount of the protein, the pharmaceutical compositions may contain a pharmaceutically acceptable carrier or excipient, which can be formulated by methods known to those skilled in the art. In other embodiments, pharmaceutical compositions of the invention may contain nucleic acid molecules encoding one or more SIRP-α variant constructs of the invention (e.g., in a vector, such as a viral vector). The nucleic acid molecule encoding a SIRP-α variant construct may be cloned into an appropriate expression vector, which may be delivered via well-known methods in gene therapy.

Acceptable carriers and excipients in the pharmaceutical compositions are nontoxic to recipients at the dosages and concentrations employed. Acceptable carriers and excipients may include buffers such as phosphate, citrate, HEPES, and TAE, antioxidants such as ascorbic acid and methionine, preservatives such as hexamethonium chloride, octadecyldimethylbenzyl ammonium chloride, resorcinol, and benzalkonium chloride, proteins such as human serum albumin, gelatin, dextran, and immunoglobulins, hydrophilic polymers such as polyvinylpyrrolidone, amino acids such as glycine, glutamine, histidine, and lysine, and carbohydrates such as glucose, mannose, sucrose, and sorbitol. Pharmaceutical compositions of the invention can be administered parenterally in the form of an injectable formulation. Pharmaceutical compositions for injection can be formulated using a sterile solution or any pharmaceutically acceptable liquid as a vehicle. Pharmaceutically acceptable vehicles include, but are not limited to, sterile water, physiological saline, and cell culture media (e.g., Dulbecco's Modified Eagle Medium (DMEM), α-Modified Eagles Medium (a-MEM), F-12 medium).

The pharmaceutical compositions of the invention may be prepared in microcapsules, such as hydroxylmethylcellulose or gelatin-microcapsule and poly-(methylmethacrylate) microcapsule. The pharmaceutical compositions of the invention may also be prepared in other drug delivery systems such as liposomes, albumin microspheres, microemulsions, nano-particles, and nanocapsules. Such techniques are described in Remington: The Science and Practice of Pharmacy 20th edition (2000). The pharmaceutical compositions to be used for *in vivo* administration must be sterile. This is readily accomplished by filtration through sterile filtration membranes.

The pharmaceutical compositions of the invention may also be prepared as a sustained-release formulation. Suitable examples of sustained-release preparations include semipermeable matrices of solid hydrophobic polymers containing the SIRP-α variant constructs of the invention. Examples of sustained release matrices include polyesters, hydrogels, polyactides (U.S. Patent No. 3,773,919), copolymers of L-glutamic acid and γ ethyl-L-glutamate, non-degradable ethylene-vinyl acetate, degradable lactic acid-glycolic acid copolymers such as LUPRON DEPOT™, and poly-D-(-)-3-hydroxybutyric acid. Some sustained-release formulations enable release of molecules over a few months, e.g., one to six months, while other formulations release pharmaceutical compositions of the invention for shorter time periods, e.g., days to weeks.

The pharmaceutical composition may be formed in a unit dose form as needed. The amount of an active component, e.g., a SIRP-α variant construct of the invention, included in the pharmaceutical preparations is such that a suitable dose within the designated range is provided (e.g., a dose within the range of 0.01-100 mg/kg of body weight).

The pharmaceutical composition for gene therapy can be in an acceptable diluent, or can comprise a slow release matrix in which the gene delivery vehicle is imbedded. Vectors that may be used as *in vivo* gene delivery vehicle include, but are not limited to, retroviral vectors, adenoviral vectors, poxviral vectors (e.g., vaccinia viral vectors, such as Modified Vaccinia Ankara (MVA)), adeno-associated viral vectors, and alphaviral vectors. In some embodiments, a vector can include internal ribosome entry site (IRES) that allows the expression of multiple SIRP-α variant constructs. Other vehicles and methods for gene delivery are described in U.S. Patent Nos. 5,972,707, 5,697,901, and 6,261,554.

Other methods of producing pharmaceutical compositions are described in, e.g., U.S. Patent Nos. 5,478,925, 8,603,778, 7,662,367, and 7,892,558.

## XVI. Routes, dosage, and timing of administration

Pharmaceutical compositions of the invention that contain one or more SIRP-α variant constructs as the therapeutic proteins may be formulated for parenteral administration, subcutaneous administration, intravenous administration, intramuscular administration, intra-arterial administration, intrathecal administration, or intraperitoneal administration. The pharmaceutical composition may also be formulated for, or administered via, nasal, spray, oral, aerosol, rectal, or vaginal administration. Methods of administering therapeutic proteins are known in the art. See, for example, U.S. Patent Nos. 6,174,529, 6,613,332, 8,518,869, 7,402,155, and 6,591,129, and U.S. Patent Application Publication Nos. US20140051634, WO1993000077, and US20110184145. One or more of these methods may be used to administer a pharmaceutical composition of the invention that contains one or more SIRP-α variant constructs of the invention. For injectable formulations, various effective pharmaceutical carriers are known in the art. See, e.g., Pharmaceutics and Pharmacy Practice, J. B. Lippincott Company, Philadelphia, Pa., Banker and Chalmers, eds., pages 238-250 (1982), and ASHP Handbook on Injectable Drugs, Toissel, 4th ed., pages 622-630 (1986).

The dosage of the pharmaceutical compositions of the invention depends on factors including the route of administration, the disease to be treated, and physical characteristics, e.g., age, weight, general health, of the subject. Typically, the amount of a SIRP-α variant construct of the invention contained within a single dose may be an amount that effectively treats the disease without inducing significant toxicity. A pharmaceutical composition of the invention may include a dosage of a SIRP-α variant construct ranging from 0.001 to 500 mg (e.g., 0.05, 0.01, 0.1, 0.2, 0.3, 0.5, 0.7, 0.8, 1 mg, 2 mg, 3 mg, 4 mg, 5 mg, 10 mg, 15 mg, 20 mg, 30 mg, 50 mg, 100 mg, 250 mg, or 500 mg) and, in a more specific embodiment, about 0.1 to about 100 mg and, in a more specific embodiment, about 0.2 to about 20 mg. The dosage may be adapted by the clinician in accordance with conventional factors such as the extent of the disease and different parameters of the subject.

A pharmaceutical composition of the invention can be administered in an amount from about 0.001 mg up to about 500 mg/kg/day (e.g., 0.05, 0.01, 0.1, 0.2, 0.3, 0.5, 0.7, 0.8, 1 mg, 2 mg, 3 mg, 4 mg, 5 mg, 10 mg, 15 mg, 20 mg, 30 mg, 50 mg, 100 mg, 250 mg, or 500 mg/kg/day). Pharmaceutical compositions of the invention that contain a SIRP-α variant construct may be administered to a subject in need thereof, for example, one or more times (e.g., 1-10 times or more) daily, weekly, monthly, biannually, annually, or as medically necessary. Dosages may be provided in either a single or multiple dosage regimens. For example, in some embodiments, the effective amount is a dose that ranges from about 0.1 to about 100 mg/kg/day, from about 0.2 mg to about 20 mg of the SIRP-α variant construct per day, about 1 mg to about 10 mg of the SIRP-α variant construct per day, from about 0.7 mg to about 210 mg of the SIRP-α variant construct per week, 1.4 mg to about 140 mg of the SIRP-α variant construct per week, about 0.3 mg to about 300 mg of the SIRP-α variant construct every three days, about 0.4 mg to about 40 mg of the SIRP-α variant construct every other day, and about 2 mg to about 20 mg of the SIRP-α variant construct every other day. The timing between administrations may decrease as the medical condition improves or increase as the health of the patient declines.

**XVII. Methods of treatment**

The invention provides SIRP-α variant constructs and pharmaceutical compositions for use in methods of treatment and that may be used to treat patients who are suffering from diseases and disorders associated with SIRP-α and/or CD47 activity, such as cancers and immunological diseases. In some embodiments, the SIRP-α variant constructs described herein may be administered to a subject in a method of increasing phagocytosis of a target cell (e.g., a cancer cell) in the subject. In some embodiments, the SIRP-α variant constructs may be administered to a subject in a method of eliminating regulatory T-cells in the subject. In some embodiments, the SIRP-α variant constructs may be administered to a subject in a method to kill cancer cells in the subject. In some embodiments, the SIRP-α variant constructs may be administered to a subject in a method of treating a disease associated with SIRP-α and/or CD47 activity in the subject, wherein the SIRP-α variant construct preferentially binds CD47 on diseased cells or at a diseased site over CD47 on non-diseased cells. In some embodiments, the SIRP-α variants may be administered to a subject in a method of increasing hematopoietic stem cell engraftment in the subject, wherein the method includes modulating the interaction between SIRP-α and CD47 in the subject. In some embodiments, the SIRP-α variant constructs may be administered to a subject in a method of altering an immune response (i.e., suppressing the immune response) in the subject.

In some embodiments, before treating a disease (e.g., cancer) in a subject, the amino acid sequence(s) of SIRP-α in the subject are determined, for example, from each of the two alleles encoding the SIRP-α gene. In this method of the invention, the method determines the amino acid sequences of SIRP-α polypeptide in a biological sample from the subject, and then administers to the subject a therapeutically effective amount of a SIRP-α variant construct. In this method, the SIRP-α variant in the SIRP-α variant construct has the same amino acid sequence as that of a SIRP-α polypeptide in the biological sample of the subject, except for the amino acids changes introduced to increase affinity of the SIRP-α variant. The SIRP-α variant construct has minimal immunogenicity in the subject after it is administered.

The SIRP-α variant constructs and pharmaceutical compositions of the invention may be used in various cancer therapies. The cancers amenable to treatment according to the invention include, but are not limited to, solid tumor cancer, hematological cancer, acute myeloid leukemia, chronic lymphocytic leukemia, chronic myeloid leukemia, acute lymphoblastic leukemia, non-Hodgkin lymphoma, Hodgkin lymphoma, multiple myeloma, bladder cancer, pancreatic cancer, cervical cancer, endometrial cancer, lung cancer, bronchus cancer, liver cancer, ovarian cancer, colon and rectal cancer, stomach cancer, gastric cancer, gallbladder cancer, gastrointestinal stromal tumor cancer, thyroid cancer, head and neck cancer, oropharyngeal cancer, esophageal cancer, melanoma, non-melanoma skin cancer, Merkel cell carcinoma, virally induced cancer, neuroblastoma, breast cancer, prostate cancer, renal cancer, renal cell cancer, renal pelvis cancer, leukemia, lymphoma, sarcoma, glioma, brain tumor, and carcinoma. In some embodiments, cancerous conditions amenable to treatment according to the invention include metastatic cancers. In some embodiments, the cancer amenable to treatment according to the invention is a solid tumor or hematological cancer.

The SIRP-α variant constructs and pharmaceutical compositions of the invention may be used in various therapies to treat immunological diseases. In some embodiments, the immunological disease is an autoimmune disease or an inflammatory disease, such as multiple sclerosis, rheumatoid arthritis, a spondyloarthropathy, systemic lupus erythematosus, an antibody-mediated inflammatory or autoimmune disease, graft versus host disease, sepsis, diabetes, psoriasis, atherosclerosis, Sjogren's syndrome, progressive systemic sclerosis, scleroderma, acute coronary syndrome, ischemic reperfusion, Crohn's Disease, endometriosis, glomerulonephritis, myasthenia gravis, idiopathic pulmonary fibrosis, asthma, acute respiratory distress syndrome (ARDS), vasculitis, or inflammatory autoimmune myositis.

## EXAMPLES

### Example 1 - Methods

*Production of SIRP-α variant constructs*

All gene constructs are generated using gene synthesis and codon optimized for expression in mammalian cells (DNA2.0). The genes are cloned into mammalian expression vectors and expressed using CMVa-intron promoter. A leader sequence has been engineered at the N-terminus of the constructs to ensure appropriate signaling and processing for secretion. The expression of SIRP-α fusion proteins is carried out using Expi293F™ cells (Life Technologies). This cell line is adapted to high density, serum-free suspension culture in Expi293F™ Expression Medium and is capable of producing high levels of recombinant proteins. Transfection procedures have been performed according to manufacturer's manual. The supernatant is typically harvested at 5-7 days post transfection. The protein constructs are designed to carry a 6xhistidine affinity tag and this allows purification by affinity chromatography. The column was first equilibrated with 5 mM imidazole, 100 mM Tris HCl (pH 8), 500 mM NaCl. The clarified media expressing the various SIRP-α variant constructs is loaded onto Hi-Trap Ni Sepharose excel affinity resin on Avant 25 (GE Healthcare). Another equilibration step is performed. After that, the column is washed with 40 mM imidazole, 100 mM Tris, 500mM NaCl, and subsequently eluted with 250 mM imidazole, 100 mM Tris, 500 mM NaCl. Eluted fractions containing the SIRP-α variant constructs are pooled and thereafter buffer exchanged into 1X PBS.

*In vitro cleavage of SIRP-α proteins*

Recombinant human uPA and matriptase are purchased from R&D systems. 3 μM of SIRP-α proteins are added to respective amounts of uPA and matriptase (0.1 to 44 ng) in 50 mM Tris HCl (pH 8.5), 0.01% Tween as described. The digestion reactions are typically incubated for 18-24 hours at 37 °C. To stop the reaction, SDS-PAGE loading dye is added to the reaction and heated at 95 °C for 3 minutes. To assess cleavage, the digested samples are separated on a 4-20% Tris-Glycine SDS-PAGE.

### Example 2 - Design of SIRP-α variant constructs that will be specifically activated in tumor tissue

The goal is to design SIRP-α variant constructs that will remain inert until activated locally to bind to CD47 in tumor tissue. This will limit binding of SIRP-α to CD47 on the cell-surface of non-diseased cells and prevent undesirable "on-target" "off tissue" toxicity. To generate such SIRP-α variant constructs, the blocking peptides (e.g., a CD47-based blocking peptide) are genetically fused to the SIRP-α variant by way of a cleavable linker. The blocking peptides explored are based on CD47 interaction sites to SIRP-α and the sequences are described below (sections (a)-(c)). Spacers containing repeated units of GGGGS are designed to flank the cleavable linker, which often encodes a protease recognition site. In some embodiments, the protease cleavage site chosen is LSGRSDNH, but many others are possible. The protease cleavage site LSGRSDNH is selected for its sensitivity to numerous proteases that are up-regulated in a variety of human carcinomas, for example, matriptase (MTSP1), urinary-type plasminogen activator (uPA), legumain, PSA (also called KLK3, kallikrein-related peptidase-3), matrix metalloproteinase-2 (MMP-2), MMP9, human neutrophil elastase (HNE), and proteinase 3 (Pr3) (Ulisse et al., Curr. Cancer Drug Targets 9:32-71, 2009; Uhland et al., Cell. Mol. Life Sci. 63:2968-2978, 2006; LeBeau et al., Proc. Natl. Acad. Sci. USA 110:93-98, 2013; Liu et al., Cancer Res. 63:2957-2964, 2003).

*(a) Blocking SIRP-α by CD47-based blocking peptides*

CD47-based blocking peptides are described previously. These peptides bind SIRP-α with different affinities and block its function. The N-terminus of CD47 is important for the interaction with SIRP-α, therefore, structural analysis predicted fusing SIRP-α to the C-terminus of CD47. To better understand the results, both N-terminal and C-terminal fusions are explored with different lengths of spacers. Different CD-47 based blocking peptides (e.g., peptides listed in Table 6) are fused to the N- or C-terminus of a SIRP-α variant with a cleavable linker and one or more spacers. Exemplary sequences of fusion proteins containing a CD47-based blocking peptide fused to a SIRP-α variant by way of a cleavable linker and one or more spacers are shown in sequences of SEQ ID NOs: 48-56, in which single-underlined portion indicates the CD47-based blocking peptide, double-underlined portion indicates the cleavable linker, and bold portion indicates the SIRP-α variant. Sequences of SEQ ID NOs: 48-51 contain CD47-based blocking peptides that include 12 or 21 amino acids and spacers of 2-3 repeats of GGGGS. Sequences of SEQ ID NOs: 52-56 contain CD47-based blocking peptides that include the CD47 IgSF domain (truncated at VVS) having a C15S substitution and spacers of 2-5 repeats of GGGGS or 3-6 repeats of GGS. Additionally, in some embodiments, an HSA may be fused to the C-terminus

of any one of the sequences of SEQ ID NOs: 48-56. Furthermore, in some embodiments, an Fc domain monomer or an HSA (SEQ ID NO: 68) may be fused to either the N- or C-terminus of any one of the fusion proteins listed in Table 10.

Table 10

| SEQ ID NO | Fusion protein of a CD47-based blocking peptide and a SIRP-α variant |
|---|---|
| 48 | SEVTELTREGETGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 49 | SEVTELTREGETGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 50 | GQYTSEVTELTREGETIIELKGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 51 | GQYTSEVTELTREGETIIELKGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 52 | QLLFNKTKSVEFTF**S**NDTVVIPCFVTNMEAQNTTEVYVKWKFKGRDIYTFDGALNKSTVPTDFSSAKIEVSQLLKGDASLKMDKSDAVSHTGNYTCEVTELTREGETIIELKYRVVSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 53 | QLLFNKTKSVEFTF**S**NDTVVIPCFVTNMEAQNTTEVYVKWKFKGRDIYTFDGALNKSTVPTDFSSAKIEVSQLLKGDASLKMDKSDAVSHTGNYTCEVTELTREGETIIELKYRVVSGGGGSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 54 | QLLFNKTKSVEFTF**S**NDTVVIPCFVTNMEAQNTTEVYVKWKFKGRDIYTFDGALNKSTVPTDFSSAKIEVSQLLKGDASLKMDKSDAVSHTGNYTCEVTELTREGETIIELKYRVVSGGGGSGGGGSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 55 | **EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS**GGSGGSGGSLSGRSDNHGGSGGSGGSGGSQLLFNKTKSVEFTF**S**NDTVVIPCFVTNMEAQNTTEVYVKWKFKGRDIYTFDGALNKSTVPTDFSSAKIEVSQLLKGDASLKMDKSDAVSHTGNYTCEVTELTREGETIIELKYRVVS |
| 56 | **EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS**GGSGGSGGSGGSLSGRSDNHGGSGGSGGSGGSGGSGGSQLLFNKTKSVEFTF**S**NDTVVIPCFVTNMEAQNTTEVYVKWKFKGRDIYTFDGALNKSTVPTDFSSAKIEVSQLLKGDASLKMDKSDAVSHTGNYTCEVTELTREGETIIELKYRVVS |

*(b) Blocking SIRP-α variant by a low affinity CD47 mutant having an extended N-terminus*

Due to the high affinity of the SIRP-α variant for CD47, there is a chance that after cleaving the linker, the fusion protein may not dissociate and the SIRP-α variant may remain blocked. To solve this, we studied the structure of the SIRP-α-CD47 complex and designed CD47 mutants with reduced binding affinity for the SIRP-α variant relative to the binding affinity for a wild-type SIRP-α. Thus, after protease cleavage of the linker, the CD47 mutant will dissociate away from the SIRP-α variant. The designed CD47 mutants are described below. Initial experiments will be performed fusing the SIRP-α variant to the wild-type CD47. These SIRP-α variant constructs including a SIRP-α variant and a wild-type CD47 or CD47 mutant will be cleaved *in vitro,* analyzed by SDS-page to ensure cleavage, and measured by biacore to see their capacity of binding to CD47 (i.e., the binding of the SIRP-α variant to wild-type CD47 after the CD47 mutant is dissociated from the SIRP-α variant). If the initial SIRP-α variant constructs containing a SIRP-α variant fused to the wild-type CD47 are expressed, able to block CD47 binding before protease cleavage, and able to bind CD47 after protease cleavage, CD47 mutants may not be needed. If these initial SIRP-α variant constructs are inactive (i.e., can be cleaved but do not bind CD47 after protease cleavage due to the lack of dissociation), then other fusion proteins containing a SIRP-α variant fused to the low affinity CD47 mutants will be tested.

In the co-crystalized structure of CD47:SIRP-α (PDB: 4KJY, 4CMM), the N-terminus of CD47 exists as a pyroglutamate and makes hydrogen bonding interactions with Thr66 of a SIRP-α variant and Leu66 of a wild-type SIRP-α (FIG. 1). It is hypothesized that extending the N-terminus of CD47 by adding an amino acid, e.g., a glycine, will prevent cyclization of glutamine to pyroglutamate and also create other unwanted contacts and interactions that will likely will disrupt the hydrogen bonding interactions with Thr66 or Leu66, and therefore perturb binding of CD47 to SIPR-α. Sequences of the fusion proteins containing a low affinity CD47 IgSF domain mutant and a SIRP-α variant are shown in SEQ ID NOs: 57-59 in Table 11, in which single-underlined portion indicates the low affinity CD47 IgSF domain mutant containing amino acids 1-118 and C15S, relative to SEQ ID NO: 46 in Table 6, double-underlined portion indicates the cleavable linker, and bold portion indicates the SIRP-α variant. SEQ ID NOs: x10-x12 also include spacers of 3-5 repeats of GGGGS. Sequences similar to SEQ ID NOs: x10-x12 may be designed and expressed in which a low affinity CD47 IgSF domain mutant is fused to the C-terminus of a SIRP-α variant by way of a cleavable linker and one or more spacers.

Table 11

| SEQ ID NO | Fusion protein of a low affinity CD47 IgSF domain mutant having an extended N-terminal glycine and a SIRP-α variant |
|---|---|
| 57 | **G**QLLFNKTKSVEFTF**S**NDTVVIPCFVTNMEAQNTTEVYVKWKFKGRDIYTFDGALNKSTVPTDFSSAKIEVSQLLKGDASLKMDKSDAVSHTGNYTCEVTELTREGETIIELKYRVVSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 58 | **G**QLLFNKTKSVEFTF**S**NDTVVIPCFVTNMEAQNTTEVYVKWKFKGRDIYTFDGALNKSTVPTDFSSAKIEVSQLLKGDASLKMDKSDAVSHTGNYTCEVTELTREGETIIELKYRVVSGGGGSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 59 | **G**QLLFNKTKSVEFTF**S**NDTVVIPCFVTNMEAQNTTEVYVKWKFKGRDIYTFDGALNKSTVPTDFSSAKIEVSQLLKGDASLKMDKSDAVSHTGNYTCEVTELTREGETIIELKYRVVSGGGGSGGGGSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |

*(c) Blocking SIRP-α by a low-affinity CD47 IgSF domain mutant having amino acid substitutions*

CD47 binds to a deep pocket to SIRP-α (PDB code: 4KJY and 4CMM). Computational modeling has been performed to identify amino acid residues in the pocket region of CD47, which, when mutated would reduce the binding affinity of CD47 to SIRP-α variant, but maintain the binding affinity of CD47 to a wild-type SIPR-α. The CD47 residues identified are L101Q, L101H, L101Y, T102Q, and T102H. It is hypothesized that a low-affinity CD47 IgSF domain mutant containing one of these substitutions will be able to block the SIRP-α variant efficiently in the tethered mode. However, upon reaching the tumor site and cleavage by proteases at the linker locally, the low-affinity CD47 IgSF domain mutant will dissociate from the SIRP-α variant to bind to a wild-type SIRP-α, leaving the SIRP-α variant free to bind CD47 on the cell-surface of tumor cells. The dissociated, low-affinity CD47 IgSF domain mutant can now block activity of wild-type SIRP-α. This will potentially result in enhanced double-blocking activity from the released low-affinity CD47 IgSF domain mutant and the SIRP-α variant. To illustrate how the amino acid residues are selected and the principle of how this may result in differential blocking of wild-type SIRP-α and SIRP-α variant, an example is shown using Ala27 of a wild-type SIRP-α (FIG. 2). For instance, Ala27 of the wild-type SIRP-α is a smaller residue than Ile27 in the SIRP-α variant. Therefore, by mutating Thr102 in the wild-type CD47 to a larger amino acid such as Gln102, Gln102 in the low-affinity CD47 IgSF domain mutant may result in a steric clash with Ile27 in the SIRP-α variant at the corresponding interaction site. However, the interaction between the CD47 mutant having Thr102Gln substitution and the wild-type SIRP-α having Ala27 would be preserved. Accordingly, the CD47 mutant would have a low binding affinity to the SIRP-α variant and a relatively higher binding affinity to the wild-type SIRP-α. Sequences of some exemplary low-affinity CD47 IgSF domain mutants are shown in SEQ ID NOs: 41-45 in Table 6. Sequences of the SIRP-α variant constructs containing a low affinity CD47 IgSF domain mutant having amino acid substitutions and a SIRP-α variant are shown in SEQ ID NOs: 60-63 in Table 12, in which single-underlined portion indicates the low affinity CD47 IgSF domain mutant containing amino acid substitution L101Q, L101Y, T102Q, and T102H, respectively, double-underlined portion indicates the cleavable linker, and bold portion indicates the SIRP-α variant. SEQ ID NOs: 60-63 also include spacers of 3-5 repeats of GGGGS. Sequences similar to SEQ ID NOs: 60-63 may be designed and expressed in which a low affinity CD47 IgSF domain mutant is fused to the C-terminus of a SIRP-α variant by way of a cleavable linker and one or more spacers.

Table 12

| SEQ ID NO | Fusion protein of a low affinity CD47 IgSF domain mutant having amino acid substitutions and a SIRP-α variant |
|---|---|
| 60 | QLLFNKTKSVEFTFSNDTVVIPCFVTNMEAQNTTEVYVKWKFKGRDIYTFDGALNKSTVPTDFSSAKIEVSQLLKGDASLKMDKSDAVSHTGNYTCEVTEQTREGETIIELKYRVVSGGGGSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 61 | QLLFNKTKSVEFTFSNDTVVIPCFVTNMEAQNTTEVYVKWKFKGRDIYTFDGALNKSTVPTDFSSAKIEVSQLLKGDASLKMDKSDAVSHTGNYTCEVTEYTREGETIIELKYRVVSGGGGSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 62 | QLLFNKTKSVEFTFSNDTVVIPCFVTNMEAQNTTEVYVKWKFKGRDIYTFDGALNKSTVPTDFSSAKIEVSQLLKGDASLKMDKSDAVSHTGNYTCEVTELQREGETIIELKYRVVSGGGGSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |
| 63 | QLLFNKTKSVEFTFSNDTVVIPCFVTNMEAQNTTEVYVKWKFKGRDIYTFDGALNKSTVPTDFSSAKIEVSQLLKGDASLKMDKSDAVSHTGNYTCEVTELHREGETIIELKYRVVSGGGGSGGGGSGGGGSGGGGSLSGRSDNHGGGGSGGGGS**EEELQIIQPDKSVLVAAGETATLRCTITSLFPVGPIQWFRGAGPGRVLIYNQRQGPFPRVTTVSDTTKRNNMDFSIRIGNITPADAGTYYCIKFRKGSPDDVEFKSGAGTELSVRAKPS** |

## Example 3 - Expression and production of SIRP-α variant constructs for *in vitro* studies

Various SIRP-α variant constructs (SEQ ID NOs: 48-56) including a SIRP-α variant and a CD47-based blocking peptide were expressed in Expi293-F mammalian cells. All the constructs were designed with a leader sequence that enabled their expression as secreted proteins into the media. All constructs were expressed in the soluble form and purified using one-step IMAC isolation to high degree of purity (FIGs. 3A and 3B). FIG. 3A shows a reduced, SDS-PAGE gel of SIRP-α variant constructs of SEQ ID NOs: 48-56 and FIG. 3B shows a non-reduced, SDS-PAGE gel of the SIRP-α variant constructs. Size exclusion data indicated that the SIRP-α variant constructs are not aggregated (data not shown).

## Example 4 *- In vitro* cleavage of SIRP-α and CD47 fusion proteins

To determine whether the SIRP-α variant constructs (e.g., SEQ ID NOs: 48-63) would be cleaved specifically *in vivo* at tumor tissues, *in vitro* experiments were performed using proteases uPA and matriptase, which are commonly known to be up-regulated in cancers, to cleave the SIRP-α variant constructs. Initial experiments were performed using SIRP-α variant construct (SEQ ID NO: 54) to determine protease cleavability and optimize cleavage conditions. FIG. 4A shows the results of testing cleavability by uPA and matriptase. 3 μM SIRP-α variant construct (SEQ ID NO: 54) was incubated for 18 hrs at 37 °C using excess uPA or matriptase. Lane 1 of FIG. 4A shows the control experiment with no addition of protease and lanes 2 and 3 show incubation of the SIRP-α variant construct (SEQ ID NO: 54) with uPA and matriptase, respectively. Data obtained as shown in FIG. 4A clearly demonstrate that the SIRP-α variant construct (SEQ ID No: 54) can be cleaved and released *in vitro* by digesting the SIRP-α variant construct with excess uPA and matriptase for 18 hrs at 37 °C. The cleaved SIRP-α variant migrates as a ~17 KDa molecular weight band. Cleaved CD47 migrates with smeary banding, most likely due to glycosylation, around 36-40 kDa. By comparing the amount of uncut SIRP-α variant construct in lanes 2 and 3 of FIG. 4A, it appears that a more complete cleavage was achieved using matriptase compared to using uPA.

Therefore further optimization of cleavage conditions was performed only using matriptase and the results are shown in FIG. 4B. Different amounts of matriptase were tested and the cleavage was performed for 18 hrs at 37 °C. Lane 1 of FIG. 4B shows the control experiment with no addition of matriptase and lanes 2-4 each shows cleavage performed with 44 ng, 0.44 ng, and 0.167 ng of matriptase, respectively. The data obtained indicate that 0.44 ng enzyme is sufficient for complete cleavage under current conditions. Next, we tested cleavage of remaining SIRP-α variant constructs using the optimized cleavage conditions. As an example and shown in FIG. 4C, respective SIRP-α variant constructs (SEQ ID NOs: 57-63) were cleaved successfully *in vitro* by matriptase using the optimized cleavage conditions. Lanes 1-7 of FIG. 4C correspond to the uncleaved fusion proteins of SEQ ID NOs: 57-63, respectively. Lanes 8-14 of FIG. 4C correspond to the fusion proteins of SEQ ID NOs: 57-63, respective, cleaved with matriptase.

## Example 5 - Binding affinities of SIRP-α variant constructs

Binding of human CD47-hFc (R & D Systems, catalog number 4670-CD) to SIRP-α variant constructs was analyzed on a Biacore T100 instrument (GE Healthcare) using phosphate buffered saline (pH 7.4) supplemented with 0.01% Tween-20 as running buffer.

370 Resonance Unit (RU) of CD47-hFc were immobilized on flow cell 2 of a CM4 sensor chip (GE Healthcare) by standard amine coupling. Flow cell 1 was activated with EDC/NHS and blocked (with ethanolamine) to serve as a reference. All SIRP-α variant constructs were injected at 50 nM or 100 nM for two minutes at a flow rate of 30 μL/min and followed by ten minutes of dissociation time. After each injection, the surface was regenerated using a 2:1 mixture of Pierce IgG elution buffer (Life Technologies, catalog number 21004) and 4 M NaCl. Complete regeneration of the surface was confirmed by injecting the SIRP-α variants at the beginning and end of the experiment. All sensorgrams were double-referenced using flow cell 1 and a buffer injection.

For all samples, the binding signal at 100 nM after 50 seconds of association was determined, normalized by the molecular weight of the SIRP-α variant construct, and expressed as percent of maximal binding response. The binding of the SIRP-α variant (SEQ ID NO: 31) at 100 nM normalized by its MW was used as maximal binding response. Results in FIG. 5A show that SIRP-α variant constructs (SEQ ID NOs: 48-51) do not block SIRP-α variants from binding to CD47 on the chip. After cleavage of the linker, the binding activity modestly increases. SIRP-α variant constructs (SEQ ID NOs: 52-54) efficiently blocked binding of the SIRP-α variant to CD47 on the chip. However, after cleavage of the linker, the binding activity of the SIRP-α variant to CD47 on the chip only modestly increased, suggesting that the high affinity of interaction between the SIRP-α variant and the IgSF domain of CD47 keeps the complex together and therefore the IgSF domain of CD47 continues block the SIRP-α variant even after the linker is cleaved. Surprisingly, when the IgSF domain of CD47 is fused to the C-terminus of SIRP-α (SEQ ID NO: 55), the intact SIRP-α variant construct is efficiently blocked from binding to CD47 on the chip (same as fusion proteins of SEQ ID NOs: 52-54), but cleavage of the linker restores 100% of the binding of the SIRP-α variant to CD47 on the chip, suggesting that the IgSF domain

of CD47 dissociated from the SIRP-α variant after linker cleavage, thus, the SIRP-α variant is free to bind to CD47 on the chip. Another construct with CD47 fused to the C-terminus of a SIRP-α variant was tested later (see FIG. 5B). This construct (SEQ ID NO: 56), which contains a longer spacer, also recovered activity after cleaving, confirming the general approach of linking the N-terminus of a CD47-based blocking peptide to the C-terminus of a SIRP-α variant obtain SIRP-α constructs in which the CD47-based blocking peptide efficiently blocks the SIRP-α variant and dissociates after cleaving of the cleavable linker.

To further examine the binding affinity of SIRP-α variant constructs, SIRP-α variant constructs of SEQ ID NOs: 52-63 were analyzed on the Biacore instrument following the same protocol described previously. SIRP-α variant constructs of SEQ ID NOs: 52-54 contain the CD47 IgSF domain having amino acids 1-117 and C15S, relative to wild-type CD47 (SEQ ID NO: 35) fused to the N-terminus of the SIRP-α variant (SEQ ID NO: 31) through the cleavable linker LSGRSDNH and multiple spacers of different lengths. SIRP-α variant constructs of SEQ ID NOs: 55 and 56 contain the CD47 IgSF domain having amino acids 1-117 and C15S, relative to wild-type CD47 (SEQ ID NO: 35) fused to the C-terminus of the SIRP-α variant (SEQ ID NO: 31) through the cleavable linker LSGRSDNH and multiple spacers of different lengths. SIRP-α variant constructs of SEQ ID NOs: 57-59 contain the CD47 IgSF domain having amino acids 1-118 and C15S, relative to SEQ ID NO: 46 in Table 6 fused to the N-terminus of the SIRP-α variant (SEQ ID NO: 31) through the cleavable linker LSGRSDNH and multiple spacers of different lengths. SIRP-α variant constructs of SEQ ID NOs: 60-63 contain the CD47 IgSF domain having amino acids 1-117 of SEQ ID NOs: 41, 42, 44, and 45 in Table 6, respectively, fused to the N-terminus of the SIRP-α variant (SEQ ID NO: 31) through the cleavable linker LSGRSDNH and multiple spacers of different lengths.

FIG. 5B shows that SIRP-α variant constructs of SEQ ID NOs: 55 and 56 were efficiently blocked from binding to CD47 on the chip before linker cleavage, but cleavage of the linker restores 100% of the binding activity. Similar results were observed for SIRP-α variant constructs of SEQ ID NO: 57-63. We observed that by extending the N-terminus of the CD47-based blocking peptide by one glycine residue generated a SIRP-α variant construct that was efficiently blocked before the linker was cleaved and subsequently recovered close to 100% of the CD47 binding activity after protease treatment (SEQ ID NOs: 57-59), demonstrating dissociation of the CD47-based blocking peptide from the SIRP-α variant after linker cleavage.

This result suggests SIRP-α variant fused to the N-terminus of CD47-based blocking peptide through a cleavable linker and spacers works well. The cleavable linker stabilizes the fusion complex and once cleaved, the extended N-terminus of the CD47-based blocking peptide, which includes a fragment of the cleavable linker attached to the N-terminus of the CD47-based blocking peptide, prevents binding of the CD47-based blocking peptide to the SIRP-α variant. The same effect is obtained by fusing a CD47-based blocking peptide having one or more amino acid additions, e.g., one glycine addition (e.g., sequences of SEQ ID NO: 46 in Table 6), at the N-terminus of the CD47-blocking peptide to the C-terminus of a SIRP-α variant by way of a cleavable linker and one or more spacers. This same effect is also observed by fusing a CD47-based blocking peptide having one or more amino acid substitutions, e.g., L101Q, L101Y, L101H, T102Q, or T102H (e.g., sequences of SEQ ID NOs: 41-45 in Table 6), to the C-terminus of a SIRP-α variant by way of a cleavable linker and one or more spacers. We have demonstrated that CD47-based blocking peptides can be fused to the C-terminus of a SIRP-α variant and can block SIRP-α variant binding to CD47 before linker cleavage and release SIRP-α variant after linker cleavage (see, e.g., SEQ ID NO: 55 in FIG. 5A, and SEQ ID NOs: 55 and 56 in FIG. 5B).

Based on this information, we can create fusion proteins of CD47-blocked SIRP-α variants, i.e., fusing an Fc domain monomer or HSA to a SIRP-α variant, by choosing the orientation (e.g., N- or C-terminal fusion) that gives better results in pharmacokinetics, efficacy, safety, production, and stability of the product.

**Example 6** - **Specific targeting of SIRP-α variants through antibody-binding peptide**

First, we used Cetuximab, which is known to contain a binding site for the DLP having the sequence of SEQ ID NO: 64, to check if the SIRP-α variant construct including a SIRP-α variant and the DLP is able to concentrate on bound antibody. We immobilized Cetuximab using EDC/NHS chemistry on a CM4 biacore chip (2000RU) and flowed the SIRP-α variant construct (SEQ ID NO: 66) at 100 nM and 50 nM using PBS 0.01% P20 as running and sample buffer at 30 μL/min onto the chip (biacore T100). FIG: 6 shows the binding of the SIRP-α variant construct, but not the SIRP-α variant alone, onto the chip. We then injected CD47-ECD and saw binding of CD47 in the case where the SIRP-α variant construct was used, demonstrating that the SIRP-α variant construct can bind EGFR and CD47 simultaneously (FIG. 6). Therefore, the SIRP-α variant construct including a SIRP-α variant and a DLP injected to a cancer patient would concentrate at the site where the therapeutic antibody (e.g., Cetuximab) accumulates, increasing efficacy and reducing toxicity.

Secondly, we demonstrated that the SIRP-α variant construct including a SIRP-α variant and a DLP can first bind Cetuximab that is bound to EGFR, and then bind CD47. A scheme of the binding complex is shown in FIG. 7A. We immobilized 3000 RUs of hrEGFR-Fc (R&D Systems) to a CM4 chip using EDC/NHS chemistry. Using PBS 0.01% P20 as sample and running buffer at 30 μL/min (biacore T100), we injected different concentrations (4, 20, and 100 nM) of

Cetuximab. Binding of Cetuximab to the immobilized hrEGFR-Fc was observed. We then injected the SIRP-α variant construct (SEQ ID NO: 66) at 100 nM and observed binding. Binding was not observed when the SIRP-α variant was injected alone. We then injected CD47-ECD at 100 nM and observed binding. The data is shown in FIGs. 7B and 7C. Therefore, we demonstrated that the formation of the quaternary complex EGFR-Cetuximab-SIRP-α variant construct of SEQ ID NO: 66-CD47 is possible. The SIRP-α variant construct including a SIRP-α variant and a DLP is able to bind and inhibit CD47 when the construct pre-concentrates at the diseased site by binding specifically to a tumor-specific antibody (e.g., Cetuximab). Furthermore, following the same concept, a SIRP-α variant construct including a SIRP-α variant, a DLP, and a CD47-based blocking peptide is also able to bind and inhibit CD47 when the construct pre-concentrates at the diseased site by binding specifically to a tumor-specific antibody (e.g., Cetuximab).

**Example 7 - Phagocytosis assay**

SIRP-α variant construct (SEQ ID NO: 66), which includes a SIRP-α variant attached to DLPs through spacers, and a SIRP-α variant (SEQ ID NO: 31) were tested in a phagocytosis assay on DLD1 cells (FIG. 8). Phagocytosis assay was performed as described in, e.g., Weiskofp et al, Science 341:88-91, 2013. An experimental protocol is described below. The SIRP-α variant construct (SEQ ID NO: 66) showed higher potency than the SIRP-α variant alone (SEQ ID NO: 31), presumably due to higher accumulation on disease cells.

Buffy coats were obtained from the Stanford Blood Center from anonymous donors, and peripheral blood mononuclear cells were enriched by density gradient centrifugation over Ficoll-Paque Premium (GE Healthcare). Monocytes were purified using Macs Miltenyi Biotec Monocyte Isolation Kit II according to the manufacturer's instructions. This is an indirect magnetic labeling system for the isolation of monocytes from human PBMCs. The isolated monocytes are differentiated into macrophages by culturing in RPMI 1640 media supplemented with 10% heat-inactivated human AB serum and 1% GlutaMax and 1% penicillin and streptomycin (GIBCO Life Technologies) for 6-10 days. For phagocytosis assay, 100,000 GFP+ DLD-1 cells are plated onto wells of Ultra low attachment U bottom 96 well plate (Corning 7007). 50 μL/well of either 4 μg/ml IgG1k isotype control or 4 μg/ml Cetuximab (Absolute Antibody, Ab00279-10.0) are added to DLD-1 tumor cells and pre-incubated for 30 minutes at room temp. After that, 50 μL/well SIRP-a variants are added and 50 μL/well macrophages (1 x $10^6$/ml) (50,000 macrophages) are also added to each well. Final dilution of antibodies and SIRP-α construct samples is 1:4. Cetuximab final concentration is 1μg/ml. The co-culturing of macrophages, tumor cells, antibodies and SIRP-a variant constructs are carried out for 2 hours at 37 °C. For analysis, cell samples were fixed, stained and analyzed by BD FACS Canto. Primary human macrophages were identified by flow cytometry using anti-CD14, anti-CD45, or anti-CD206 antibodies (BioLegend). Dead cells were excluded from the analysis by staining with DAPI (Sigma). Phagocytosis was evaluated as the percentage of GFP+ macrophages and normalized to the maximal response by each independent donor against each cell line.

**Example 8 - Modeling pH dependent binding of SIRP-α variants to CD47**

To engineer pH-dependent binding of a SIRP-α variant of the invention, histidine mutagenesis may be performed on the SIRP-α, especially on the region of SIRP-α that interacts with CD47. Crystal structures of a SIRP-α and CD47 complex (see, e.g., PDB ID No. 2JJS) and computer modeling may be used to visualize the three-dimensional binding site of SIRP-α and CD47. Computational design and modeling methods useful in designing a protein with pH-sensitive binding properties are known in the literature and described in, e.g., Strauch et al., Proc Natl Acad Sci 111:675-80, 2014, which is incorporated by reference herein in its entirety. In some embodiments, computer modeling may be used to identify key contact residues at the interface of SIRP-α and CD47. Identified key contact residues may be substituted with histidine residues using available protein design software (e.g., RosettaDesign), which can generate various protein designs that can be optimized, filtered, and ranked based on computed binding energy and shape complementarity. Therefore, energetically favorable histidine substitutions at certain amino acid positions may be identified using computational design methods. Computer modeling may be also be used to predict the change in the three-dimensional structure of SIRP-α. Histidine substitutions that generate a significant change in the three-dimensional structure of SIRP-α may be avoided.

Once energetically and structurally optimal amino acid substitutions are identified, the amino acids may be systematically substituted with histidine residues. In some embodiments, one or more (e.g., one, two, three, four, five, six, seven, eight, nine, ten, etc, with a maximum of 20) amino acids of SIRP-α may be substituted with histidine residues. In particular, amino acids located at the interface of SIRP-α and CD47, preferably, amino acids directly involved in the binding of SIRP-α to CD47, may be substituted with histidine residues. The SIRP-α variants of the invention may include one or more (e.g., one, two, three, four, five, six, seven, eight, nine, ten, etc, with a maximum of 20) histidine residue substitutions. In other embodiments, naturally occurring histidine residues of SIRP-α may be substituted with other amino acid residues. In yet other embodiments, one or more amino acids of SIRP-α may be substituted with non-histidine residues in order to affect the binding of naturally occurring or substituted histidine residues with CD47. For example,

substituting amino acids surrounding a naturally occurring histidine residue with other amino acids may "bury" the naturally occurring histidine residue. In some embodiments, amino acids not directly involved in binding with CD47, i.e., internal amino acids (e.g., amino acids located at the core of SIRP-α) may also be substituted with histidine residues. Table 4 lists specific SIRP-α amino acids that may be substituted with histidine residues. Contact residues are the amino acids located at the interface of SIRP-α and CD47. Core residues are the internal amino acids not directly involved in the binding between SIRP-α and CD47. The SIRP-α variants of the invention may include one or more (e.g., one, two, three, four, five, six, seven, eight, nine, ten, etc, or all) of the substitutions listed in Table 4.

**Example 9 - Generating and screening SIRP-α variants with pH-dependent binding to CD47**

The SIRP-α variants containing one or more (e.g., one, two, three, four, five, six, seven, eight, nine, ten, etc, with a maximum of 20) substitutions of amino acids with histidine residues may be generated using conventional molecular cloning and protein expression techniques. A nucleic acid molecule encoding a SIRP-α variant of the invention may be cloned into a vector optimized for expression in bacteria using well known molecular biology techniques. The vector can then be transformed into bacteria cells (e.g., *E. coli* cells), which may be grown to optimal density prior to protein expression induction. After protein expression induction (i.e., using IPTG), bacterial cells may be allowed to grow for an additional 24 hours. Cells can be collected and the expressed SIRP-α variant protein may be purified from the cell culture supernatant using, e.g., affinity column chromatography. Purified SIRP-α variant may be analyzed by SDS-PAGE followed by Coomassie Blue staining to confirm the presence of protein bands of expected size.

Purified SIRP-α variants may be screened for pH-dependent binding to CD47 using available techniques in the art, such as phage display, yeast display, surface plasmon resonance, scintillation proximity assays, ELISA, ORIGEN immunoassay (IGEN), fluorescence quenching, and/or fluorescence transfer. Binding may also be screened using a suitable bioassay. The desired SIRP-α variant binds with higher affinity to CD47 under acidic pH (e.g., less than pH 7 (e.g., pH 6)) than under neutral pH (e.g., pH 7.4). The $K_D$ of a SIRP-α/CD47 complex at pH 6 would be lower than $K_D$ of a SIRP-α/CD47 complex at pH 7.4.

**Example 10** - **Testing SIRP-α variants with pH-dependent binding to CD47 in mice**

Genetically engineered mouse models of various cancers, e.g., solid tumor and hematological cancer, may be used to test the pH-dependent binding of SIRP-α variants of the invention to CD47 at a diseased site in a mouse model. A SIRP-α variant may be injected directly or indirectly to the diseased site in a mouse, which may be dissected at the later time to detect the presence of the complex of SIRP-α variant and CD47 at the diseased site. Antibodies specific to SIRP-α variant or CD47 may be used in the detection.

## Claims

**1.** A signal-regulatory protein $\alpha$ (SIRP-$\alpha$) variant construct, comprising a SIRP-$\alpha$ variant attached to a CD47-based blocking peptide by way of a cleavable linker,
wherein the CD47-based blocking peptide has at least 80% sequence identity to a sequence of wild-type, IgSF domain of CD47 (SEQ ID NO: 35) or a fragment thereof that can bind a SIRP-$\alpha$ variant, and
wherein the SIRP-$\alpha$ variant comprises the amino acid sequence set forth in $EEEX_1QX_2IQPDKSVLVAAGETX_3TLRCTX_4TSLX_5VGPIQWFRGAGPGRX_6LIYNQX_7X_8$ $GX_9FPRVTTVSDX_{10}TX_{11}RNNMDFSIRIGX_{12}ITX_{13}ADAGTYYCX_{14}KX_{15}RKGSPDDVEX_{16}$ KSGAGTELSVRAKPS (SEQ ID NO: 13), wherein $X_1$ is L, I, or V; $X_2$ is V, L, or, I; $X_3$ is A or V; $X_4$ is A, I, or L; $X_5$ is I, T, S, or F; $X_6$ is E, V, or L; $X_7$ is K or R; $X_8$ is E or Q; $X_9$ is H, P, or R; $X_{10}$ is L, T, or G; $X_{11}$ is K or R; $X_{12}$ is N, A, C, D, E, F, G, H, I, K, L, M, P, Q, R, S, T, V, W, or Y; $X_{13}$ is P, A, C, D, E, F, G, H, I, K, L, M, N, Q, R, S, T, V, W, or Y; $X_{14}$ is V or I; $X_{15}$ is F, L, or V; and $X_{16}$ is F or V.

**2.** The SIRP-$\alpha$ variant construct of claim 1, wherein the SIRP-$\alpha$ variant has at least 80% sequence identity to a sequence according to any one of SEQ ID NOs: 3-12 and 24-34.

**3.** The SIRP-$\alpha$ variant construct of claim 1 or claim 2, wherein the CD47-based blocking peptide has a sequence according to any one of SEQ ID NOs: 36-46.

**4.** The SIRP-$\alpha$ variant construct of any one of claims 1-3, wherein the cleavable linker comprises one or more spacers.

**5.** The SIRP-$\alpha$ variant construct of any one of claims 1-4, wherein the cleavable linker is cleavable under acidic pH and/or hypoxic condition.

**6.** The SIRP-$\alpha$ variant construct of any one of claims 1-5, wherein the cleavable linker is cleavable by a tumor-associated enzyme, optionally wherein the tumor associated enzyme is a protease selected from the group consisting of matriptase (MTSP1), urinary-type plasminogen activator (uPA), legumain, PSA (also called KLK3, kallikrein-related peptidase-3), matrix metalloproteinase-2 (MMP-2), MMP9, human neutrophil elastase (HNE), and proteinase 3 (Pr3), preferably matriptase (MTSP1).

**7.** The SIRP-$\alpha$ variant construct of any one of claims 1-4, wherein the cleavable linker comprises a sequence set forth in any one of SEQ ID NOs: 47 or 69-99, preferably LSGRSDNH (SEQ ID NO: 47).

**8.** The SIRP-$\alpha$ variant construct of any one of claims 1-7, wherein the SIRP-$\alpha$ variant construct has a sequence according to any one of SEQ ID NOs: 48-63.

**9.** The SIRP-$\alpha$ variant construct of any one of claims 1-8, wherein the SIRP-$\alpha$ variant is attached to an antibody-binding peptide, optionally wherein the antibody-binding peptide has at least 75% amino acid sequence identity to the sequence of a disease localization peptide (DLP) (SEQ ID NO: 64 or 65) or a fragment thereof.

10. The SIRP-α variant construct of any one of claims 1-9, wherein said SIRP-α variant is attached to an Fc domain monomer.

11. A pharmaceutical composition, comprising: a) a therapeutically effective amount of the SIRP-α variant construct of any one of claims 1-10, and (b) a pharmaceutically acceptable excipient.

12. The SIRP-α variant construct of any one of claims 1-10 or the pharmaceutical composition of claim 11, for use as a medicament.

13. The SIRP-α variant construct of any one of claims 1-10 or the pharmaceutical composition of claim 11, for use in the treatment of a cancer, optionally wherein said cancer is selected from solid tumor cancer, hematological cancer, acute myeloid leukemia, chronic lymphocytic leukemia, chronic myeloid leukemia, acute lymphoblastic leukemia, non-Hodgkin lymphoma, Hodgkin lymphoma, multiple myeloma, bladder cancer, pancreatic cancer, cervical cancer, endometrial cancer, lung cancer, bronchus cancer, liver cancer, ovarian cancer, colon and rectal cancer, stomach cancer, gastric cancer, gallbladder cancer, gastrointestinal stromal tumor cancer, thyroid cancer, head and neck cancer, oropharyngeal cancer, esophageal cancer, melanoma, non-melanoma skin cancer, Merkel cell carcinoma, virally induced cancer, neuroblastoma, breast cancer, prostate cancer, renal cancer, renal cell cancer, renal pelvis cancer, leukemia, lymphoma, sarcoma, glioma, brain tumor, and carcinoma.

14. A nucleic acid encoding the SIRP-α variant construct of any one of claims 1-10.

15. A vector comprising the nucleic acid of claim 14.

16. A host cell comprising the nucleic acid of claim 14 or the vector of claim 15.

17. A method of making the SIRP-α variant construct of any one of claims 1-10, comprising:

(a) culturing the host cell of claim 16 under conditions where the construct is produced, and;
(b) recovering the construct produced by the host cell.

**Patentansprüche**

1. Signalregulatorisches Protein α (SIRP-α) -Variantenkonstrukt, umfassend eine SIRP-α-Variante, die mittels eines spaltbaren Linkers an ein CD-47-basiertes blockierendes Peptid gebunden ist,
wobei das CD-47-basierte blockierende Peptid mindestens 80 % Sequenzidentität mit einer Sequenz der Wildtyp-, IgSF-Domäne von CD47 (SEQ ID NO: 35) oder einem Fragment davon aufweist, das eine SIRP-α-Variante binden kann, und
wobei die SIRP-α-Variante die Aminosäuresequenz umfasst, die in $EEEX_1QX_2IQPDKSVLVAAGETX_3TLRCTX_4TSLX_5PVGPIQWFRGAGPGRX_6LIYNQX_7X_8GX_9FPRVTTVSDX_{10}TX_{11}RNNMDFSIRIGX_{12}ITX_{13}ADAGTYYCX_{14}KX_{15}RKGSPDDVEX_{16}KSGAGTELSVRAKPS$ (SEQ ID NO: 13) dargelegt ist, wobei $X_1$ L, I oder V ist; $X_2$ V, L oder I ist; $X_3$ A oder V ist; $X_4$ A, I oder L ist; $X_5$ I, T, S oder F ist; $X_6$ E, V oder L ist; $X_7$ K oder R ist; $X_8$ E oder Q ist; $X_9$ H, P oder R ist; $X_{10}$ L, T oder G ist; $X_{11}$ K oder R ist; $X_{12}$ N, A, C, D, E, F, G, H, I, K, L, M, P, Q, R, S, T, V, W oder Y ist; $X_{13}$ P, A, C, D, E, F, G, H, I, K, L, M, N, Q, R, S, T, V, W oder Y ist; $X_{14}$ V oder I ist; $X_{15}$ F, L, oder V ist; und $X_{16}$ F oder V ist.

2. SIRP-α-Variantenkonstrukt nach Anspruch 1, wobei die SIRP-α-Variante mindestens 80 % Sequenzidentität mit einer Sequenz gemäß einer der SEQ ID NOs: 3-12 und 24-34 aufweist.

3. SIRP-α-Variantenkonstrukt nach Anspruch 1 oder Anspruch 2, wobei das CD47-basierte blockierende Peptid eine Sequenz gemäß einer der SEQ ID NOs: 36-46 aufweist.

4. SIRP-α-Variantenkonstrukt nach einem der Ansprüche 1 bis 3, wobei der spaltbare Linker einen oder mehrere Spacer umfasst.

5. SIRP-α-Variantenkonstrukt nach einem der Ansprüche 1-4, wobei der spaltbare Linker unter saurem pH-Wert und/oder hypoxischen Bedingungen spaltbar ist.

6. SIRP-α-Variantenkonstrukt nach einem der Ansprüche 1-5, wobei der spaltbare Linker durch ein Tumor-assoziiertes Enzym spaltbar ist, wobei das Tumor-assoziierte Enzym optional eine Protease ist, die ausgewählt ist aus der Gruppe bestehend aus Matriptase (MTSP1), Urin-Plasminogenaktivator (uPA), Legumain, PSA (auch KLK3 genannt, Kallikrein-verwandte Peptidase-3), Matrix-Metalloproteinase-2 (MMP-2), MMP9, menschlicher neutrophiler Elastase (HNE) und Proteinase 3 (Pr3), vorzugsweise Matriptase (MTSP1).

7. SIRP-α-Variantenkonstrukt nach einem der Ansprüche 1-4, wobei der spaltbare Linker eine Sequenz umfasst, die in einer der SEQ ID NOs: 47 oder 69-99 dargelegt ist, vorzugsweise LSGRSDNH (SEQ ID NO: 47).

8. SIRP-α-Variantenkonstrukt nach einem der Ansprüche 1-7, wobei das SIRP-α-Variantenkonstrukt eine Sequenz gemäß einer der SEQ ID NOs: 48-63 aufweist.

9. SIRP-α-Variantenkonstrukt nach einem der Ansprüche 1-8, wobei die SIRP-α-Variante an ein Antikörper-bindendes Peptid gebunden ist, wobei das Antikörper-bindende Peptid optional mindestens 75 % Aminosäuresequenzidentität mit der Sequenz eines Krankheitslokalisierungspeptids (DLP) (SEQ ID NO: 64 oder 65) oder einem Fragment davon aufweist.

10. SIRP-α-Variantenkonstrukt nach einem der Ansprüche 1-9, wobei die SIRP-α-Variante an ein Fc-Domänenmonomer gebunden ist.

11. Pharmazeutische Zusammensetzung, umfassend: a) eine therapeutisch wirksame Menge des SIRP-α-Variantenkonstrukts nach einem der Ansprüche 1-10 und (b) einen pharmazeutisch annehmbaren Hilfsstoff.

12. SIRP-α-Variantenkonstrukt nach einem der Ansprüche 1-10 oder pharmazeutische Zusammensetzung nach Anspruch 11 zur Verwendung als Medikament.

13. SIRP-α-Variantenkonstrukt nach einem der Ansprüche 1-10 oder pharmazeutische Zusammensetzung nach Anspruch 11 zur Verwendung bei der Behandlung eines Krebses, wobei der Krebs optional ausgewählt ist aus solidem Tumorkrebs, hämatologischem Krebs, akuter myeloischer Leukämie, chronischer lymphatischer Leukämie, chronischer myeloischer Leukämie, akuter lymphoblastischer Leukämie, Nicht-Hodgkin-Lymphom, Hodgkin-Lymphom, Multiplem Myelom, Blasenkrebs, Bauchspeicheldrüsenkrebs, Gebärmutterhalskrebs, Endometriumkrebs, Lungenkrebs, Bronchialkrebs, Leberkrebs, Eierstockkrebs, Dickdarm- und Enddarmkrebs, Magenkrebs, Magenkarzinom, Gallenblasenkrebs, gastrointestinalem Stromatumorkrebs, Schilddrüsenkrebs, Kopf- und Halskrebs, Mundrachenkrebs, Speiseröhrenkrebs, Melanom, Nicht-Melanom-Hautkrebs, Merkelzellkarzinom, viral induziertem Krebs, Neuroblastom, Brustkrebs, Prostatakrebs, Nierenkrebs, Nierenzellkrebs, Nierenbeckenkrebs, Leukämie, Lymphom, Sarkom, Gliom, Hirntumor und Karzinom.

14. Nukleinsäure, die das SIRP-α-Variantenkonstrukt nach einem der Ansprüche 1-10 kodiert.

15. Vektor, umfassend die Nukleinsäure nach Anspruch 14.

16. Wirtszelle, umfassend die Nukleinsäure nach Anspruch 14 oder den Vektor nach Anspruch 15.

17. Verfahren zur Herstellung des SIRP-α-Variantenkonstrukts nach einem der Ansprüche 1-10, umfassend:

(a) Kultivieren der Wirtszelle nach Anspruch 16 unter Bedingungen, unter denen das Konstrukt erzeugt wird, und;
(b) Gewinnen des von der Wirtszelle produzierten Konstrukts.

## Revendications

1. Construction de variant de protéine de régulation de signal α (SIRP-a), comprenant un variant de SIRP-α attaché à un peptide bloquant à base de CD47 par le biais d'un lieur clivable,
dans laquelle le peptide bloquant à base de CD47 a au moins 80 % d'identité de séquence avec une séquence de domaine IgSF, de type sauvage, de CD47 (SEQ ID n° : 35) ou un fragment de celui-ci qui peut se lier à un variant de SIRP-α, et
dans laquelle le variant de SIRP-α comprend la séquence d'acides aminés présentée dans $EEEX_1QX_2IQPDKSVLVAAGETX_3TLRCTX_4TSLX_5PVGPIQWFRGAGPGRX_6LI$

YNQ$X_7$$X_8$G$X_9$FPRVTTVSD$X_{10}$T$X_{11}$RNNMDFSIRIG$X_{12}$IT$X_{13}$ADAGTYYC$X_{14}$KX $_{15}$RKGSPDDVE$X_{16}$KSGAGTELSVRAKPS (SEQ ID n° : 13), dans laquelle $X_1$ est L, I, ou V; $X_2$ est V, L, ou, I; $X_3$ est A ou V ; $X_4$ est A, I, ou L; $X_5$ est I, T, S, ou F ; $X_6$ est E, V, ou L; $X_7$ est K ou R; $X_8$ est E ou Q ; $X_9$ est H, P, ou R; $X_{10}$ est L, T, ou G ; $X_{11}$ est K ou R ; $X_{12}$ est N, A, C, D, E, F, G, H, I, K, L, M, P, Q, R, S, T, V, W, ou Y ; $X_{13}$ est P, A, C, D, E, F, G, H, I, K, L, M, N, Q, R, S, T, V, W, ou Y; $X_{14}$ est V ou I; $X_{15}$ est F, L, ou V; et $X_{16}$ est F ou V.

**2.** Construction de variant de SIRP-α selon la revendication 1, dans laquelle le variant de SIRP-α a au moins 80 % d'identité de séquence avec une séquence selon l'un quelconque de SEQ ID n° : 3-12 et 24-34.

**3.** Construction de variant de SIRP-α selon la revendication 1 ou la revendication 2, dans laquelle le peptide bloquant à base de CD47 a une séquence selon l'un quelconque de SEQ ID n° : 36-46.

**4.** Construction de variant de SIRP-α selon l'une quelconque des revendications 1 à 3, dans laquelle le lieur clivable comprend un ou plusieurs espaceurs.

**5.** Construction de variant de SIRP-α selon l'une quelconque des revendications 1 à 4, dans laquelle le lieur clivable est clivable dans une condition hypoxique et/ou à pH acide.

**6.** Construction de variant de SIRP-α selon l'une quelconque des revendications 1 à 5, dans laquelle le lieur clivable est clivable par un enzyme associé à tumeur, optionnellement dans laquelle l'enzyme associé à tumeur est une protéase sélectionnée à partir du groupe constitué de : matriptase (MTSP1), activateur de plasminogène de type urinaire (uPA), légumaine, PSA (également appelé KLK3, peptidase-3 connexe à la kallikréine), métalloprotéinase matricielle-2 (MMP-2), MMP9, élastase neutrophile humaine (HNE), et protéinase 3 (Pr3), de préférence matriptase (MTSP1).

**7.** Construction de variant de SIRP-α selon l'une quelconque des revendications 1 à 4, dans laquelle le lieur clivable comprend une séquence présentée dans l'un quelconque de SEQ ID n° : 47 ou 69-99, de préférence LSGRSDNH (SEQ ID n° : 47).

**8.** Construction de variant de SIRP-α selon l'une quelconque des revendications 1 à 7, dans laquelle la construction de variant de SIRP-α a une séquence selon l'un quelconque de SEQ ID n° : 48-63.

**9.** Construction de variant de SIRP-α selon l'une quelconque des revendications 1 à 8, dans laquelle le variant de SIRP-α est attaché à un peptide de liaison à l'anticorps, optionnellement dans laquelle le peptide de liaison à l'anticorps a au moins 75 % d'identité de séquence d'acide aminé avec la séquence d'un peptide de localisation de maladie (DLP) (SEQ ID n° : 64 ou 65) ou un fragment de celui-ci.

**10.** Construction de variant de SIRP-α selon l'une quelconque des revendications 1 à 9, dans laquelle ledit variant de SIRP-α est attaché à un monomère de domaine Fc.

**11.** Composition pharmaceutique, comprenant : a) une quantité thérapeutiquement efficace de la construction de variant de SIRP-α de l'une quelconque des revendications 1 à 10, et (b) un excipient pharmaceutiquement acceptable.

**12.** Construction de variant de SIRP-α selon l'une quelconque des revendications 1 à 10 ou composition pharmaceutique selon la revendication 11, pour l'utilisation en tant que médicament.

**13.** Construction de variant de SIRP-α selon l'une quelconque des revendications 1 à 10 ou composition pharmaceutique selon la revendication 11, pour l'utilisation dans le traitement d'un cancer, optionnellement dans laquelle ledit cancer est sélectionné à partir de : cancer à tumeurs solides, cancer hématologique, leucémie myéloïde aiguë, leucémie lymphocytaire chronique, leucémie myéloïde chronique, leucémie lymphoblastique aiguë, lymphome non-hodgkinien, lymphome hodgkinien, myélome multiple, cancer de la vessie, cancer pancréatique, cancer cervical, cancer endométrial, cancer du poumon, cancer des bronches, cancer du foie, cancer ovarien, cancer du côlon et rectal, cancer de l'estomac, cancer gastrique, cancer de la vésicule biliaire, cancer à tumeur stromale gastrointestinal, cancer de la thyroïde, cancer de la tête et du cou, cancer oropharyngé, cancer œsophagique, mélanome, cancer de la peau sans présence de mélanome, carcinome à cellules de Merkel, cancer à induction virale, neuroblastome, cancer du sein, cancer de la prostate, cancer rénal, cancer des cellules rénales, cancer du pelvis rénal, leucémie, lymphome, sarcome, gliome, tumeur du cerveau, et carcinome.

**14.** Acide nucléique encodant la construction de variant de SIRP-α de l'une quelconque des revendications 1 à 10.

**15.** Vecteur, comprenant l'acide nucléique de la revendication 14.

**16.** Cellule hôte, comprenant l'acide nucléique de la revendication 14 ou le vecteur de la revendication 15.

**17.** Procédé de production de la construction de variant de SIRP-α de l'une quelconque des revendications 1 à 10, comprenant :

(a) la mise en culture de la cellule hôte de la revendication 16 dans des conditions où la construction est produite, et ;
(b) la récupération de la construction produite par la cellule hôte.

FIG. 1

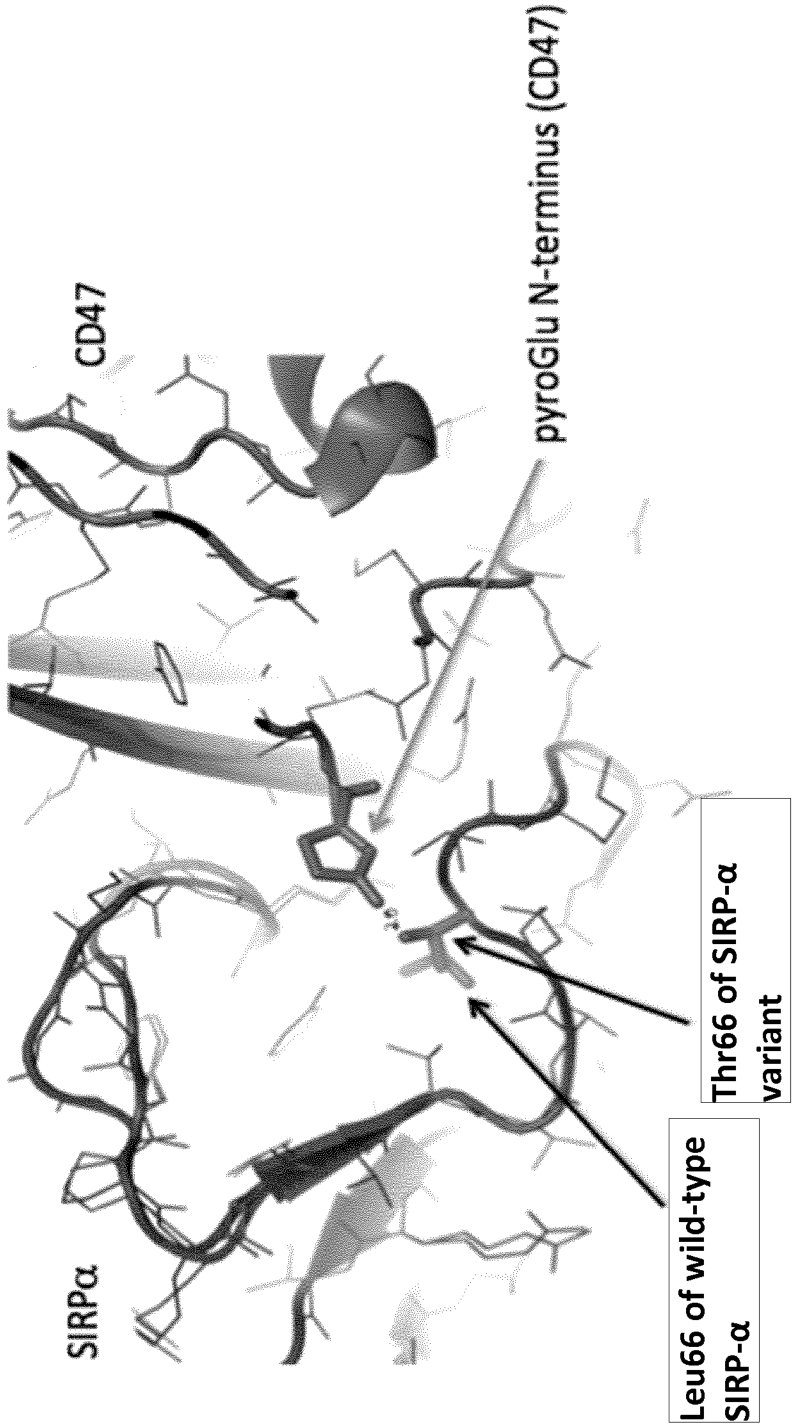
CD47
pyroGlu N-terminus (CD47)
SIRPα
Thr66 of SIRP-α variant
Leu66 of wild-type SIRP-α

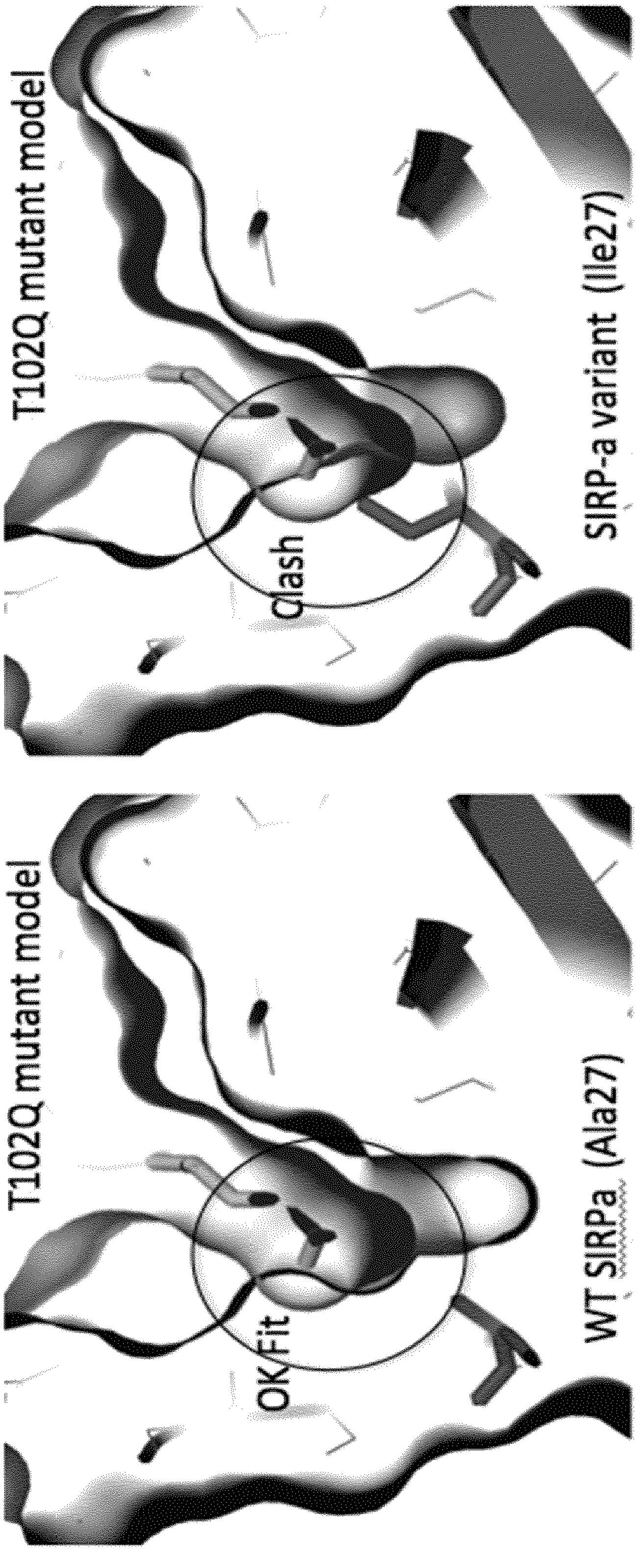
T102Q mutant model
OK Fit
WT SIRPa (Ala27)
T102Q mutant model
Clash
SIRP-a variant (Ile27)

FIG. 2

FIG. 3

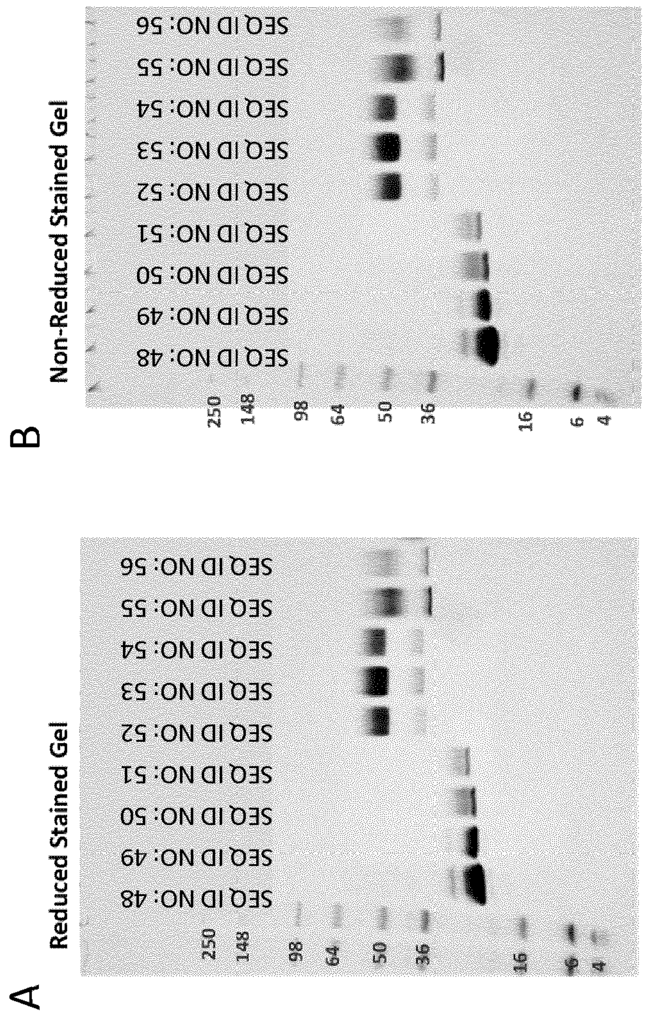
A
Reduced Stained Gel
B
Non-Reduced Stained Gel
SEQ ID NO: 48
SEQ ID NO: 49
SEQ ID NO: 50
SEQ ID NO: 51
SEQ ID NO: 52
SEQ ID NO: 53
SEQ ID NO: 54
SEQ ID NO: 55
SEQ ID NO: 56
250
148
98
64
50
36
16
6
4

FIG. 4

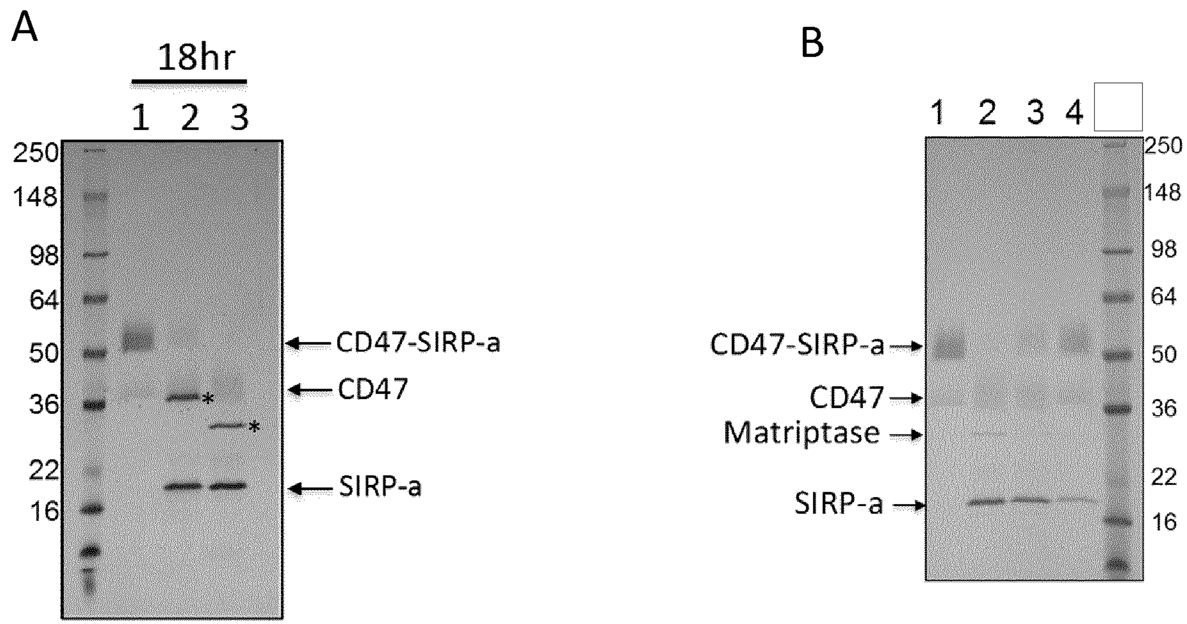


Lanes:
1. No protease
2. uPA
3. Matriptase

*indicates band position for excess uPA or matriptase

Lanes:
1. No matriptase
2. 44 ng of matriptase
3. 0.44 ng of matriptase
4. 0.167 ng of matriptase

FIG. 4 C

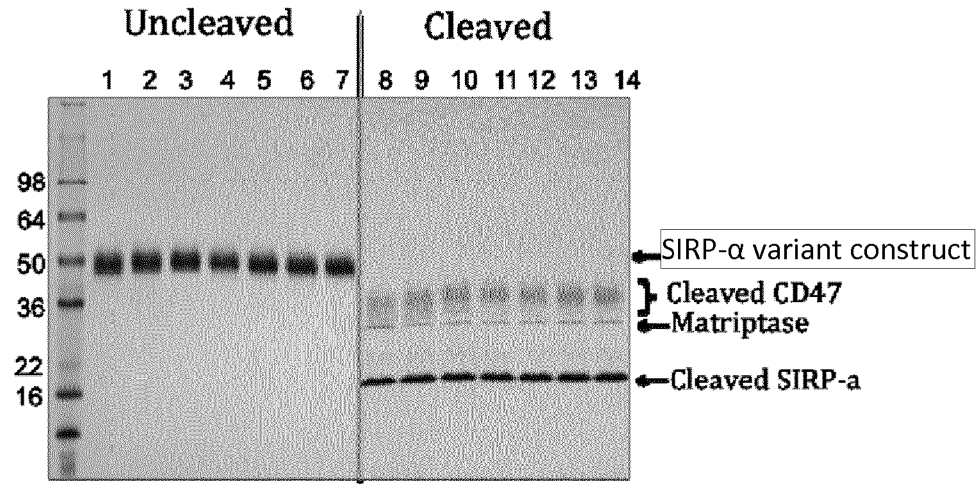


Lanes:

1. SIRP-α variant construct of SEQ ID NO: 57
2. SIRP-α variant construct of SEQ ID NO: 58
3. SIRP-α variant construct of SEQ ID NO: 59
4. SIRP-α variant construct of SEQ ID NO: 60
5. SIRP-α variant construct of SEQ ID NO: 61
6. SIRP-α variant construct of SEQ ID NO: 62
7. SIRP-α variant construct of SEQ ID NO: 63

(Lanes 1–7: No matriptase)

8. SIRP-α variant construct of SEQ ID NO: 57
9. SIRP-α variant construct of SEQ ID NO: 58
10. SIRP-α variant construct of SEQ ID NO: 59
11. SIRP-α variant construct of SEQ ID NO: 60
12. SIRP-α variant construct of SEQ ID NO: 61
13. SIRP-α variant construct of SEQ ID NO: 62
14. SIRP-α variant construct of SEQ ID NO: 63

(Lanes 8–14: 0.44 ng matriptase)

FIG. 5

A

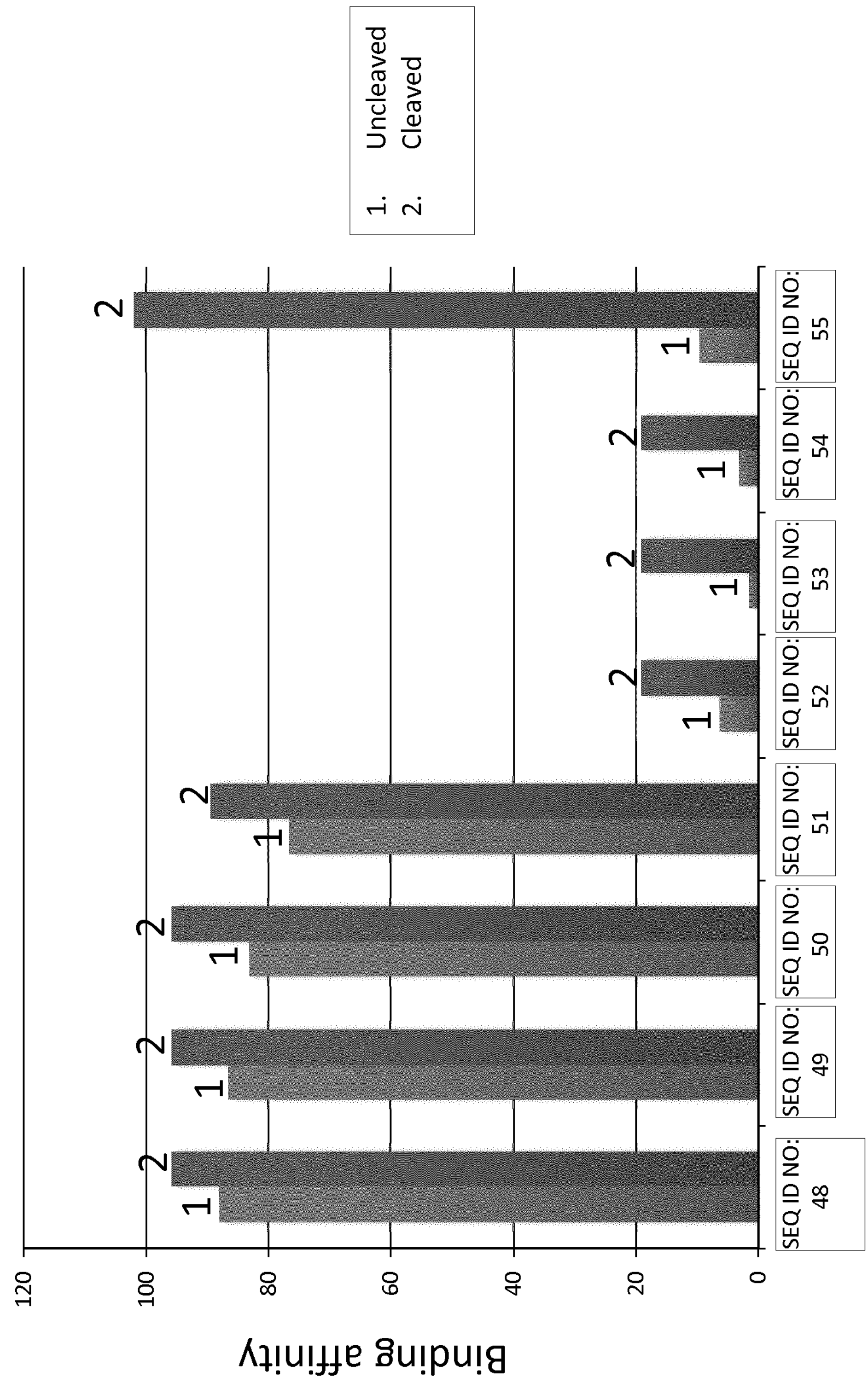
Binding affinity
0
20
40
60
80
100
120
SEQ ID NO: 48
SEQ ID NO: 49
SEQ ID NO: 50
SEQ ID NO: 51
SEQ ID NO: 52
SEQ ID NO: 53
SEQ ID NO: 54
SEQ ID NO: 55
1. Uncleaved
2. Cleaved

FIG. 5

B

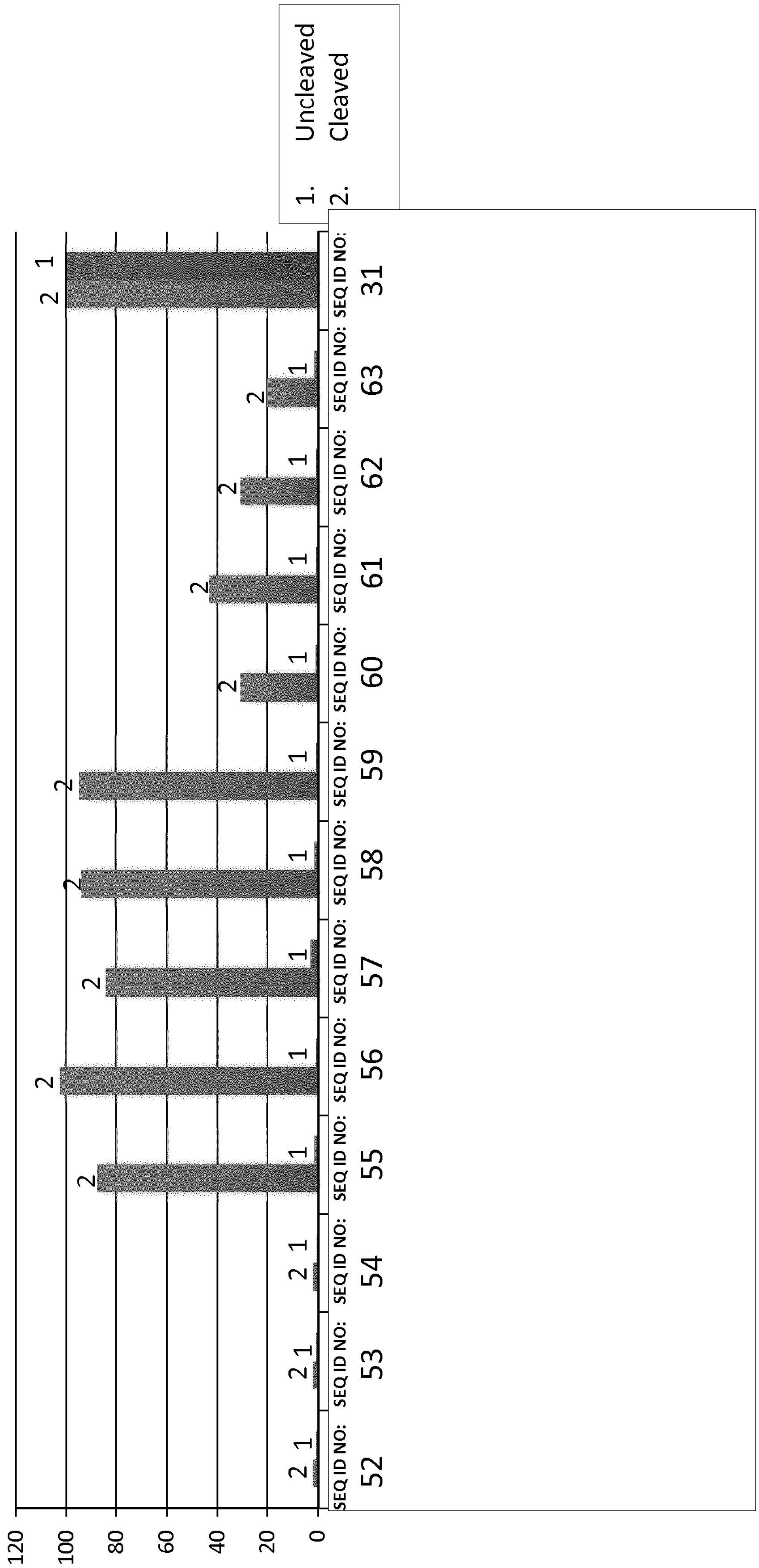
1. Uncleaved
2. Cleaved
120
100
80
60
40
20
0
SEQ ID NO: 52
SEQ ID NO: 53
SEQ ID NO: 54
SEQ ID NO: 55
SEQ ID NO: 56
SEQ ID NO: 57
SEQ ID NO: 58
SEQ ID NO: 59
SEQ ID NO: 60
SEQ ID NO: 61
SEQ ID NO: 62
SEQ ID NO: 63
SEQ ID NO: 31

FIG. 6

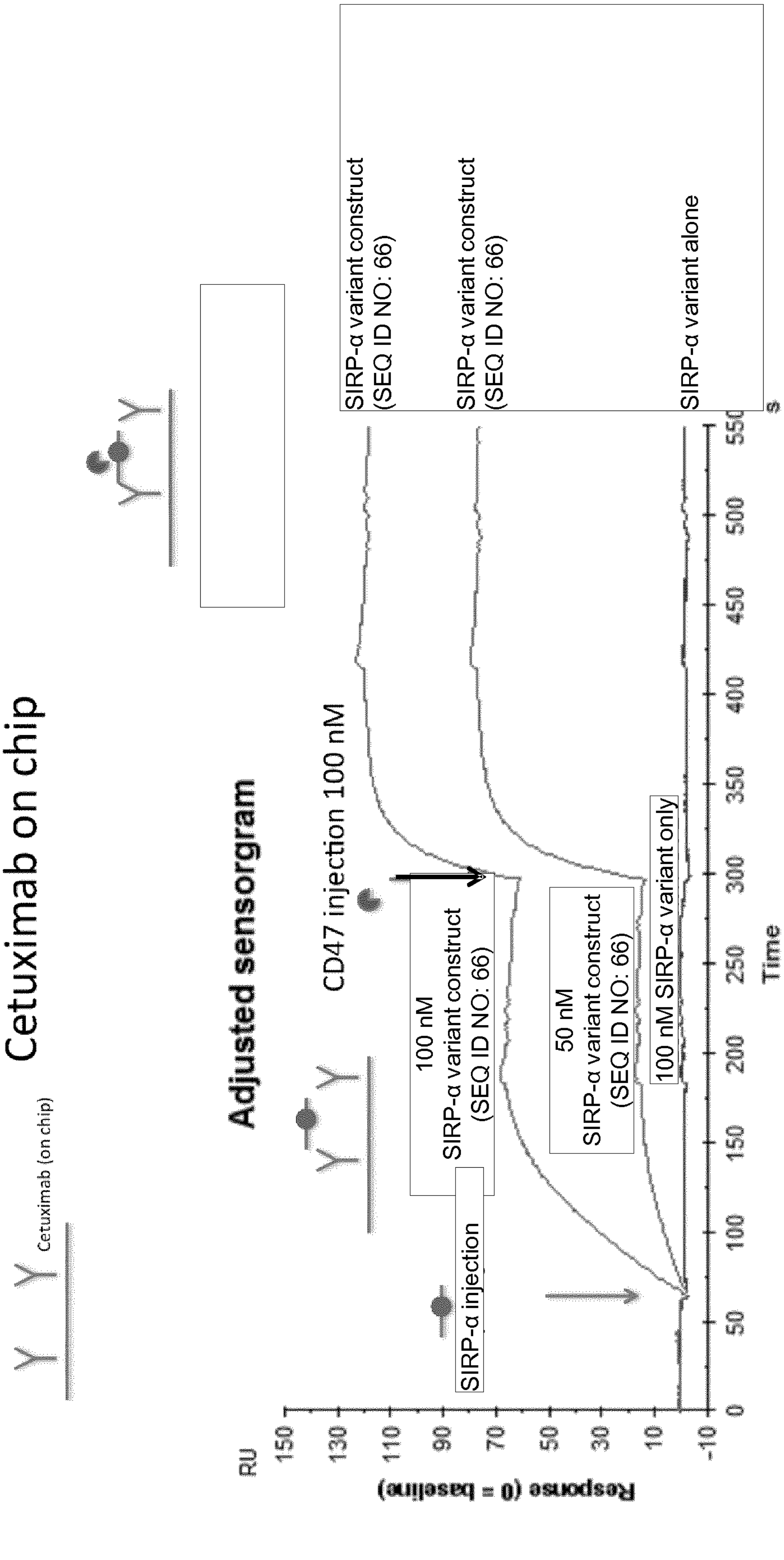
Cetuximab on chip
Cetuximab (on chip)
Adjusted sensorgram
SIRP-α injection
100 nM
SIRP-α variant construct
(SEQ ID NO: 66)
50 nM
SIRP-α variant construct
(SEQ ID NO: 66)
100 nM SIRP-α variant only
CD47 injection 100 nM
SIRP-α variant construct
(SEQ ID NO: 66)
SIRP-α variant construct
(SEQ ID NO: 66)
SIRP-α variant alone
RU
Response (0 = baseline)
150
130
110
90
70
50
30
10
-10
0
50
100
150
200
250
300
350
400
450
500
550
s
Time

FIG. 7

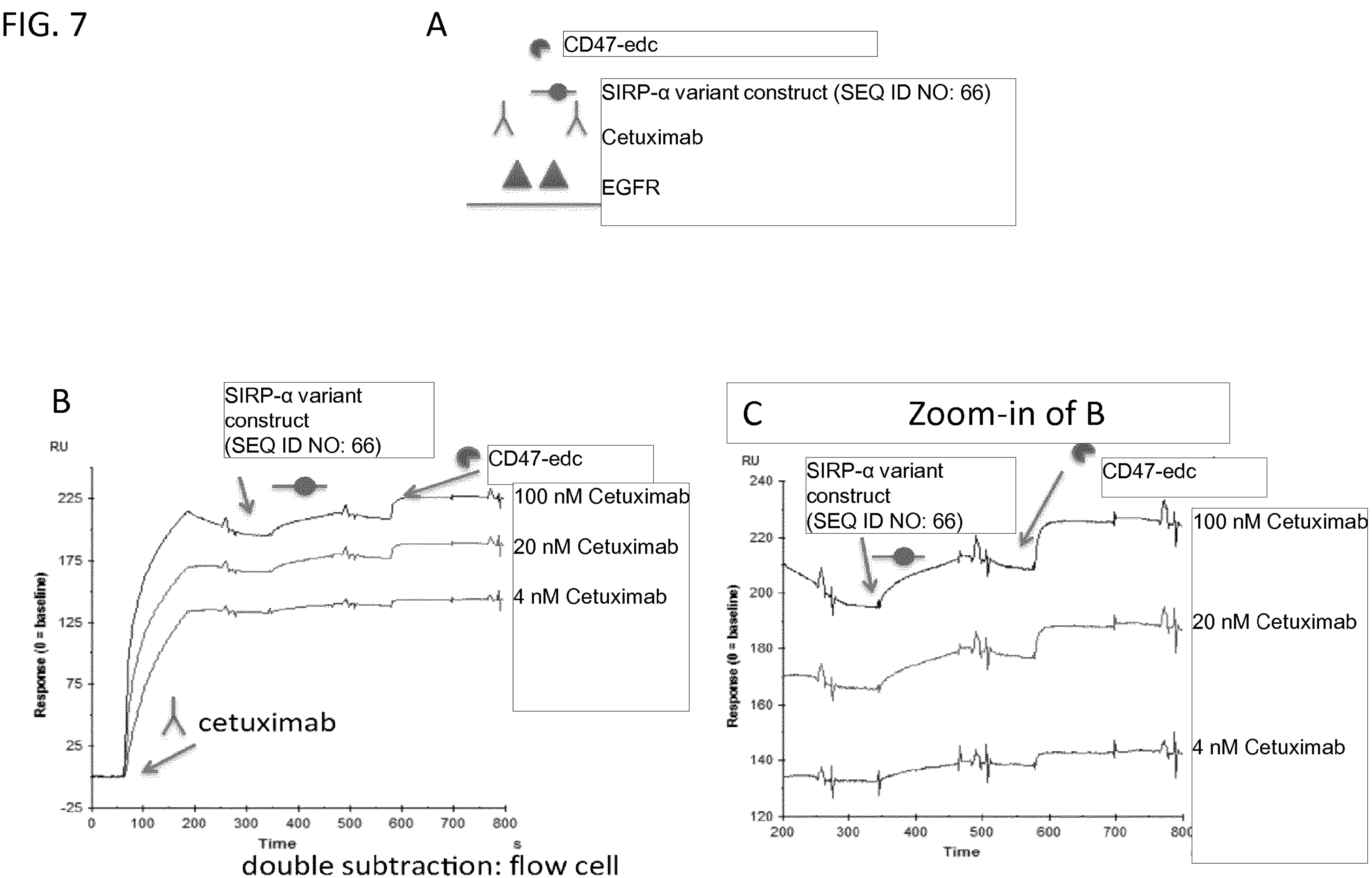
A
CD47-edc
SIRP-α variant construct (SEQ ID NO: 66)
Cetuximab
EGFR
B
SIRP-α variant construct (SEQ ID NO: 66)
CD47-edc
100 nM Cetuximab
20 nM Cetuximab
4 nM Cetuximab
cetuximab
RU
Response (0 = baseline)
Time
s
double subtraction: flow cell
C
Zoom-in of B
SIRP-α variant construct (SEQ ID NO: 66)
CD47-edc
100 nM Cetuximab
20 nM Cetuximab
4 nM Cetuximab
RU
Response (0 = baseline)
Time

FIG. 8

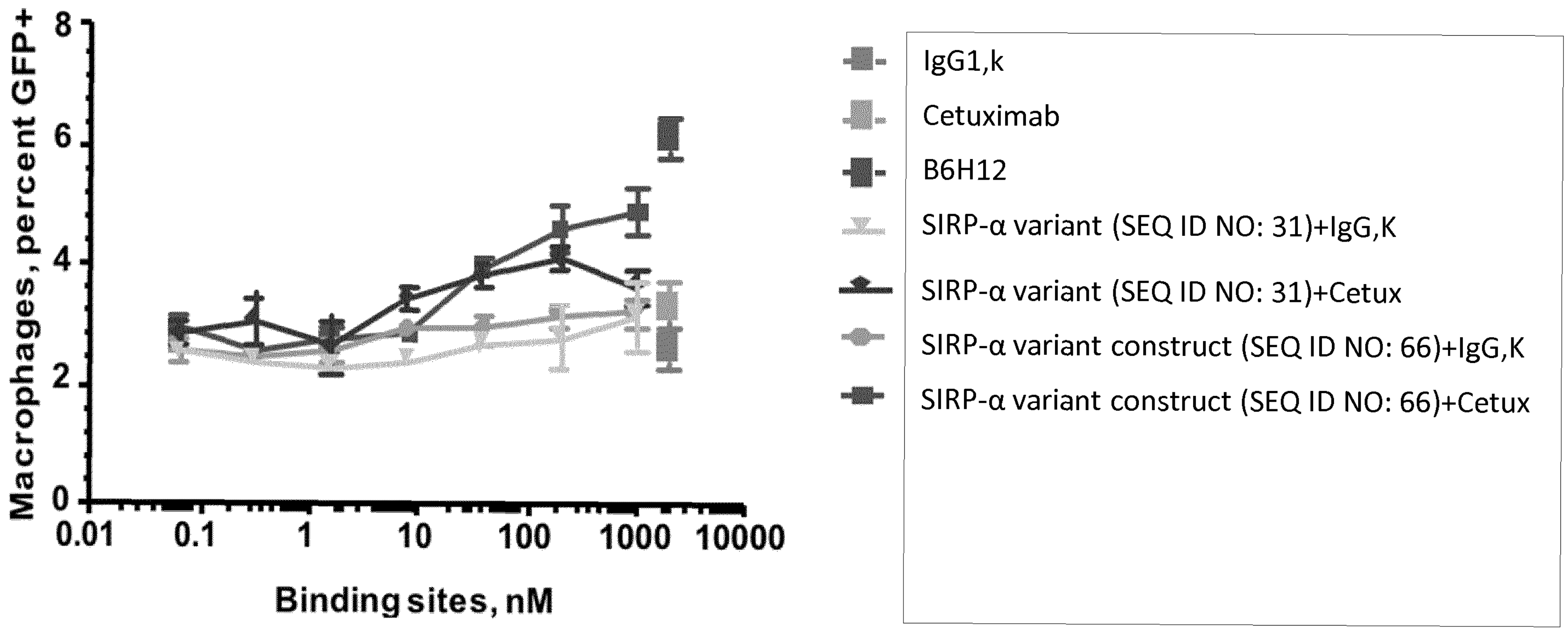
Macrophages, percent GFP+
8
6
4
2
0
0.01
0.1
1
10
100
1000
10000
Binding sites, nM
IgG1,k
Cetuximab
B6H12
SIRP-α variant (SEQ ID NO: 31)+IgG,K
SIRP-α variant (SEQ ID NO: 31)+Cetux
SIRP-α variant construct (SEQ ID NO: 66)+IgG,K
SIRP-α variant construct (SEQ ID NO: 66)+Cetux

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016023040 A
- WO 2010070047 A
- WO 2013109752 A
- US 20100189651 A
- US 20120283408 A
- US 8748399 B
- US 5306809 A
- US 5505931 A
- US 8399219 B
- US 8216805
- US 20140024111 A
- US 5648237 A
- US 3773919 A
- US 5972707 A
- US 5697901 A
- US 6261554 B
- US 5478925 A
- US 8603778 B
- US 7662367 B
- US 7892558 B
- US 6174529 B
- US 6613332 B
- US 8518869 B
- US 7402155 B
- US 6591129 B
- US 20140051634 A
- WO 1993000077 A
- US 20110184145 A
- US 62202772 B
- US 62202775 B
- US 62202779 B
- TW 104125902
- US 14971931 B


### Non-patent literature cited in the description


- **JAWA et al.** *Clinical Immunology,* 2013, vol. 149, 534-555
- **ICARD et al.** *Biochim. Biophys. Acta,* 2012, vol. 1826, 423-433
- **STRAUCH et al.** *Proc Natl Acad Sci,* 2014, vol. 111, 675-80
- **KLING et al.** *Nature Biotechnology,* 2012, vol. 30, 381
- **DONALDSON et al.** *Proc Natl Acad Sci USA.,* 2013, vol. 110, 17456-17461
- **WEISKOPF et al.** *Science,* 2013, vol. 341, 88-91
- **DONALDSON et al.** *Proc Natl Acad Sci,* 2013, vol. 110, 17456-61
- **LAURENT et al.** *Bioconjugate Chem.,* 2010, vol. 21, 5-13
- **CASTANEDA et al.** *Chem. Commun.,* 2013, vol. 49, 8187-8189
- **DUCRY et al.** *Bioconjug. Chem.,* 2010, vol. 21, 5-13
- **SHIGENAGA et al.** *European Journal of Chemical Biology,* 2012, vol. 13, 968-971
- **SHIGENAGA et al.** *European J. Chem, Biol.,* 2012, vol. 13, 968-971
- **DUAN et al.** *J. Med. Chem.,* 2008, vol. 51, 2412-2420
- **LA ROCCA et al.** *Brit. J. Cancer,* vol. 90, 1414-1421
- **KOBLINSKI et al.** *Chim. Acta,* 2000, vol. 291, 113-135
- **DENNIS et al.** *J. Biol. Chem.,* 2002, vol. 277, 35035-35043
- **MIYAKAWA et al.** *J. Pharm. Sci.,* 2013, vol. 102, 3110-3118
- **RIDGWAY et al.** *Protein Eng.,* 1996, vol. 9, 617-612
- **ATWELL et al.** *J Mol Biol.,* 1997, vol. 270, 26-35
- **MERCHANT et al.** *Nat Biotechnol.,* 1998, vol. 16, 677-681
- **MERCHANT et al.** *Nature Biotechnology,* 1998, vol. 16, 677-681
- **MERCHANT et al.** *Proc Natl Acad Sci USA.,* 2013, vol. 110, E2987-E2996
- **GUNASEKARAN et al.** *J Biol Chem.,* 2010, vol. 285, 19637-46
- **MARTENS et al.** *Clin Cancer Res.,* 2006, vol. 12, 6144-52
- **MILLA et al.** *Curr Drug Metab.,* 2012, vol. 13, 105-119
- **GREGORIADIS et al.** *Cell Mol. Life Sci.,* 2000, vol. 57, 1964-1969
- **CONSTANTINOU et al.** *Bioconjug. Chem.,* 2008, vol. 19, 643-650
- **SCHLAPSCHY et al.** *Protein Eng. Des. Sel.,* 2013, vol. 26, 489-501

- **SCHELLENBERGER et al.** *Nat. Biotechnol.,* 2009, vol. 27, 1186-1192
- **TRUSSEL et al.** *Bioconjug Chem.,* 2009, vol. 20, 2286-2292
- Remington: The Science and Practice of Pharmacy. 2000
- Pharmaceutics and Pharmacy Practice. J. B. Lippincott Company, 1982, 238-250
- ASHP Handbook on Injectable Drugs. 1986, 622-630
- **ULISSE et al.** *Curr. Cancer Drug Targets,* 2009, vol. 9, 32-71
- **UHLAND et al.** *Cell. Mol. Life Sci.,* 2006, vol. 63, 2968-2978
- **LEBEAU et al.** *Proc. Natl. Acad. Sci. USA,* 2013, vol. 110, 93-98
- **LIU et al.** *Cancer Res.,* 2003, vol. 63, 2957-2964
- **WEISKOFP et al.** *Science,* 2013, vol. 341, 88-91

SEQUENCE LISTING

<110> ALEXO THERAPEUTICS INC.

<120> SIRP-ALPHA VARIANT CONSTRUCTS AND USES THEREOF

<130> P236028EP

<150> US 62/202,772
<151> 2015-08-07

<150> US 62/202,775
<151> 2015-08-07

<150> US 62/202,779
<151> 2015-08-07

<150> TW 104125902
<151> 2015-08-10

<150> US 14/971,931
<151> 2015-12-16

<160> 142

<170> PatentIn version 3.5

<210> 1
<211> 504
<212> PRT
<213> Homo sapiens

<400> 1

```
Met Glu Pro Ala Gly Pro Ala Pro Gly Arg Leu Gly Pro Leu Leu Cys
1               5                   10                  15

Leu Leu Leu Ala Ala Ser Cys Ala Trp Ser Gly Val Ala Gly Glu Glu
            20                  25                  30

Glu Leu Gln Val Ile Gln Pro Asp Lys Ser Val Leu Val Ala Ala Gly
        35                  40                  45

Glu Thr Ala Thr Leu Arg Cys Thr Ala Thr Ser Leu Ile Pro Val Gly
    50                  55                  60

Pro Ile Gln Trp Phe Arg Gly Ala Gly Pro Gly Arg Glu Leu Ile Tyr
65                  70                  75                  80

Asn Gln Lys Glu Gly His Phe Pro Arg Val Thr Thr Val Ser Asp Leu
                85                  90                  95

Thr Lys Arg Asn Asn Met Asp Phe Ser Ile Arg Ile Gly Asn Ile Thr
            100                 105                 110

Pro Ala Asp Ala Gly Thr Tyr Tyr Cys Val Lys Phe Arg Lys Gly Ser
        115                 120                 125
```

```
Pro Asp Asp Val Glu Phe Lys Ser Gly Ala Gly Thr Glu Leu Ser Val
    130                 135                 140

Arg Ala Lys Pro Ser Ala Pro Val Val Ser Gly Pro Ala Ala Arg Ala
145                 150                 155                 160

Thr Pro Gln His Thr Val Ser Phe Thr Cys Glu Ser His Gly Phe Ser
                165                 170                 175

Pro Arg Asp Ile Thr Leu Lys Trp Phe Lys Asn Gly Asn Glu Leu Ser
            180                 185                 190

Asp Phe Gln Thr Asn Val Asp Pro Val Gly Glu Ser Val Ser Tyr Ser
        195                 200                 205

Ile His Ser Thr Ala Lys Val Val Leu Thr Arg Glu Asp Val His Ser
    210                 215                 220

Gln Val Ile Cys Glu Val Ala His Val Thr Leu Gln Gly Asp Pro Leu
225                 230                 235                 240

Arg Gly Thr Ala Asn Leu Ser Glu Thr Ile Arg Val Pro Pro Thr Leu
                245                 250                 255

Glu Val Thr Gln Gln Pro Val Arg Ala Glu Asn Gln Val Asn Val Thr
            260                 265                 270

Cys Gln Val Arg Lys Phe Tyr Pro Gln Arg Leu Gln Leu Thr Trp Leu
        275                 280                 285

Glu Asn Gly Asn Val Ser Arg Thr Glu Thr Ala Ser Thr Val Thr Glu
    290                 295                 300

Asn Lys Asp Gly Thr Tyr Asn Trp Met Ser Trp Leu Leu Val Asn Val
305                 310                 315                 320

Ser Ala His Arg Asp Asp Val Lys Leu Thr Cys Gln Val Glu His Asp
                325                 330                 335

Gly Gln Pro Ala Val Ser Lys Ser His Asp Leu Lys Val Ser Ala His
            340                 345                 350

Pro Lys Glu Gln Gly Ser Asn Thr Ala Ala Glu Asn Thr Gly Ser Asn
        355                 360                 365

Glu Arg Asn Ile Tyr Ile Val Val Gly Val Val Cys Thr Leu Leu Val
```

370 375 380

Ala Leu Leu Met Ala Ala Leu Tyr Leu Val Arg Ile Arg Gln Lys Lys
385 390 395 400

Ala Gln Gly Ser Thr Ser Ser Thr Arg Leu His Glu Pro Glu Lys Asn
405 410 415

Ala Arg Glu Ile Thr Gln Asp Thr Asn Asp Ile Thr Tyr Ala Asp Leu
420 425 430

Asn Leu Pro Lys Gly Lys Lys Pro Ala Pro Gln Ala Ala Glu Pro Asn
435 440 445

Asn His Thr Glu Tyr Ala Ser Ile Gln Thr Ser Pro Gln Pro Ala Ser
450 455 460

Glu Asp Thr Leu Thr Tyr Ala Asp Leu Asp Met Val His Leu Asn Arg
465 470 475 480

Thr Pro Lys Gln Pro Ala Pro Lys Pro Glu Pro Ser Phe Ser Glu Tyr
485 490 495

Ala Ser Val Gln Val Pro Arg Lys
500

<210> 2
<211> 11
<212> PRT
<213> Artificial Sequence

<220>
<223> Description of Artificial Sequence: Synthetic peptide

<400> 2

Asp Ile Cys Leu Pro Arg Trp Gly Cys Leu Trp
1 5 10

<210> 3
<211> 119
<212> PRT
<213> Homo sapiens

<400> 3

Glu Glu Glu Leu Gln Val Ile Gln Pro Asp Lys Ser Val Leu Val Ala
1 5 10 15

Ala Gly Glu Thr Ala Thr Leu Arg Cys Thr Ala Thr Ser Leu Ile Pro
20 25 30

Val Gly Pro Ile Gln Trp Phe Arg Gly Ala Gly Pro Gly Arg Glu Leu
35 40 45

Ile Tyr Asn Gln Lys Glu Gly His Phe Pro Arg Val Thr Thr Val Ser
50 55 60

Asp Leu Thr Lys Arg Asn Asn Met Asp Phe Ser Ile Arg Ile Gly Asn
65 70 75 80

Ile Thr Pro Ala Asp Ala Gly Thr Tyr Tyr Cys Val Lys Phe Arg Lys
85 90 95

Gly Ser Pro Asp Asp Val Glu Phe Lys Ser Gly Ala Gly Thr Glu Leu
100 105 110

Ser Val Arg Ala Lys Pro Ser
115

<210> 4
<211> 118
<212> PRT
<213> Homo sapiens

<400> 4

Glu Glu Glu Leu Gln Val Ile Gln Pro Asp Lys Ser Val Ser Val Ala
1 5 10 15

Ala Gly Glu Ser Ala Ile Leu His Cys Thr Val Thr Ser Leu Ile Pro
20 25 30

Val Gly Pro Ile Gln Trp Phe Arg Gly Ala Gly Pro Ala Arg Glu Leu
35 40 45

Ile Tyr Asn Gln Lys Glu Gly His Phe Pro Arg Val Thr Thr Val Ser
50 55 60

Glu Ser Thr Lys Arg Glu Asn Met Asp Phe Ser Ile Ser Ile Ser Asn
65 70 75 80

Ile Thr Pro Ala Asp Ala Gly Thr Tyr Tyr Cys Val Lys Phe Arg Lys
85 90 95

Gly Ser Pro Asp Thr Glu Phe Lys Ser Gly Ala Gly Thr Glu Leu Ser
100 105 110

Val Arg Ala Lys Pro Ser
115

<210> 5
<211> 118
<212> PRT
<213> Homo sapiens

<400> 5

```
Glu Glu Glu Leu Gln Val Ile Gln Pro Asp Lys Ser Val Ser Val Ala
1               5                   10                  15

Ala Gly Glu Ser Ala Ile Leu Leu Cys Thr Val Thr Ser Leu Ile Pro
            20                  25                  30

Val Gly Pro Ile Gln Trp Phe Arg Gly Ala Gly Pro Ala Arg Glu Leu
        35                  40                  45

Ile Tyr Asn Gln Lys Glu Gly His Phe Pro Arg Val Thr Thr Val Ser
    50                  55                  60

Glu Ser Thr Lys Arg Glu Asn Met Asp Phe Ser Ile Ser Ile Ser Asn
65                  70                  75                  80

Ile Thr Pro Ala Asp Ala Gly Thr Tyr Tyr Cys Val Lys Phe Arg Lys
                85                  90                  95

Gly Ser Pro Asp Thr Glu Phe Lys Ser Gly Ala Gly Thr Glu Leu Ser
            100                 105                 110

Val Arg Ala Lys Pro Ser
        115
```

<210> 6
<211> 119
<212> PRT
<213> Homo sapiens

<400> 6

```
Glu Glu Gly Leu Gln Val Ile Gln Pro Asp Lys Ser Val Ser Val Ala
1               5                   10                  15

Ala Gly Glu Ser Ala Ile Leu His Cys Thr Ala Thr Ser Leu Ile Pro
            20                  25                  30

Val Gly Pro Ile Gln Trp Phe Arg Gly Ala Gly Pro Gly Arg Glu Leu
        35                  40                  45

Ile Tyr Asn Gln Lys Glu Gly His Phe Pro Arg Val Thr Thr Val Ser
    50                  55                  60

Asp Leu Thr Lys Arg Asn Asn Met Asp Phe Ser Ile Arg Ile Gly Asn
65                  70                  75                  80

Ile Thr Pro Ala Asp Ala Gly Thr Tyr Tyr Cys Val Lys Phe Arg Lys
                85                  90                  95

Gly Ser Pro Asp Asp Val Glu Phe Lys Ser Gly Ala Gly Thr Glu Leu
            100                 105                 110

Ser Val Arg Ala Lys Pro Ser
        115
```

<210> 7
<211> 119
<212> PRT
<213> Homo sapiens

<400> 7

```
Glu Glu Glu Leu Gln Val Ile Gln Pro Asp Lys Phe Val Leu Val Ala
1               5                   10                  15

Ala Gly Glu Thr Ala Thr Leu Arg Cys Thr Ala Thr Ser Leu Ile Pro
            20                  25                  30

Val Gly Pro Ile Gln Trp Phe Arg Gly Ala Gly Pro Gly Arg Glu Leu
        35                  40                  45

Ile Tyr Asn Gln Lys Glu Gly His Phe Pro Arg Val Thr Thr Val Ser
    50                  55                  60

Asp Leu Thr Lys Arg Asn Asn Met Asp Phe Ser Ile Arg Ile Gly Asn
65                  70                  75                  80

Ile Thr Pro Ala Asp Ala Gly Thr Tyr Tyr Cys Val Lys Phe Arg Lys
                85                  90                  95

Gly Ser Pro Asp Asp Val Glu Phe Lys Ser Gly Ala Gly Thr Glu Leu
            100                 105                 110

Ser Val Arg Ala Lys Pro Ser
        115
```

<210> 8
<211> 119
<212> PRT
<213> Homo sapiens

<400> 8

```
Glu Glu Glu Leu Gln Val Ile Gln Pro Asp Lys Ser Val Leu Val Ala
1               5                   10                  15

Ala Gly Glu Thr Ala Thr Leu Arg Cys Thr Ala Thr Ser Leu Ile Pro
            20                  25                  30
```

```
Val Gly Pro Ile Gln Trp Phe Arg Gly Ala Gly Pro Gly Arg Glu Leu
        35                  40                  45

Ile Tyr Asn Gln Lys Glu Gly His Phe Pro Arg Val Thr Thr Val Ser
    50                  55                  60

Asp Leu Thr Lys Arg Asn Asn Met Asp Phe Pro Ile Arg Ile Gly Asn
65                  70                  75                  80

Ile Thr Pro Ala Asp Ala Gly Thr Tyr Tyr Cys Val Lys Phe Arg Lys
                85                  90                  95

Gly Ser Pro Asp Asp Val Glu Phe Lys Ser Gly Ala Gly Thr Glu Leu
            100                 105                 110

Ser Val Arg Ala Lys Pro Ser
        115
```

<210> 9
<211> 118
<212> PRT
<213> Homo sapiens

<400> 9

```
Glu Glu Glu Leu Gln Val Ile Gln Pro Asp Lys Ser Val Ser Val Ala
1               5                   10                  15

Ala Gly Glu Ser Ala Ile Leu His Cys Thr Val Thr Ser Leu Ile Pro
            20                  25                  30

Val Gly Pro Ile Gln Trp Phe Arg Gly Ala Gly Pro Ala Arg Glu Leu
        35                  40                  45

Ile Tyr Asn Gln Lys Glu Gly His Phe Pro Arg Val Thr Thr Val Ser
    50                  55                  60

Glu Ser Thr Lys Arg Glu Asn Met Asp Phe Ser Ile Ser Ile Ser Asn
65                  70                  75                  80

Ile Thr Pro Ala Asp Ala Gly Thr Tyr Tyr Cys Val Lys Phe Arg Lys
                85                  90                  95

Gly Ser Pro Asp Thr Glu Phe Lys Ser Gly Ala Gly Thr Glu Leu Ser
            100                 105                 110

Val Arg Gly Lys Pro Ser
        115
```

<210> 10
<211> 118
<212> PRT
<213> Homo sapiens

<400> 10

```
Glu Glu Glu Leu Gln Val Ile Gln Pro Asp Lys Ser Val Leu Val Ala
1               5                   10                  15

Ala Gly Glu Thr Ala Thr Leu Arg Cys Thr Ala Thr Ser Leu Ile Pro
            20                  25                  30

Val Gly Pro Ile Gln Trp Phe Arg Gly Ala Gly Pro Ala Arg Glu Leu
        35                  40                  45

Ile Tyr Asn Gln Lys Glu Gly His Phe Pro Arg Val Thr Thr Val Ser
    50                  55                  60

Glu Ser Thr Lys Arg Glu Asn Met Asp Phe Ser Ile Ser Ile Ser Asn
65                  70                  75                  80

Ile Thr Pro Ala Asp Ala Gly Thr Tyr Tyr Cys Val Lys Phe Arg Lys
                85                  90                  95

Gly Ser Pro Asp Thr Glu Phe Lys Ser Gly Ala Gly Thr Glu Leu Ser
            100                 105                 110

Val Arg Ala Lys Pro Ser
        115
```

<210> 11
<211> 119
<212> PRT
<213> Homo sapiens

<400> 11

```
Glu Glu Glu Leu Gln Val Ile Gln Pro Asp Lys Ser Val Leu Val Ala
1               5                   10                  15

Ala Gly Glu Thr Ala Thr Leu Arg Cys Thr Ala Thr Ser Leu Ile Pro
            20                  25                  30

Val Gly Pro Ile Gln Trp Phe Arg Gly Ala Gly Pro Gly Arg Glu Leu
        35                  40                  45

Ile Tyr Asn Gln Lys Glu Gly His Phe Pro Arg Val Thr Thr Val Ser
    50                  55                  60

Asp Leu Thr Lys Arg Asn Asn Met Asp Phe Ser Ile Arg Ile Ser Asn
65                  70                  75                  80

Ile Thr Pro Ala Asp Ala Gly Thr Tyr Tyr Cys Val Lys Phe Arg Lys
                85                  90                  95

Gly Ser Pro Asp Asp Val Glu Phe Lys Ser Gly Ala Gly Thr Glu Leu
            100                 105                 110

Ser Val Arg Ala Lys Pro Ser
        115
```

<210> 12
<211> 118
<212> PRT
<213> Homo sapiens

<400> 12

```
Glu Glu Glu Leu Gln Val Ile Gln Pro Asp Lys Ser Val Ser Val Ala
1               5                   10                  15

Ala Gly Glu Ser Ala Ile Leu His Cys Thr Val Thr Ser Leu Ile Pro
            20                  25                  30

Val Gly Pro Ile Gln Trp Phe Arg Gly Ala Gly Pro Ala Arg Glu Leu
        35                  40                  45

Ile Tyr Asn Gln Lys Glu Gly His Phe Pro Arg Val Thr Thr Val Ser
    50                  55                  60

Glu Ser Thr Lys Arg Glu Asn Met Asp Phe Ser Ile Ser Ile Ser Asn
65                  70                  75                  80

Ile Thr Pro Ala Asp Ala Gly Thr Tyr Tyr Cys Val Lys Phe Arg Lys
                85                  90                  95

Gly Ser Pro Asp Thr Glu Phe Lys Ser Gly Ala Gly Thr Glu Leu Ser
            100                 105                 110

Val Arg Ala Lys Pro Ser
        115
```

<210> 13
<211> 119
<212> PRT
<213> Artificial Sequence

<220>
<223> Description of Artificial Sequence: Synthetic polypeptide

<220>
<221> MOD_RES
<222> (4)..(4)
<223> L, I or V

<220>
<221> MOD_RES
<222> (6) .. (6)
<223> V, L or I

<220>
<221> MOD_RES
<222> (21) .. (21)
<223> A or V

<220>
<221> MOD_RES
<222> (27) .. (27)
<223> A, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> L, T or G

<220>
<221> MOD_RES
<222> (68) .. (68)
<223> K or R

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (104)..(104)

<223> F or V

<400> 13

```
Glu Glu Glu Xaa Gln Xaa Ile Gln Pro Asp Lys Ser Val Leu Val Ala
1               5                   10                  15

Ala Gly Glu Thr Xaa Thr Leu Arg Cys Thr Xaa Thr Ser Leu Xaa Pro
            20                  25                  30

Val Gly Pro Ile Gln Trp Phe Arg Gly Ala Gly Pro Gly Arg Xaa Leu
        35                  40                  45

Ile Tyr Asn Gln Xaa Xaa Gly Xaa Phe Pro Arg Val Thr Thr Val Ser
    50                  55                  60

Asp Xaa Thr Xaa Arg Asn Asn Met Asp Phe Ser Ile Arg Ile Gly Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Asp Val Glu Xaa Lys Ser Gly Ala Gly Thr Glu Leu
            100                 105                 110

Ser Val Arg Ala Lys Pro Ser
        115
```

<210> 14
<211> 118
<212> PRT
<213> Artificial Sequence

<220>
<223> Description of Artificial Sequence: Synthetic polypeptide

<220>
<221> MOD_RES
<222> (4)..(4)
<223> L, I or V

<220>
<221> MOD_RES
<222> (6) .. (6)
<223> V, L or I

<220>
<221> MOD_RES
<222> (21) .. (21)
<223> A or V

<220>

<221> MOD_RES
<222> (27) .. (27)
<223> V, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> S, T or G

<220>
<221> MOD_RES
<222> (68) .. (68)
<223> K or R

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (103) .. (103)
<223> F or V

<400> 14

```
Glu Glu Glu Xaa Gln Xaa Ile Gln Pro Asp Lys Ser Val Ser Val Ala
1               5                   10                  15

Ala Gly Glu Ser Xaa Ile Leu His Cys Thr Xaa Thr Ser Leu Xaa Pro
            20                  25                  30

Val Gly Pro Ile Gln Trp Phe Arg Gly Ala Gly Pro Ala Arg Xaa Leu
        35                  40                  45

Ile Tyr Asn Gln Xaa Xaa Gly Xaa Phe Pro Arg Val Thr Thr Val Ser
    50                  55                  60

Glu Xaa Thr Xaa Arg Glu Asn Met Asp Phe Ser Ile Ser Ile Ser Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Thr Glu Xaa Lys Ser Gly Ala Gly Thr Glu Leu Ser
            100                 105                 110

Val Arg Ala Lys Pro Ser
        115
```

<210> 15
<211> 118
<212> PRT
<213> Artificial Sequence

<220>
<223> Description of Artificial Sequence: Synthetic polypeptide

<220>
<221> MOD_RES
<222> (4)..(4)
<223> L, I or V

<220>
<221> MOD_RES
<222> (6) .. (6)
<223> V, L or I

<220>
<221> MOD_RES
<222> (21) .. (21)

<223> A or V

<220>
<221> MOD_RES
<222> (27) .. (27)
<223> V, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> S, T or G

<220>
<221> MOD_RES
<222> (68) .. (68)
<223> K or R

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)
<223> V or I

<220>

<221> MOD_RES
<222> (94) .. (94)
<223> F, L or V

<220>
<221> MOD_RES
<222> (103) .. (103)
<223> F or V

<400> 15

```
Glu Glu Glu Xaa Gln Xaa Ile Gln Pro Asp Lys Ser Val Ser Val Ala
1               5                   10                  15

Ala Gly Glu Ser Xaa Ile Leu Leu Cys Thr Xaa Thr Ser Leu Xaa Pro
            20                  25                  30

Val Gly Pro Ile Gln Trp Phe Arg Gly Ala Gly Pro Ala Arg Xaa Leu
        35                  40                  45

Ile Tyr Asn Gln Xaa Xaa Gly Xaa Phe Pro Arg Val Thr Thr Val Ser
    50                  55                  60

Glu Xaa Thr Xaa Arg Glu Asn Met Asp Phe Ser Ile Ser Ile Ser Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Thr Glu Xaa Lys Ser Gly Ala Gly Thr Glu Leu Ser
            100                 105                 110

Val Arg Ala Lys Pro Ser
        115
```

<210> 16
<211> 119
<212> PRT
<213> Artificial Sequence

<220>
<223> Description of Artificial Sequence: Synthetic polypeptide

<220>
<221> MOD_RES
<222> (4)..(4)
<223> L, I or V

<220>
<221> MOD_RES
<222> (6) .. (6)
<223> V, L or I

<220>
<221> MOD_RES
<222> (21) .. (21)
<223> A or V

<220>
<221> MOD_RES
<222> (27) .. (27)
<223> A, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> L, T or G

<220>
<221> MOD_RES
<222> (68) .. (68)
<223> K or R

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)

<223> V or I

<220>
<221> MOD_RES
<222> (94) .. (94)
<223> F, L or V

<220>
<221> MOD_RES
<222> (104)..(104)
<223> F or V

<400> 16

```
Glu Glu Gly Xaa Gln Xaa Ile Gln Pro Asp Lys Ser Val Ser Val Ala
1               5                   10                  15

Ala Gly Glu Ser Xaa Ile Leu His Cys Thr Xaa Thr Ser Leu Xaa Pro
            20                  25                  30

Val Gly Pro Ile Gln Trp Phe Arg Gly Ala Gly Pro Gly Arg Xaa Leu
        35                  40                  45

Ile Tyr Asn Gln Xaa Xaa Gly Xaa Phe Pro Arg Val Thr Thr Val Ser
    50                  55                  60

Asp Xaa Thr Xaa Arg Asn Asn Met Asp Phe Ser Ile Arg Ile Gly Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Asp Val Glu Xaa Lys Ser Gly Ala Gly Thr Glu Leu
            100                 105                 110

Ser Val Arg Ala Lys Pro Ser
        115
```

<210> 17
<211> 119
<212> PRT
<213> Artificial Sequence

<220>
<223> Description of Artificial Sequence: Synthetic polypeptide

<220>
<221> MOD_RES
<222> (4)..(4)
<223> L, I or V

<220>

<221> MOD_RES
<222> (6) .. (6)
<223> V, L or I

<220>
<221> MOD_RES
<222> (21) .. (21)
<223> A or V

<220>
<221> MOD_RES
<222> (27) .. (27)
<223> A, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> L, T or G

<220>
<221> MOD_RES
<222> (68) .. (68)
<223> K or R

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (104)..(104)
<223> F or V

<400> 17

```
Glu Glu Glu Xaa Gln Xaa Ile Gln Pro Asp Lys Phe Val Leu Val Ala
1               5                   10                  15

Ala Gly Glu Thr Xaa Thr Leu Arg Cys Thr Xaa Thr Ser Leu Xaa Pro
            20                  25                  30

Val Gly Pro Ile Gln Trp Phe Arg Gly Ala Gly Pro Gly Arg Xaa Leu
        35                  40                  45

Ile Tyr Asn Gln Xaa Xaa Gly Xaa Phe Pro Arg Val Thr Thr Val Ser
    50                  55                  60

Asp Xaa Thr Xaa Arg Asn Asn Met Asp Phe Ser Ile Arg Ile Gly Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Asp Val Glu Xaa Lys Ser Gly Ala Gly Thr Glu Leu
            100                 105                 110

Ser Val Arg Ala Lys Pro Ser
        115
```

<210> 18
<211> 119
<212> PRT
<213> Artificial Sequence

<220>
<223> Description of Artificial Sequence: Synthetic polypeptide

<220>
<221> MOD_RES
<222> (4)..(4)

<223> L, I or V

<220>
<221> MOD_RES
<222> (6) .. (6)
<223> V, L or I

<220>
<221> MOD_RES
<222> (21) .. (21)
<223> A or V

<220>
<221> MOD_RES
<222> (27) .. (27)
<223> A, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> L, T or G

<220>
<221> MOD_RES
<222> (68) .. (68)
<223> K or R

<220>
<221> MOD_RES
<222> (80)..(80)
<223> Any amino acid

<220>

<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (104)..(104)
<223> F or V

<400> 18

```
Glu Glu Glu Xaa Gln Xaa Ile Gln Pro Asp Lys Ser Val Leu Val Ala
1               5                   10                  15

Ala Gly Glu Thr Xaa Thr Leu Arg Cys Thr Xaa Thr Ser Leu Xaa Pro
            20                  25                  30

Val Gly Pro Ile Gln Trp Phe Arg Gly Ala Gly Pro Gly Arg Xaa Leu
        35                  40                  45

Ile Tyr Asn Gln Xaa Xaa Gly Xaa Phe Pro Arg Val Thr Thr Val Ser
    50                  55                  60

Asp Xaa Thr Xaa Arg Asn Asn Met Asp Phe Pro Ile Arg Ile Gly Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Asp Val Glu Xaa Lys Ser Gly Ala Gly Thr Glu Leu
            100                 105                 110

Ser Val Arg Ala Lys Pro Ser
        115
```

<210> 19
<211> 118
<212> PRT
<213> Artificial Sequence

<220>
<223> Description of Artificial Sequence: Synthetic polypeptide

<220>
<221> MOD_RES
<222> (4)..(4)
<223> L, I or V

<220>
<221> MOD_RES
<222> (6) .. (6)
<223> V, L or I

<220>
<221> MOD_RES
<222> (21) .. (21)
<223> A or V

<220>
<221> MOD_RES
<222> (27) .. (27)
<223> V, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> S, T or G

<220>
<221> MOD_RES
<222> (68) .. (68)
<223> K or R

<220>
<221> MOD_RES
<222> (80)..(80)

<223> Any amino acid

<220>
<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (103) .. (103)
<223> F or V

<400> 19

```
Glu Glu Glu Xaa Gln Xaa Ile Gln Pro Asp Lys Ser Val Ser Val Ala
1               5                   10                  15

Ala Gly Glu Ser Xaa Ile Leu His Cys Thr Xaa Thr Ser Leu Xaa Pro
            20                  25                  30

Val Gly Pro Ile Gln Trp Phe Arg Gly Ala Gly Pro Ala Arg Xaa Leu
        35                  40                  45

Ile Tyr Asn Gln Xaa Xaa Gly Xaa Phe Pro Arg Val Thr Thr Val Ser
    50                  55                  60

Glu Xaa Thr Xaa Arg Glu Asn Met Asp Phe Ser Ile Ser Ile Ser Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Thr Glu Xaa Lys Ser Gly Ala Gly Thr Glu Leu Ser
            100                 105                 110

Val Arg Gly Lys Pro Ser
        115
```

<210> 20
<211> 118
<212> PRT
<213> Artificial Sequence

<220>
<223> Description of Artificial Sequence: Synthetic polypeptide

<220>
<221> MOD_RES
<222> (4)..(4)
<223> L, I or V

<220>
<221> MOD_RES
<222> (6) .. (6)
<223> V, L or I

<220>
<221> MOD_RES
<222> (21) .. (21)
<223> A or V

<220>
<221> MOD_RES
<222> (27) .. (27)
<223> A, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> S, T or G

<220>
<221> MOD_RES
<222> (68) .. (68)
<223> K or R

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (103) .. (103)
<223> F or V

<400> 20

```
Glu Glu Glu Xaa Gln Xaa Ile Gln Pro Asp Lys Ser Val Leu Val Ala
1               5                   10                  15

Ala Gly Glu Thr Xaa Thr Leu Arg Cys Thr Xaa Thr Ser Leu Xaa Pro

            20                  25                  30

Val Gly Pro Ile Gln Trp Phe Arg Gly Ala Gly Pro Ala Arg Xaa Leu
        35                  40                  45

Ile Tyr Asn Gln Xaa Xaa Gly Xaa Phe Pro Arg Val Thr Thr Val Ser
    50                  55                  60

Glu Xaa Thr Xaa Arg Glu Asn Met Asp Phe Ser Ile Ser Ile Ser Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Thr Glu Xaa Lys Ser Gly Ala Gly Thr Glu Leu Ser
            100                 105                 110

Val Arg Ala Lys Pro Ser
        115
```

<210> 21
<211> 119
<212> PRT
<213> Artificial Sequence

<220>
<223> Description of Artificial Sequence: Synthetic polypeptide

<220>
<221> MOD_RES
<222> (4)..(4)
<223> L, I or V

<220>
<221> MOD_RES
<222> (6) .. (6)
<223> V, L or I

<220>
<221> MOD_RES
<222> (21) .. (21)
<223> A or V

<220>
<221> MOD_RES
<222> (27) .. (27)
<223> A, I or L

<220>
<221> MOD_RES
<222> (31)..(31)
<223> I, T, S or F

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> L, T or G

<220>
<221> MOD_RES
<222> (68) .. (68)
<223> K or R

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (104)..(104)
<223> F or V

<400> 21

```
Glu Glu Glu Xaa Gln Xaa Ile Gln Pro Asp Lys Ser Val Leu Val Ala
1               5                   10                  15

Ala Gly Glu Thr Xaa Thr Leu Arg Cys Thr Xaa Thr Ser Leu Xaa Pro
            20                  25                  30
```

Val Gly Pro Ile Gln Trp Phe Arg Gly Ala Gly Pro Gly Arg Xaa Leu
35 40 45

Ile Tyr Asn Gln Xaa Xaa Gly Xaa Phe Pro Arg Val Thr Thr Val Ser
50 55 60

Asp Xaa Thr Xaa Arg Asn Asn Met Asp Phe Ser Ile Arg Ile Ser Xaa
65 70 75 80

Ile Thr Xaa Ala Asp Ala Gly Thr Tyr Tyr Cys Xaa Lys Xaa Arg Lys
85 90 95

Gly Ser Pro Asp Asp Val Glu Xaa Lys Ser Gly Ala Gly Thr Glu Leu
100 105 110

Ser Val Arg Ala Lys Pro Ser
115

<210> 22
<211> 118
<212> PRT
<213> Artificial Sequence

<220>
<223> Description of Artificial Sequence: Synthetic polypeptide

<220>
<221> MOD_RES
<222> (4)..(4)
<223> L, I or V

<220>
<221> MOD_RES
<222> (6) .. (6)
<223> V, L or I

<220>
<221> MOD_RES
<222> (21) .. (21)
<223> A or V

<220>
<221> MOD_RES
<222> (27) .. (27)
<223> V, I or L

<220>
<221> MOD_RES
<222> (31)..(31)
<223> I, T, S or F

<220>
<221> MOD_RES
<222> (47)..(47)

<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (66) .. (66)
<223> S, T or G

<220>
<221> MOD_RES
<222> (68) .. (68)
<223> K or R

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (103) .. (103)
<223> F or V

<400> 22

Glu Glu Glu Xaa Gln Xaa Ile Gln Pro Asp Lys Ser Val Ser Val Ala
1               5                   10                  15

Ala Gly Glu Ser Xaa Ile Leu His Cys Thr Xaa Thr Ser Leu Xaa Pro
            20                  25                  30

Val Gly Pro Ile Gln Trp Phe Arg Gly Ala Gly Pro Ala Arg Xaa Leu
        35                  40                  45

Ile Tyr Asn Gln Xaa Xaa Gly Xaa Phe Pro Arg Val Thr Thr Val Ser
    50                  55                  60

Glu Xaa Thr Xaa Arg Glu Asn Met Asp Phe Ser Ile Ser Ile Ser Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Thr Glu Xaa Lys Ser Gly Ala Gly Thr Glu Leu Ser
            100                 105                 110

Val Arg Ala Lys Pro Ser
        115

<210> 23
<211> 119
<212> PRT
<213> Artificial Sequence

<220>
<223> Description of Artificial Sequence: Synthetic polypeptide

<220>
<221> MOD_RES
<222> (3)..(3)
<223> E or G

<220>
<221> MOD_RES
<222> (4)..(4)
<223> L, I or V

<220>
<221> MOD_RES
<222> (6) .. (6)
<223> V, L or I

<220>
<221> MOD_RES
<222> (12) .. (12)
<223> S or F

<220>

<221> MOD_RES
<222> (14)..(14)
<223> L or S

<220>
<221> MOD_RES
<222> (20) .. (20)
<223> S or T

<220>
<221> MOD_RES
<222> (21) .. (21)
<223> A or V

<220>
<221> MOD_RES
<222> (22) .. (22)
<223> I or T

<220>
<221> MOD_RES
<222> (24) .. (24)
<223> H or R

<220>
<221> MOD_RES
<222> (27) .. (27)
<223> A, V, I or L

<220>
<221> MOD_RES
<222> (31)..(31)
<223> I, T, S or F

<220>
<221> MOD_RES
<222> (45)..(45)
<223> A or G

<220>
<221> MOD_RES
<222> (47)..(47)
<223> E, V or L

<220>
<221> MOD_RES
<222> (53)..(53)
<223> K or R

<220>
<221> MOD_RES
<222> (54)..(54)
<223> E or Q

<220>
<221> MOD_RES
<222> (56) .. (56)
<223> H, P or R

<220>
<221> MOD_RES
<222> (65) .. (65)
<223> D or E

<220>
<221> MOD_RES
<222> (66) .. (66)
<223> S, L, T or G

<220>
<221> MOD_RES
<222> (68) .. (68)
<223> K or R

<220>
<221> MOD_RES
<222> (70)..(70)
<223> E or N

<220>
<221> MOD_RES
<222> (75)..(75)
<223> S or P

<220>
<221> MOD_RES
<222> (77)..(77)
<223> S or R

<220>
<221> MOD_RES
<222> (79) .. (79)
<223> S or G

<220>
<221> MOD_RES
<222> (80)..(80)
<223> Any amino acid

<220>
<221> MOD_RES
<222> (83)..(83)
<223> Any amino acid

<220>
<221> MOD_RES
<222> (92) .. (92)
<223> V or I

<220>
<221> MOD_RES
<222> (94) .. (94)
<223> F, L or V

<220>
<221> MOD_RES
<222> (101) .. (101)

<223> D or absent

<220>
<221> MOD_RES
<222> (102) .. (102)
<223> T or V

<220>
<221> MOD_RES
<222> (104)..(104)
<223> F or V

<220>
<221> MOD_RES
<222> (116) .. (116)
<223> A or G

<400> 23

```
Glu Glu Xaa Xaa Gln Xaa Ile Gln Pro Asp Lys Xaa Val Xaa Val Ala
1               5                   10                  15

Ala Gly Glu Xaa Xaa Xaa Leu Xaa Cys Thr Xaa Thr Ser Leu Xaa Pro
            20                  25                  30

Val Gly Pro Ile Gln Trp Phe Arg Gly Ala Gly Pro Xaa Arg Xaa Leu
        35                  40                  45

Ile Tyr Asn Gln Xaa Xaa Gly Xaa Phe Pro Arg Val Thr Thr Val Ser
    50                  55                  60

Xaa Xaa Thr Xaa Arg Xaa Asn Met Asp Phe Xaa Ile Xaa Ile Xaa Xaa
65                  70                  75                  80

Ile Thr Xaa Ala Asp Ala Gly Thr Tyr Tyr Cys Xaa Lys Xaa Arg Lys
                85                  90                  95

Gly Ser Pro Asp Xaa Xaa Glu Xaa Lys Ser Gly Ala Gly Thr Glu Leu
            100                 105                 110

Ser Val Arg Xaa Lys Pro Ser
        115
```

<210> 24
<211> 118
<212> PRT
<213> Homo sapiens

<400> 24

```
Glu Glu Glu Leu Gln Val Ile Gln Pro Asp Lys Ser Val Ser Val Ala
1               5                   10                  15

Ala Gly Glu Ser Ala Ile Leu His Cys Thr Ile Thr Ser Leu Ile Pro
            20                  25                  30

Val Gly Pro Ile Gln Trp Phe Arg Gly Ala Gly Pro Ala Arg Glu Leu
        35                  40                  45

Ile Tyr Asn Gln Arg Glu Gly His Phe Pro Arg Val Thr Thr Val Ser
    50                  55                  60

Glu Thr Thr Arg Arg Glu Asn Met Asp Phe Ser Ile Ser Ile Ser Asn
65                  70                  75                  80

Ile Thr Pro Ala Asp Ala Gly Thr Tyr Tyr Cys Val Lys Phe Arg Lys
                85                  90                  95

Gly Ser Pro Asp Thr Glu Val Lys Ser Gly Ala Gly Thr Glu Leu Ser
            100                 105                 110

Val Arg Ala Lys Pro Ser
        115
```

<210> 25
<211> 118
<212> PRT
<213> Homo sapiens

<400> 25

Glu Glu Glu Val Gln Val Ile Gln Pro Asp Lys Ser Val Ser Val Ala
1               5                   10                  15

Ala Gly Glu Ser Ala Ile Leu His Cys Thr Leu Thr Ser Leu Ile Pro
            20                  25                  30

Val Gly Pro Ile Gln Trp Phe Arg Gly Ala Gly Pro Ala Arg Val Leu
        35                  40                  45

Ile Tyr Asn Gln Arg Gln Gly His Phe Pro Arg Val Thr Thr Val Ser
    50                  55                  60

Glu Gly Thr Arg Arg Glu Asn Met Asp Phe Ser Ile Ser Ile Ser Asn
65                  70                  75                  80

Ile Thr Pro Ala Asp Ala Gly Thr Tyr Tyr Cys Ile Lys Phe Arg Lys
                85                  90                  95

Gly Ser Pro Asp Thr Glu Phe Lys Ser Gly Ala Gly Thr Glu Leu Ser
            100                 105                 110

Val Arg Ala Lys Pro Ser
        115

<210> 26
<211> 118
<212> PRT
<213> Homo sapiens

<400> 26

Glu Glu Glu Val Gln Ile Ile Gln Pro Asp Lys Ser Val Ser Val Ala
1               5                   10                  15

Ala Gly Glu Ser Val Ile Leu His Cys Thr Ile Thr Ser Leu Thr Pro
            20                  25                  30

Val Gly Pro Ile Gln Trp Phe Arg Gly Ala Gly Pro Ala Arg Leu Leu
        35                  40                  45

Ile Tyr Asn Gln Arg Glu Gly Pro Phe Pro Arg Val Thr Thr Val Ser
    50                  55                  60

```
Glu Thr Thr Arg Arg Glu Asn Met Asp Phe Ser Ile Ser Ile Ser Asn
65                  70                  75                  80

Ile Thr Pro Ala Asp Ala Gly Thr Tyr Tyr Cys Val Lys Leu Arg Lys
                85                  90                  95

Gly Ser Pro Asp Thr Glu Phe Lys Ser Gly Ala Gly Thr Glu Leu Ser
            100                 105                 110

Val Arg Ala Lys Pro Ser
        115
```

<210> 27
<211> 118
<212> PRT
<213> Homo sapiens

<400> 27

```
Glu Glu Glu Leu Gln Ile Ile Gln Pro Asp Lys Ser Val Ser Val Ala
1               5                   10                  15

Ala Gly Glu Ser Ala Ile Leu His Cys Thr Ile Thr Ser Leu Ser Pro
            20                  25                  30

Val Gly Pro Ile Gln Trp Phe Arg Gly Ala Gly Pro Ala Arg Val Leu
        35                  40                  45

Ile Tyr Asn Gln Arg Gln Gly Pro Phe Pro Arg Val Thr Thr Val Ser
    50                  55                  60

Glu Gly Thr Lys Arg Glu Asn Met Asp Phe Ser Ile Ser Ile Ser Asn
65                  70                  75                  80

Ile Thr Pro Ala Asp Ala Gly Thr Tyr Tyr Cys Ile Lys Leu Arg Lys
                85                  90                  95

Gly Ser Pro Asp Thr Glu Phe Lys Ser Gly Ala Gly Thr Glu Leu Ser
            100                 105                 110

Val Arg Ala Lys Pro Ser
        115
```

<210> 28
<211> 118
<212> PRT
<213> Homo sapiens

<400> 28

```
Glu Glu Glu Ile Gln Val Ile Gln Pro Asp Lys Ser Val Ser Val Ala
1               5                   10                  15

Ala Gly Glu Ser Val Ile Ile His Cys Thr Val Thr Ser Leu Phe Pro
            20                  25                  30

Val Gly Pro Ile Gln Trp Phe Arg Gly Ala Gly Pro Ala Arg Val Leu
        35                  40                  45

Ile Tyr Asn Gln Arg Gln Gly Arg Phe Pro Arg Val Thr Thr Val Ser
    50                  55                  60

Glu Gly Thr Lys Arg Glu Asn Met Asp Phe Ser Ile Ser Ile Ser Asn
65                  70                  75                  80

Ile Thr Pro Ala Asp Ala Gly Thr Tyr Tyr Cys Val Lys Val Arg Lys
                85                  90                  95

Gly Ser Pro Asp Thr Glu Val Lys Ser Gly Ala Gly Thr Glu Leu Ser
            100                 105                 110

Val Arg Ala Lys Pro Ser
        115
```

<210> 29
<211> 118
<212> PRT
<213> Homo sapiens

<400> 29

```
Glu Glu Glu Val Gln Ile Ile Gln Pro Asp Lys Ser Val Ser Val Ala
1               5                   10                  15

Ala Gly Glu Ser Ile Ile Leu His Cys Thr Val Thr Ser Leu Phe Pro
            20                  25                  30

Val Gly Pro Ile Gln Trp Phe Arg Gly Ala Gly Pro Ala Arg Val Leu
        35                  40                  45

Ile Tyr Asn Gln Arg Glu Gly Arg Phe Pro Arg Val Thr Thr Val Ser
    50                  55                  60

Glu Gly Thr Arg Arg Glu Asn Met Asp Phe Ser Ile Ser Ile Ser Asn
65                  70                  75                  80

Ile Thr Pro Ala Asp Ala Gly Thr Tyr Tyr Cys Ile Lys Leu Arg Lys
                85                  90                  95

Gly Ser Pro Asp Thr Glu Phe Lys Ser Gly Ala Gly Thr Glu Leu Ser
            100                 105                 110

Val Arg Ala Lys Pro Ser
        115
```

<210> 30
<211> 118
<212> PRT
<213> Homo sapiens

<400> 30

```
Glu Glu Glu Val Gln Leu Ile Gln Pro Asp Lys Ser Val Ser Val Ala
1               5                   10                  15

Ala Gly Glu Ser Ala Ile Leu His Cys Thr Val Thr Ser Leu Phe Pro
            20                  25                  30

Val Gly Pro Ile Gln Trp Phe Arg Gly Ala Gly Pro Ala Arg Val Leu
        35                  40                  45

Ile Tyr Asn Gln Arg Glu Gly Pro Phe Pro Arg Val Thr Thr Val Ser
    50                  55                  60

Glu Gly Thr Lys Arg Glu Asn Met Asp Phe Ser Ile Ser Ile Ser Asn
65                  70                  75                  80

Ile Thr Pro Ala Asp Ala Gly Thr Tyr Tyr Cys Ile Lys Phe Arg Lys
                85                  90                  95

Gly Ser Pro Asp Thr Glu Val Lys Ser Gly Ala Gly Thr Glu Leu Ser
            100                 105                 110

Val Arg Ala Lys Pro Ser
        115
```

<210> 31
<211> 119
<212> PRT
<213> Homo sapiens

<400> 31

```
Glu Glu Glu Leu Gln Ile Ile Gln Pro Asp Lys Ser Val Leu Val Ala
1               5                   10                  15

Ala Gly Glu Thr Ala Thr Leu Arg Cys Thr Ile Thr Ser Leu Phe Pro
            20                  25                  30

Val Gly Pro Ile Gln Trp Phe Arg Gly Ala Gly Pro Gly Arg Val Leu
        35                  40                  45

Ile Tyr Asn Gln Arg Gln Gly Pro Phe Pro Arg Val Thr Thr Val Ser
```

50 55 60

Asp Thr Thr Lys Arg Asn Asn Met Asp Phe Ser Ile Arg Ile Gly Asn
65 70 75 80

Ile Thr Pro Ala Asp Ala Gly Thr Tyr Tyr Cys Ile Lys Phe Arg Lys
85 90 95

Gly Ser Pro Asp Asp Val Glu Phe Lys Ser Gly Ala Gly Thr Glu Leu
100 105 110

Ser Val Arg Ala Lys Pro Ser
115

<210> 32
<211> 118
<212> PRT
<213> Homo sapiens

<400> 32

Glu Glu Glu Leu Gln Ile Ile Gln Pro Asp Lys Ser Val Ser Val Ala
1 5 10 15

Ala Gly Glu Ser Ala Ile Leu His Cys Thr Ile Thr Ser Leu Phe Pro
20 25 30

Val Gly Pro Ile Gln Trp Phe Arg Gly Ala Gly Pro Ala Arg Leu Leu
35 40 45

Ile Tyr Asn Gln Arg Gln Gly Pro Phe Pro Arg Val Thr Thr Val Ser
50 55 60

Glu Thr Thr Lys Arg Glu Asn Met Asp Phe Ser Ile Ser Ile Ser Asn
65 70 75 80

Ile Thr Pro Ala Asp Ala Gly Thr Tyr Tyr Cys Val Lys Phe Arg Lys
85 90 95

Gly Ser Pro Asp Thr Glu Phe Lys Ser Gly Ala Gly Thr Glu Leu Ser
100 105 110

Val Arg Ala Lys Pro Ser
115

<210> 33
<211> 118
<212> PRT

<213> Homo sapiens

<400> 33

```
Glu Glu Glu Val Gln Ile Ile Gln Pro Asp Lys Ser Val Ser Val Ala
1               5                   10                  15

Ala Gly Glu Ser Ala Ile Leu His Cys Thr Ile Thr Ser Leu Phe Pro
            20                  25                  30

Val Gly Pro Ile Gln Trp Phe Arg Gly Ala Gly Pro Ala Arg Val Leu
        35                  40                  45

Ile Tyr Asn Gln Lys Gln Gly Pro Phe Pro Arg Val Thr Thr Ile Ser
    50                  55                  60

Glu Thr Thr Arg Arg Glu Asn Met Asp Phe Ser Ile Ser Ile Ser Asn
65                  70                  75                  80

Ile Thr Pro Ala Asp Ala Gly Thr Tyr Tyr Cys Ile Lys Phe Arg Lys
                85                  90                  95

Gly Ser Pro Asp Thr Glu Phe Lys Ser Gly Ala Gly Thr Glu Leu Ser
            100                 105                 110

Val Arg Ala Lys Pro Ser
        115
```

<210> 34
<211> 118
<212> PRT
<213> Homo sapiens

<400> 34

```
Glu Glu Glu Leu Gln Ile Ile Gln Pro Asp Lys Ser Val Ser Val Ala
1               5                   10                  15

Ala Gly Glu Ser Ala Ile Leu His Cys Thr Ile Thr Ser Leu Thr Pro
            20                  25                  30

Val Gly Pro Ile Gln Trp Phe Arg Gly Ala Gly Pro Ala Arg Val Leu
        35                  40                  45

Ile Tyr Asn Gln Arg Gln Gly Pro Phe Pro Arg Val Thr Thr Val Ser
    50                  55                  60

Glu Gly Thr Arg Arg Glu Asn Met Asp Phe Ser Ile Ser Ile Ser Asn
65                  70                  75                  80

Ile Thr Pro Ala Asp Ala Gly Thr Tyr Tyr Cys Ile Lys Phe Arg Lys
                85                  90                  95

Gly Ser Pro Asp Thr Glu Val Lys Ser Gly Ala Gly Thr Glu Leu Ser

                100                 105                 110

    Val Arg Ala Lys Pro Ser
            115
```

<210> 35
<211> 123
<212> PRT
<213> Homo sapiens

<400> 35

Gln Leu Leu Phe Asn Lys Thr Lys Ser Val Glu Phe Thr Phe Cys Asn
1               5                   10                  15

Asp Thr Val Val Ile Pro Cys Phe Val Thr Asn Met Glu Ala Gln Asn
            20                  25                  30

Thr Thr Glu Val Tyr Val Lys Trp Lys Phe Lys Gly Arg Asp Ile Tyr
        35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65                  70                  75                  80

Lys Met Asp Lys Ser Asp Ala Val Ser His Thr Gly Asn Tyr Thr Cys
                85                  90                  95

Glu Val Thr Glu Leu Thr Arg Glu Gly Glu Thr Ile Ile Glu Leu Lys
            100                 105                 110

Tyr Arg Val Val Ser Trp Phe Ser Pro Asn Glu
        115                 120

<210> 36
<211> 10
<212> PRT
<213> Artificial Sequence

<220>
<223> Description of Artificial Sequence: Synthetic peptide

<400> 36

Glu Val Thr Glu Leu Thr Arg Glu Gly Glu
1               5                   10

<210> 37
<211> 12
<212> PRT
<213> Artificial Sequence

<220>
<223> Description of Artificial Sequence: Synthetic peptide

<400> 37

Cys Glu Val Thr Glu Leu Thr Arg Glu Gly Glu Cys
1               5                   10

<210> 38
<211> 12

<212> PRT
<213> Artificial Sequence

<220>
<223> Description of Artificial Sequence: Synthetic peptide

<400> 38

```
Ser Glu Val Thr Glu Leu Thr Arg Glu Gly Glu Thr
1               5                   10
```

<210> 39
<211> 21
<212> PRT
<213> Artificial Sequence

<220>
<223> Description of Artificial Sequence: Synthetic peptide

<400> 39

```
Gly Asn Tyr Thr Cys Glu Val Thr Glu Leu Thr Arg Glu Gly Glu Thr
1               5                   10                  15

Ile Ile Glu Leu Lys
            20
```

<210> 40
<211> 21
<212> PRT
<213> Artificial Sequence

<220>
<223> Description of Artificial Sequence: Synthetic peptide

<400> 40

```
Gly Gln Tyr Thr Ser Glu Val Thr Glu Leu Thr Arg Glu Gly Glu Thr
1               5                   10                  15

Ile Ile Glu Leu Lys
            20
```

<210> 41
<211> 123
<212> **PRT**
<213> Artificial Sequence

<220>
<223> Description of Artificial Sequence: Synthetic polypeptide

<400> 41

Gln Leu Leu Phe Asn Lys Thr Lys Ser Val Glu Phe Thr Phe Cys Asn
1               5                   10                  15

Asp Thr Val Val Ile Pro Cys Phe Val Thr Asn Met Glu Ala Gln Asn
            20                  25                  30

Thr Thr Glu Val Tyr Val Lys Trp Lys Phe Lys Gly Arg Asp Ile Tyr
        35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65                  70                  75                  80

Lys Met Asp Lys Ser Asp Ala Val Ser His Thr Gly Asn Tyr Thr Cys
                85                  90                  95

Glu Val Thr Glu Gln Thr Arg Glu Gly Glu Thr Ile Ile Glu Leu Lys
            100                 105                 110

Tyr Arg Val Val Ser Trp Phe Ser Pro Asn Glu
        115                 120

<210> 42
<211> 123
<212> PRT
<213> Artificial Sequence

<220>
<223> Description of Artificial Sequence: Synthetic polypeptide

<400> 42

Gln Leu Leu Phe Asn Lys Thr Lys Ser Val Glu Phe Thr Phe Cys Asn
1               5                   10                  15

Asp Thr Val Val Ile Pro Cys Phe Val Thr Asn Met Glu Ala Gln Asn
            20                  25                  30

Thr Thr Glu Val Tyr Val Lys Trp Lys Phe Lys Gly Arg Asp Ile Tyr
        35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65 70 75 80

Lys Met Asp Lys Ser Asp Ala Val Ser His Thr Gly Asn Tyr Thr Cys
85 90 95

Glu Val Thr Glu Tyr Thr Arg Glu Gly Glu Thr Ile Ile Glu Leu Lys
100 105 110

Tyr Arg Val Val Ser Trp Phe Ser Pro Asn Glu
115 120

<210> 43
<211> 123
<212> PRT
<213> Artificial Sequence

<220>
<223> Description of Artificial Sequence: Synthetic polypeptide

<400> 43

Gln Leu Leu Phe Asn Lys Thr Lys Ser Val Glu Phe Thr Phe Cys Asn
1 5 10 15

Asp Thr Val Val Ile Pro Cys Phe Val Thr Asn Met Glu Ala Gln Asn
20 25 30

Thr Thr Glu Val Tyr Val Lys Trp Lys Phe Lys Gly Arg Asp Ile Tyr
35 40 45

Thr Phe Asp Gly Ala Leu Asn Lys Ser Thr Val Pro Thr Asp Phe Ser
50 55 60

Ser Ala Lys Ile Glu Val Ser Gln Leu Leu Lys Gly Asp Ala Ser Leu
65 70 75 80

Lys Met Asp Lys Ser Asp Ala Val Ser His Thr Gly Asn Tyr Thr Cys
85 90 95

Glu Val Thr Glu His Thr Arg Glu Gly Glu Thr Ile Ile Glu Leu Lys
100 105 110

Tyr Arg Val Val Ser Trp Phe Ser Pro Asn Glu
115 120

<210> **44**
<211> 123
<212> **PRT**

<213> Artificial Sequence

<220>
<223> Description of Artificial Sequence: Synthetic polypeptide

<400> 44

```
Gln Leu Leu Phe Asn Lys Thr Lys Ser Val Glu Phe Thr Phe Cys Asn
1               5                   10                  15

Asp Thr Val Val Ile Pro Cys Phe Val Thr Asn Met Glu Ala Gln Asn
            20                  25                  30

Thr Thr Glu Val Tyr Val Lys Trp Lys Phe Lys Gly Arg Asp Ile Tyr
        35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65                  70                  75                  80

Lys Met Asp Lys Ser Asp Ala Val Ser His Thr Gly Asn Tyr Thr Cys
                85                  90                  95

Glu Val Thr Glu Leu Gln Arg Glu Gly Glu Thr Ile Ile Glu Leu Lys
            100                 105                 110

Tyr Arg Val Val Ser Trp Phe Ser Pro Asn Glu
        115                 120
```

<210> 45
<211> 123
<212> PRT
<213> Artificial Sequence

<220>
<223> Description of Artificial Sequence: Synthetic polypeptide

<400> 45

```
Gln Leu Leu Phe Asn Lys Thr Lys Ser Val Glu Phe Thr Phe Cys Asn
1               5                   10                  15

Asp Thr Val Val Ile Pro Cys Phe Val Thr Asn Met Glu Ala Gln Asn
            20                  25                  30

Thr Thr Glu Val Tyr Val Lys Trp Lys Phe Lys Gly Arg Asp Ile Tyr
        35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65                  70                  75                  80

Lys Met Asp Lys Ser Asp Ala Val Ser His Thr Gly Asn Tyr Thr Cys
                85                  90                  95

Glu Val Thr Glu Leu His Arg Glu Gly Glu Thr Ile Ile Glu Leu Lys
            100                 105                 110

Tyr Arg Val Val Ser Trp Phe Ser Pro Asn Glu
        115                 120
```

<210> 46
<211> 124
<212> PRT
<213> Artificial Sequence

<220>
<223> Description of Artificial Sequence: Synthetic polypeptide

<400> 46

Gly Gln Leu Leu Phe Asn Lys Thr Lys Ser Val Glu Phe Thr Phe Cys
1               5                   10                  15

Asn Asp Thr Val Val Ile Pro Cys Phe Val Thr Asn Met Glu Ala Gln
            20                  25                  30

Asn Thr Thr Glu Val Tyr Val Lys Trp Lys Phe Lys Gly Arg Asp Ile
        35                  40                  45

Tyr Thr Phe Asp Gly Ala Leu Asn Lys Ser Thr Val Pro Thr Asp Phe
    50                  55                  60

Ser Ser Ala Lys Ile Glu Val Ser Gln Leu Leu Lys Gly Asp Ala Ser
65                  70                  75                  80

Leu Lys Met Asp Lys Ser Asp Ala Val Ser His Thr Gly Asn Tyr Thr
                85                  90                  95

Cys Glu Val Thr Glu Leu Thr Arg Glu Gly Glu Thr Ile Ile Glu Leu
            100                 105                 110

Lys Tyr Arg Val Val Ser Trp Phe Ser Pro Asn Glu
        115                 120

<210> 47
<211> 8
<212> **PRT**
<213> Unknown

<220>
<223> Description of Unknown: Cleavable peptide

<400> 47

Leu Ser Gly Arg Ser Asp Asn His
1               5

<210> 48
<211> 159
<212> PRT
<213> Artificial Sequence

<220>
<223> Description of Artificial Sequence: Synthetic polypeptide

<400> 48

```
Ser Glu Val Thr Glu Leu Thr Arg Glu Gly Glu Thr Gly Gly Gly Gly
1               5                   10                  15

Ser Gly Gly Gly Gly Ser Leu Ser Gly Arg Ser Asp Asn His Gly Gly
            20                  25                  30

Gly Gly Ser Gly Gly Gly Gly Ser Glu Glu Glu Leu Gln Ile Ile Gln
        35                  40                  45

Pro Asp Lys Ser Val Leu Val Ala Ala Gly Glu Thr Ala Thr Leu Arg
    50                  55                  60

Cys Thr Ile Thr Ser Leu Phe Pro Val Gly Pro Ile Gln Trp Phe Arg
65                  70                  75                  80

Gly Ala Gly Pro Gly Arg Val Leu Ile Tyr Asn Gln Arg Gln Gly Pro
                85                  90                  95

Phe Pro Arg Val Thr Thr Val Ser Asp Thr Thr Lys Arg Asn Asn Met
            100                 105                 110

Asp Phe Ser Ile Arg Ile Gly Asn Ile Thr Pro Ala Asp Ala Gly Thr
        115                 120                 125

Tyr Tyr Cys Ile Lys Phe Arg Lys Gly Ser Pro Asp Asp Val Glu Phe
    130                 135                 140

Lys Ser Gly Ala Gly Thr Glu Leu Ser Val Arg Ala Lys Pro Ser
145                 150                 155
```

<210> 49
<211> 164
<212> PRT
<213> Artificial Sequence

<220>
<223> Description of Artificial Sequence: Synthetic polypeptide

<400> 49

```
Ser Glu Val Thr Glu Leu Thr Arg Glu Gly Glu Thr Gly Gly Gly Gly
1               5                   10                  15

Ser Gly Gly Gly Gly Ser Gly Gly Gly Gly Ser Leu Ser Gly Arg Ser
            20                  25                  30

Asp Asn His Gly Gly Gly Gly Ser Gly Gly Gly Gly Ser Glu Glu Glu
        35                  40                  45

Leu Gln Ile Ile Gln Pro Asp Lys Ser Val Leu Val Ala Ala Gly Glu
    50                  55                  60

Thr Ala Thr Leu Arg Cys Thr Ile Thr Ser Leu Phe Pro Val Gly Pro
65                  70                  75                  80

Ile Gln Trp Phe Arg Gly Ala Gly Pro Gly Arg Val Leu Ile Tyr Asn
                85                  90                  95

Gln Arg Gln Gly Pro Phe Pro Arg Val Thr Thr Val Ser Asp Thr Thr
            100                 105                 110

Lys Arg Asn Asn Met Asp Phe Ser Ile Arg Ile Gly Asn Ile Thr Pro
        115                 120                 125

Ala Asp Ala Gly Thr Tyr Tyr Cys Ile Lys Phe Arg Lys Gly Ser Pro
    130                 135                 140

Asp Asp Val Glu Phe Lys Ser Gly Ala Gly Thr Glu Leu Ser Val Arg
145                 150                 155                 160

Ala Lys Pro Ser
```

<210> 50
<211> 168
<212> PRT
<213> Artificial Sequence

<220>
<223> Description of Artificial Sequence: Synthetic polypeptide

<400> 50

```
Gly Gln Tyr Thr Ser Glu Val Thr Glu Leu Thr Arg Glu Gly Glu Thr
1               5                   10                  15
```

```
Ile Ile Glu Leu Lys Gly Gly Gly Gly Ser Gly Gly Gly Gly Ser Leu
            20                  25                  30

Ser Gly Arg Ser Asp Asn His Gly Gly Gly Gly Ser Gly Gly Gly Gly
        35                  40                  45

Ser Glu Glu Glu Leu Gln Ile Ile Gln Pro Asp Lys Ser Val Leu Val
    50                  55                  60

Ala Ala Gly Glu Thr Ala Thr Leu Arg Cys Thr Ile Thr Ser Leu Phe
65                  70                  75                  80

Pro Val Gly Pro Ile Gln Trp Phe Arg Gly Ala Gly Pro Gly Arg Val
                85                  90                  95

Leu Ile Tyr Asn Gln Arg Gln Gly Pro Phe Pro Arg Val Thr Thr Val
            100                 105                 110

Ser Asp Thr Thr Lys Arg Asn Asn Met Asp Phe Ser Ile Arg Ile Gly
        115                 120                 125

Asn Ile Thr Pro Ala Asp Ala Gly Thr Tyr Tyr Cys Ile Lys Phe Arg
    130                 135                 140

Lys Gly Ser Pro Asp Asp Val Glu Phe Lys Ser Gly Ala Gly Thr Glu
145                 150                 155                 160

Leu Ser Val Arg Ala Lys Pro Ser
                165
```

<210> 51
<211> 173
<212> PRT
<213> Artificial Sequence

<220>
<223> Description of Artificial Sequence: Synthetic polypeptide

<400> 51

```
Gly Gln Tyr Thr Ser Glu Val Thr Glu Leu Thr Arg Glu Gly Glu Thr
1               5                   10                  15

Ile Ile Glu Leu Lys Gly Gly Gly Gly Ser Gly Gly Gly Gly Ser Gly
            20                  25                  30

Gly Gly Gly Ser Leu Ser Gly Arg Ser Asp Asn His Gly Gly Gly Gly
        35                  40                  45
```

Ser Gly Gly Gly Gly Ser Glu Glu Glu Leu Gln Ile Ile Gln Pro Asp
50 55 60

Lys Ser Val Leu Val Ala Ala Gly Glu Thr Ala Thr Leu Arg Cys Thr
65 70 75 80

Ile Thr Ser Leu Phe Pro Val Gly Pro Ile Gln Trp Phe Arg Gly Ala
85 90 95

Gly Pro Gly Arg Val Leu Ile Tyr Asn Gln Arg Gln Gly Pro Phe Pro
100 105 110

Arg Val Thr Thr Val Ser Asp Thr Thr Lys Arg Asn Asn Met Asp Phe
115 120 125

Ser Ile Arg Ile Gly Asn Ile Thr Pro Ala Asp Ala Gly Thr Tyr Tyr
130 135 140

Cys Ile Lys Phe Arg Lys Gly Ser Pro Asp Asp Val Glu Phe Lys Ser
145 150 155 160

Gly Ala Gly Thr Glu Leu Ser Val Arg Ala Lys Pro Ser
165 170

<210> 52
<211> 269
<212> PRT
<213> Artificial Sequence

<220>
<223> Description of Artificial Sequence: Synthetic polypeptide

<400> 52

Gln Leu Leu Phe Asn Lys Thr Lys Ser Val Glu Phe Thr Phe Ser Asn
1 5 10 15

Asp Thr Val Val Ile Pro Cys Phe Val Thr Asn Met Glu Ala Gln Asn
20 25 30

Thr Thr Glu Val Tyr Val Lys Trp Lys Phe Lys Gly Arg Asp Ile Tyr
35 40 45

Thr Phe Asp Gly Ala Leu Asn Lys Ser Thr Val Pro Thr Asp Phe Ser
50 55 60

Ser Ala Lys Ile Glu Val Ser Gln Leu Leu Lys Gly Asp Ala Ser Leu
65 70 75 80

```
Lys Met Asp Lys Ser Asp Ala Val Ser His Thr Gly Asn Tyr Thr Cys
                85                  90                  95

Glu Val Thr Glu Leu Thr Arg Glu Gly Glu Thr Ile Ile Glu Leu Lys
            100                 105                 110

Tyr Arg Val Val Ser Gly Gly Gly Gly Ser Gly Gly Gly Gly Ser Gly
        115                 120                 125

Gly Gly Gly Ser Leu Ser Gly Arg Ser Asp Asn His Gly Gly Gly Gly
    130                 135                 140

Ser Gly Gly Gly Gly Ser Glu Glu Glu Leu Gln Ile Ile Gln Pro Asp
145                 150                 155                 160

Lys Ser Val Leu Val Ala Ala Gly Glu Thr Ala Thr Leu Arg Cys Thr
                165                 170                 175

Ile Thr Ser Leu Phe Pro Val Gly Pro Ile Gln Trp Phe Arg Gly Ala
            180                 185                 190

Gly Pro Gly Arg Val Leu Ile Tyr Asn Gln Arg Gln Gly Pro Phe Pro
        195                 200                 205

Arg Val Thr Thr Val Ser Asp Thr Thr Lys Arg Asn Asn Met Asp Phe
    210                 215                 220

Ser Ile Arg Ile Gly Asn Ile Thr Pro Ala Asp Ala Gly Thr Tyr Tyr
225                 230                 235                 240

Cys Ile Lys Phe Arg Lys Gly Ser Pro Asp Asp Val Glu Phe Lys Ser
                245                 250                 255

Gly Ala Gly Thr Glu Leu Ser Val Arg Ala Lys Pro Ser
            260                 265
```

<210> 53
<211> 274
<212> PRT
<213> Artificial Sequence

<220>
<223> Description of Artificial Sequence: Synthetic polypeptide

<400> 53

```
Gln Leu Leu Phe Asn Lys Thr Lys Ser Val Glu Phe Thr Phe Ser Asn
1               5                   10                  15

Asp Thr Val Val Ile Pro Cys Phe Val Thr Asn Met Glu Ala Gln Asn
```

```
                20                  25                  30

Thr Thr Glu Val Tyr Val Lys Trp Lys Phe Lys Gly Arg Asp Ile Tyr
            35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65                  70                  75                  80

Lys Met Asp Lys Ser Asp Ala Val Ser His Thr Gly Asn Tyr Thr Cys
                85                  90                  95

Glu Val Thr Glu Leu Thr Arg Glu Gly Glu Thr Ile Ile Glu Leu Lys
            100                 105                 110

Tyr Arg Val Val Ser Gly Gly Gly Gly Ser Gly Gly Gly Gly Ser Gly
        115                 120                 125

Gly Gly Gly Ser Gly Gly Gly Gly Ser Leu Ser Gly Arg Ser Asp Asn
    130                 135                 140

His Gly Gly Gly Gly Ser Gly Gly Gly Gly Ser Glu Glu Glu Leu Gln
145                 150                 155                 160

Ile Ile Gln Pro Asp Lys Ser Val Leu Val Ala Ala Gly Glu Thr Ala
                165                 170                 175

Thr Leu Arg Cys Thr Ile Thr Ser Leu Phe Pro Val Gly Pro Ile Gln
            180                 185                 190

Trp Phe Arg Gly Ala Gly Pro Gly Arg Val Leu Ile Tyr Asn Gln Arg
        195                 200                 205

Gln Gly Pro Phe Pro Arg Val Thr Thr Val Ser Asp Thr Thr Lys Arg
    210                 215                 220

Asn Asn Met Asp Phe Ser Ile Arg Ile Gly Asn Ile Thr Pro Ala Asp
225                 230                 235                 240

Ala Gly Thr Tyr Tyr Cys Ile Lys Phe Arg Lys Gly Ser Pro Asp Asp
                245                 250                 255

Val Glu Phe Lys Ser Gly Ala Gly Thr Glu Leu Ser Val Arg Ala Lys
            260                 265                 270
```

Pro Ser

<210> 54
<211> 279
<212> PRT
<213> Artificial Sequence

<220>
<223> Description of Artificial Sequence: Synthetic polypeptide

<400> 54

```
Gln Leu Leu Phe Asn Lys Thr Lys Ser Val Glu Phe Thr Phe Ser Asn
1               5                   10                  15

Asp Thr Val Val Ile Pro Cys Phe Val Thr Asn Met Glu Ala Gln Asn
            20                  25                  30

Thr Thr Glu Val Tyr Val Lys Trp Lys Phe Lys Gly Arg Asp Ile Tyr
        35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65                  70                  75                  80

Lys Met Asp Lys Ser Asp Ala Val Ser His Thr Gly Asn Tyr Thr Cys
                85                  90                  95

Glu Val Thr Glu Leu Thr Arg Glu Gly Glu Thr Ile Ile Glu Leu Lys
            100                 105                 110

Tyr Arg Val Val Ser Gly Gly Gly Gly Ser Gly Gly Gly Gly Ser Gly
        115                 120                 125

Gly Gly Gly Ser Gly Gly Gly Gly Ser Gly Gly Gly Gly Ser Leu Ser
    130                 135                 140

Gly Arg Ser Asp Asn His Gly Gly Gly Gly Ser Gly Gly Gly Gly Ser
145                 150                 155                 160

Glu Glu Glu Leu Gln Ile Ile Gln Pro Asp Lys Ser Val Leu Val Ala
                165                 170                 175

Ala Gly Glu Thr Ala Thr Leu Arg Cys Thr Ile Thr Ser Leu Phe Pro
            180                 185                 190

Val Gly Pro Ile Gln Trp Phe Arg Gly Ala Gly Pro Gly Arg Val Leu
```

```
            195                 200                 205

Ile Tyr Asn Gln Arg Gln Gly Pro Phe Pro Arg Val Thr Thr Val Ser
    210                 215                 220

Asp Thr Thr Lys Arg Asn Asn Met Asp Phe Ser Ile Arg Ile Gly Asn
225                 230                 235                 240

Ile Thr Pro Ala Asp Ala Gly Thr Tyr Tyr Cys Ile Lys Phe Arg Lys
                245                 250                 255

Gly Ser Pro Asp Asp Val Glu Phe Lys Ser Gly Ala Gly Thr Glu Leu
            260                 265                 270

Ser Val Arg Ala Lys Pro Ser
        275
```

<210> 55
<211> 265
<212> PRT
<213> Artificial Sequence

<220>
<223> Description of Artificial Sequence: Synthetic polypeptide

<400> 55

```
Glu Glu Glu Leu Gln Ile Ile Gln Pro Asp Lys Ser Val Leu Val Ala
1               5                   10                  15

Ala Gly Glu Thr Ala Thr Leu Arg Cys Thr Ile Thr Ser Leu Phe Pro
            20                  25                  30

Val Gly Pro Ile Gln Trp Phe Arg Gly Ala Gly Pro Gly Arg Val Leu
        35                  40                  45

Ile Tyr Asn Gln Arg Gln Gly Pro Phe Pro Arg Val Thr Thr Val Ser
    50                  55                  60

Asp Thr Thr Lys Arg Asn Asn Met Asp Phe Ser Ile Arg Ile Gly Asn
65                  70                  75                  80

Ile Thr Pro Ala Asp Ala Gly Thr Tyr Tyr Cys Ile Lys Phe Arg Lys
                85                  90                  95

Gly Ser Pro Asp Asp Val Glu Phe Lys Ser Gly Ala Gly Thr Glu Leu
            100                 105                 110

Ser Val Arg Ala Lys Pro Ser Gly Gly Ser Gly Gly Ser Gly Gly Ser
        115                 120                 125
```

Leu Ser Gly Arg Ser Asp Asn His Gly Gly Ser Gly Gly Ser Gly Gly
    130                 135                 140

Ser Gly Gly Ser Gln Leu Leu Phe Asn Lys Thr Lys Ser Val Glu Phe
145                 150                 155                 160

Thr Phe Ser Asn Asp Thr Val Val Ile Pro Cys Phe Val Thr Asn Met
                165                 170                 175

Glu Ala Gln Asn Thr Thr Glu Val Tyr Val Lys Trp Lys Phe Lys Gly
            180                 185                 190

Arg Asp Ile Tyr Thr Phe Asp Gly Ala Leu Asn Lys Ser Thr Val Pro
        195                 200                 205

Thr Asp Phe Ser Ser Ala Lys Ile Glu Val Ser Gln Leu Leu Lys Gly
    210                 215                 220

Asp Ala Ser Leu Lys Met Asp Lys Ser Asp Ala Val Ser His Thr Gly
225                 230                 235                 240

Asn Tyr Thr Cys Glu Val Thr Glu Leu Thr Arg Glu Gly Glu Thr Ile
                245                 250                 255

Ile Glu Leu Lys Tyr Arg Val Val Ser
            260                 265

<210> 56
<211> 274
<212> PRT
<213> Artificial Sequence

<220>
<223> Description of Artificial Sequence: Synthetic polypeptide

<400> 56

Glu Glu Glu Leu Gln Ile Ile Gln Pro Asp Lys Ser Val Leu Val Ala
1               5                   10                  15

Ala Gly Glu Thr Ala Thr Leu Arg Cys Thr Ile Thr Ser Leu Phe Pro
            20                  25                  30

Val Gly Pro Ile Gln Trp Phe Arg Gly Ala Gly Pro Gly Arg Val Leu
        35                  40                  45

Ile Tyr Asn Gln Arg Gln Gly Pro Phe Pro Arg Val Thr Thr Val Ser
    50                  55                  60

```
Asp Thr Thr Lys Arg Asn Asn Met Asp Phe Ser Ile Arg Ile Gly Asn
65                  70                  75                  80

Ile Thr Pro Ala Asp Ala Gly Thr Tyr Tyr Cys Ile Lys Phe Arg Lys
                85                  90                  95

Gly Ser Pro Asp Asp Val Glu Phe Lys Ser Gly Ala Gly Thr Glu Leu
            100                 105                 110

Ser Val Arg Ala Lys Pro Ser Gly Gly Ser Gly Gly Ser Gly Gly Ser
        115                 120                 125

Gly Gly Ser Leu Ser Gly Arg Ser Asp Asn His Gly Gly Ser Gly Gly
    130                 135                 140

Ser Gly Gly Ser Gly Gly Ser Gly Gly Ser Gly Gly Ser Gln Leu Leu
145                 150                 155                 160

Phe Asn Lys Thr Lys Ser Val Glu Phe Thr Phe Ser Asn Asp Thr Val
                165                 170                 175

Val Ile Pro Cys Phe Val Thr Asn Met Glu Ala Gln Asn Thr Thr Glu
            180                 185                 190

Val Tyr Val Lys Trp Lys Phe Lys Gly Arg Asp Ile Tyr Thr Phe Asp
        195                 200                 205

Gly Ala Leu Asn Lys Ser Thr Val Pro Thr Asp Phe Ser Ser Ala Lys
    210                 215                 220

Ile Glu Val Ser Gln Leu Leu Lys Gly Asp Ala Ser Leu Lys Met Asp
225                 230                 235                 240

Lys Ser Asp Ala Val Ser His Thr Gly Asn Tyr Thr Cys Glu Val Thr
                245                 250                 255

Glu Leu Thr Arg Glu Gly Glu Thr Ile Ile Glu Leu Lys Tyr Arg Val
            260                 265                 270

Val Ser
```

<210> 57
<211> 270
<212> PRT
<213> Artificial Sequence

<220>
<223> Description of Artificial Sequence: Synthetic polypeptide

<400> 57

```
Gly Gln Leu Leu Phe Asn Lys Thr Lys Ser Val Glu Phe Thr Phe Ser
1               5                   10                  15

Asn Asp Thr Val Val Ile Pro Cys Phe Val Thr Asn Met Glu Ala Gln
            20                  25                  30

Asn Thr Thr Glu Val Tyr Val Lys Trp Lys Phe Lys Gly Arg Asp Ile
        35                  40                  45

Tyr Thr Phe Asp Gly Ala Leu Asn Lys Ser Thr Val Pro Thr Asp Phe
    50                  55                  60

Ser Ser Ala Lys Ile Glu Val Ser Gln Leu Leu Lys Gly Asp Ala Ser
65                  70                  75                  80

Leu Lys Met Asp Lys Ser Asp Ala Val Ser His Thr Gly Asn Tyr Thr
                85                  90                  95

Cys Glu Val Thr Glu Leu Thr Arg Glu Gly Glu Thr Ile Ile Glu Leu
            100                 105                 110

Lys Tyr Arg Val Val Ser Gly Gly Gly Gly Ser Gly Gly Gly Gly Ser
        115                 120                 125

Gly Gly Gly Gly Ser Leu Ser Gly Arg Ser Asp Asn His Gly Gly Gly
    130                 135                 140

Gly Ser Gly Gly Gly Gly Ser Glu Glu Glu Leu Gln Ile Ile Gln Pro
145                 150                 155                 160

Asp Lys Ser Val Leu Val Ala Ala Gly Glu Thr Ala Thr Leu Arg Cys
                165                 170                 175

Thr Ile Thr Ser Leu Phe Pro Val Gly Pro Ile Gln Trp Phe Arg Gly
            180                 185                 190

Ala Gly Pro Gly Arg Val Leu Ile Tyr Asn Gln Arg Gln Gly Pro Phe
        195                 200                 205

Pro Arg Val Thr Thr Val Ser Asp Thr Thr Lys Arg Asn Asn Met Asp
    210                 215                 220

Phe Ser Ile Arg Ile Gly Asn Ile Thr Pro Ala Asp Ala Gly Thr Tyr
225                 230                 235                 240
```

Tyr Cys Ile Lys Phe Arg Lys Gly Ser Pro Asp Asp Val Glu Phe Lys
245 250 255

Ser Gly Ala Gly Thr Glu Leu Ser Val Arg Ala Lys Pro Ser
260 265 270

<210> 58
<211> 275
<212> PRT
<213> Artificial Sequence

<220>
<223> Description of Artificial Sequence: Synthetic polypeptide

<400> 58

Gly Gln Leu Leu Phe Asn Lys Thr Lys Ser Val Glu Phe Thr Phe Ser
1 5 10 15

Asn Asp Thr Val Val Ile Pro Cys Phe Val Thr Asn Met Glu Ala Gln
20 25 30

Asn Thr Thr Glu Val Tyr Val Lys Trp Lys Phe Lys Gly Arg Asp Ile
35 40 45

Tyr Thr Phe Asp Gly Ala Leu Asn Lys Ser Thr Val Pro Thr Asp Phe
50 55 60

Ser Ser Ala Lys Ile Glu Val Ser Gln Leu Leu Lys Gly Asp Ala Ser
65 70 75 80

Leu Lys Met Asp Lys Ser Asp Ala Val Ser His Thr Gly Asn Tyr Thr
85 90 95

Cys Glu Val Thr Glu Leu Thr Arg Glu Gly Glu Thr Ile Ile Glu Leu
100 105 110

Lys Tyr Arg Val Val Ser Gly Gly Gly Gly Ser Gly Gly Gly Gly Ser
115 120 125

Gly Gly Gly Gly Ser Gly Gly Gly Gly Ser Leu Ser Gly Arg Ser Asp
130 135 140

Asn His Gly Gly Gly Gly Ser Gly Gly Gly Gly Ser Glu Glu Glu Leu
145 150 155 160

Gln Ile Ile Gln Pro Asp Lys Ser Val Leu Val Ala Ala Gly Glu Thr
165 170 175

```
Ala Thr Leu Arg Cys Thr Ile Thr Ser Leu Phe Pro Val Gly Pro Ile
            180                 185                 190

Gln Trp Phe Arg Gly Ala Gly Pro Gly Arg Val Leu Ile Tyr Asn Gln
        195                 200                 205

Arg Gln Gly Pro Phe Pro Arg Val Thr Thr Val Ser Asp Thr Thr Lys
    210                 215                 220

Arg Asn Asn Met Asp Phe Ser Ile Arg Ile Gly Asn Ile Thr Pro Ala
225                 230                 235                 240

Asp Ala Gly Thr Tyr Tyr Cys Ile Lys Phe Arg Lys Gly Ser Pro Asp
                245                 250                 255

Asp Val Glu Phe Lys Ser Gly Ala Gly Thr Glu Leu Ser Val Arg Ala
            260                 265                 270

Lys Pro Ser
        275
```

<210> 59
<211> 280
<212> PRT
<213> Artificial Sequence

<220>
<223> Description of Artificial Sequence: Synthetic polypeptide

<400> 59

```
Gly Gln Leu Leu Phe Asn Lys Thr Lys Ser Val Glu Phe Thr Phe Ser
1               5                   10                  15

Asn Asp Thr Val Val Ile Pro Cys Phe Val Thr Asn Met Glu Ala Gln
            20                  25                  30

Asn Thr Thr Glu Val Tyr Val Lys Trp Lys Phe Lys Gly Arg Asp Ile
        35                  40                  45

Tyr Thr Phe Asp Gly Ala Leu Asn Lys Ser Thr Val Pro Thr Asp Phe
    50                  55                  60

Ser Ser Ala Lys Ile Glu Val Ser Gln Leu Leu Lys Gly Asp Ala Ser
65                  70                  75                  80

Leu Lys Met Asp Lys Ser Asp Ala Val Ser His Thr Gly Asn Tyr Thr
                85                  90                  95

Cys Glu Val Thr Glu Leu Thr Arg Glu Gly Glu Thr Ile Ile Glu Leu
```

```
                100                 105                 110

Lys Tyr Arg Val Val Ser Gly Gly Gly Gly Ser Gly Gly Gly Gly Ser
        115                 120                 125

Gly Gly Gly Gly Ser Gly Gly Gly Gly Ser Gly Gly Gly Gly Ser Leu
    130                 135                 140

Ser Gly Arg Ser Asp Asn His Gly Gly Gly Gly Ser Gly Gly Gly Gly
145                 150                 155                 160

Ser Glu Glu Glu Leu Gln Ile Ile Gln Pro Asp Lys Ser Val Leu Val
                165                 170                 175

Ala Ala Gly Glu Thr Ala Thr Leu Arg Cys Thr Ile Thr Ser Leu Phe
            180                 185                 190

Pro Val Gly Pro Ile Gln Trp Phe Arg Gly Ala Gly Pro Gly Arg Val
        195                 200                 205

Leu Ile Tyr Asn Gln Arg Gln Gly Pro Phe Pro Arg Val Thr Thr Val
    210                 215                 220

Ser Asp Thr Thr Lys Arg Asn Asn Met Asp Phe Ser Ile Arg Ile Gly
225                 230                 235                 240

Asn Ile Thr Pro Ala Asp Ala Gly Thr Tyr Tyr Cys Ile Lys Phe Arg
                245                 250                 255

Lys Gly Ser Pro Asp Asp Val Glu Phe Lys Ser Gly Ala Gly Thr Glu
            260                 265                 270

Leu Ser Val Arg Ala Lys Pro Ser
        275                 280
```

<210> 60
<211> 274
<212> PRT
<213> Artificial Sequence

<220>
<223> Description of Artificial Sequence: Synthetic polypeptide

<400> 60

```
Gln Leu Leu Phe Asn Lys Thr Lys Ser Val Glu Phe Thr Phe Ser Asn
1               5                   10                  15

Asp Thr Val Val Ile Pro Cys Phe Val Thr Asn Met Glu Ala Gln Asn
            20                  25                  30
```

```
Thr Thr Glu Val Tyr Val Lys Trp Lys Phe Lys Gly Arg Asp Ile Tyr
        35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65                  70                  75                  80

Lys Met Asp Lys Ser Asp Ala Val Ser His Thr Gly Asn Tyr Thr Cys
                85                  90                  95

Glu Val Thr Glu Gln Thr Arg Glu Gly Glu Thr Ile Ile Glu Leu Lys
            100                 105                 110

Tyr Arg Val Val Ser Gly Gly Gly Gly Ser Gly Gly Gly Gly Ser Gly
        115                 120                 125

Gly Gly Gly Ser Gly Gly Gly Gly Ser Leu Ser Gly Arg Ser Asp Asn
    130                 135                 140

His Gly Gly Gly Gly Ser Gly Gly Gly Gly Ser Glu Glu Glu Leu Gln
145                 150                 155                 160

Ile Ile Gln Pro Asp Lys Ser Val Leu Val Ala Ala Gly Glu Thr Ala
                165                 170                 175

Thr Leu Arg Cys Thr Ile Thr Ser Leu Phe Pro Val Gly Pro Ile Gln
            180                 185                 190

Trp Phe Arg Gly Ala Gly Pro Gly Arg Val Leu Ile Tyr Asn Gln Arg
        195                 200                 205

Gln Gly Pro Phe Pro Arg Val Thr Thr Val Ser Asp Thr Thr Lys Arg
    210                 215                 220

Asn Asn Met Asp Phe Ser Ile Arg Ile Gly Asn Ile Thr Pro Ala Asp
225                 230                 235                 240

Ala Gly Thr Tyr Tyr Cys Ile Lys Phe Arg Lys Gly Ser Pro Asp Asp
                245                 250                 255

Val Glu Phe Lys Ser Gly Ala Gly Thr Glu Leu Ser Val Arg Ala Lys
            260                 265                 270

Pro Ser
```

<210> 61
<211> 274
<212> PRT
<213> Artificial Sequence

<220>
<223> Description of Artificial Sequence: Synthetic polypeptide

<400> 61

```
Gln Leu Leu Phe Asn Lys Thr Lys Ser Val Glu Phe Thr Phe Ser Asn
1               5                   10                  15

Asp Thr Val Val Ile Pro Cys Phe Val Thr Asn Met Glu Ala Gln Asn
            20                  25                  30

Thr Thr Glu Val Tyr Val Lys Trp Lys Phe Lys Gly Arg Asp Ile Tyr
        35                  40                  45

Thr Phe Asp Gly Ala Leu Asn Lys Ser Thr Val Pro Thr Asp Phe Ser
    50                  55                  60

Ser Ala Lys Ile Glu Val Ser Gln Leu Leu Lys Gly Asp Ala Ser Leu
65                  70                  75                  80

Lys Met Asp Lys Ser Asp Ala Val Ser His Thr Gly Asn Tyr Thr Cys
                85                  90                  95

Glu Val Thr Glu Tyr Thr Arg Glu Gly Glu Thr Ile Ile Glu Leu Lys
            100                 105                 110

Tyr Arg Val Val Ser Gly Gly Gly Gly Ser Gly Gly Gly Gly Ser Gly
        115                 120                 125

Gly Gly Gly Ser Gly Gly Gly Gly Ser Leu Ser Gly Arg Ser Asp Asn
    130                 135                 140

His Gly Gly Gly Gly Ser Gly Gly Gly Gly Ser Glu Glu Glu Leu Gln
145                 150                 155                 160

Ile Ile Gln Pro Asp Lys Ser Val Leu Val Ala Ala Gly Glu Thr Ala
                165                 170                 175

Thr Leu Arg Cys Thr Ile Thr Ser Leu Phe Pro Val Gly Pro Ile Gln
            180                 185                 190

Trp Phe Arg Gly Ala Gly Pro Gly Arg Val Leu Ile Tyr Asn Gln Arg
        195                 200                 205
```

Gln Gly Pro Phe Pro Arg Val Thr Thr Val Ser Asp Thr Thr Lys Arg
210 215 220

Asn Asn Met Asp Phe Ser Ile Arg Ile Gly Asn Ile Thr Pro Ala Asp
225 230 235 240

Ala Gly Thr Tyr Tyr Cys Ile Lys Phe Arg Lys Gly Ser Pro Asp Asp
245 250 255

Val Glu Phe Lys Ser Gly Ala Gly Thr Glu Leu Ser Val Arg Ala Lys
260 265 270

Pro Ser

<210> 62
<211> 274
<212> PRT
<213> Artificial Sequence

<220>
<223> Description of Artificial Sequence: Synthetic polypeptide

<400> 62

Gln Leu Leu Phe Asn Lys Thr Lys Ser Val Glu Phe Thr Phe Ser Asn
1 5 10 15

Asp Thr Val Val Ile Pro Cys Phe Val Thr Asn Met Glu Ala Gln Asn
20 25 30

Thr Thr Glu Val Tyr Val Lys Trp Lys Phe Lys Gly Arg Asp Ile Tyr
35 40 45

Thr Phe Asp Gly Ala Leu Asn Lys Ser Thr Val Pro Thr Asp Phe Ser
50 55 60

Ser Ala Lys Ile Glu Val Ser Gln Leu Leu Lys Gly Asp Ala Ser Leu
65 70 75 80

Lys Met Asp Lys Ser Asp Ala Val Ser His Thr Gly Asn Tyr Thr Cys
85 90 95

Glu Val Thr Glu Leu Gln Arg Glu Gly Glu Thr Ile Ile Glu Leu Lys
100 105 110

Tyr Arg Val Val Ser Gly Gly Gly Gly Ser Gly Gly Gly Gly Ser Gly
115 120 125

```
Gly Gly Gly Ser Gly Gly Gly Gly Ser Leu Ser Gly Arg Ser Asp Asn
    130                 135                 140

His Gly Gly Gly Gly Ser Gly Gly Gly Gly Ser Glu Glu Glu Leu Gln
145                 150                 155                 160

Ile Ile Gln Pro Asp Lys Ser Val Leu Val Ala Ala Gly Glu Thr Ala
                165                 170                 175

Thr Leu Arg Cys Thr Ile Thr Ser Leu Phe Pro Val Gly Pro Ile Gln
            180                 185                 190

Trp Phe Arg Gly Ala Gly Pro Gly Arg Val Leu Ile Tyr Asn Gln Arg
        195                 200                 205

Gln Gly Pro Phe Pro Arg Val Thr Thr Val Ser Asp Thr Thr Lys Arg
    210                 215                 220

Asn Asn Met Asp Phe Ser Ile Arg Ile Gly Asn Ile Thr Pro Ala Asp
225                 230                 235                 240

Ala Gly Thr Tyr Tyr Cys Ile Lys Phe Arg Lys Gly Ser Pro Asp Asp
                245                 250                 255

Val Glu Phe Lys Ser Gly Ala Gly Thr Glu Leu Ser Val Arg Ala Lys
            260                 265                 270

Pro Ser
```

<210> 63
<211> 274
<212> PRT
<213> Artificial Sequence

<220>
<223> Description of Artificial Sequence: Synthetic polypeptide

<400> 63

```
Gln Leu Leu Phe Asn Lys Thr Lys Ser Val Glu Phe Thr Phe Ser Asn
1               5                   10                  15

Asp Thr Val Val Ile Pro Cys Phe Val Thr Asn Met Glu Ala Gln Asn
            20                  25                  30

Thr Thr Glu Val Tyr Val Lys Trp Lys Phe Lys Gly Arg Asp Ile Tyr
        35                  40                  45
```

Thr Phe Asp Gly Ala Leu Asn Lys Ser Thr Val Pro Thr Asp Phe Ser
50 55 60

Ser Ala Lys Ile Glu Val Ser Gln Leu Leu Lys Gly Asp Ala Ser Leu
65 70 75 80

Lys Met Asp Lys Ser Asp Ala Val Ser His Thr Gly Asn Tyr Thr Cys
85 90 95

Glu Val Thr Glu Leu His Arg Glu Gly Glu Thr Ile Ile Glu Leu Lys
100 105 110

Tyr Arg Val Val Ser Gly Gly Gly Gly Ser Gly Gly Gly Gly Ser Gly
115 120 125

Gly Gly Gly Ser Gly Gly Gly Gly Ser Leu Ser Gly Arg Ser Asp Asn
130 135 140

His Gly Gly Gly Gly Ser Gly Gly Gly Gly Ser Glu Glu Glu Leu Gln
145 150 155 160

Ile Ile Gln Pro Asp Lys Ser Val Leu Val Ala Ala Gly Glu Thr Ala
165 170 175

Thr Leu Arg Cys Thr Ile Thr Ser Leu Phe Pro Val Gly Pro Ile Gln
180 185 190

Trp Phe Arg Gly Ala Gly Pro Gly Arg Val Leu Ile Tyr Asn Gln Arg
195 200 205

Gln Gly Pro Phe Pro Arg Val Thr Thr Val Ser Asp Thr Thr Lys Arg
210 215 220

Asn Asn Met Asp Phe Ser Ile Arg Ile Gly Asn Ile Thr Pro Ala Asp
225 230 235 240

Ala Gly Thr Tyr Tyr Cys Ile Lys Phe Arg Lys Gly Ser Pro Asp Asp
245 250 255

Val Glu Phe Lys Ser Gly Ala Gly Thr Glu Leu Ser Val Arg Ala Lys
260 265 270

Pro Ser

<210> 64
<211> 12
<212> PRT

<213> Artificial Sequence

<220>
<223> Description of Artificial Sequence: Synthetic peptide

<400> 64

```
Cys Gln Phe Asp Leu Ser Thr Arg Arg Leu Lys Cys
1               5                   10
```

<210> 65
<211> 12
<212> PRT
<213> Artificial Sequence

<220>
<223> Description of Artificial Sequence: Synthetic peptide

<400> 65

```
Cys Gln Tyr Asn Leu Ser Ser Arg Ala Leu Lys Cys
1               5                   10
```

<210> 66
<211> 183
<212> PRT
<213> Artificial Sequence

<220>
<223> Description of Artificial Sequence: Synthetic polypeptide

<400> 66

```
Cys Gln Phe Asp Leu Ser Thr Arg Arg Leu Lys Cys Gly Gly Gly Gly
1               5                   10                  15

Ser Gly Gly Gly Gly Ser Gly Gly Gly Gly Ser Gly Gly Gly Gly Ser
            20                  25                  30

Glu Glu Glu Leu Gln Ile Ile Gln Pro Asp Lys Ser Val Leu Val Ala
        35                  40                  45

Ala Gly Glu Thr Ala Thr Leu Arg Cys Thr Ile Thr Ser Leu Phe Pro
    50                  55                  60

Val Gly Pro Ile Gln Trp Phe Arg Gly Ala Gly Pro Gly Arg Val Leu
65                  70                  75                  80

Ile Tyr Asn Gln Arg Gln Gly Pro Phe Pro Arg Val Thr Thr Val Ser
                85                  90                  95

Asp Thr Thr Lys Arg Asn Asn Met Asp Phe Ser Ile Arg Ile Gly Asn
            100                 105                 110
```

```
Ile Thr Pro Ala Asp Ala Gly Thr Tyr Tyr Cys Ile Lys Phe Arg Lys
        115                 120                 125

Gly Ser Pro Asp Asp Val Glu Phe Lys Ser Gly Ala Gly Thr Glu Leu
    130                 135                 140

Ser Val Arg Ala Lys Pro Ser Gly Gly Gly Gly Ser Gly Gly Gly Gly
145                 150                 155                 160

Ser Gly Gly Gly Gly Ser Gly Gly Gly Gly Ser Cys Gln Phe Asp Leu
                165                 170                 175

Ser Thr Arg Arg Leu Lys Cys
            180
```

<210> 67
<211> 585
<212> PRT
<213> Homo sapiens

<400> 67

Asp Ala His Lys Ser Glu Val Ala His Arg Phe Lys Asp Leu Gly Glu
1 5 10 15

Glu Asn Phe Lys Ala Leu Val Leu Ile Ala Phe Ala Gln Tyr Leu Gln
20 25 30

Gln Cys Pro Phe Glu Asp His Val Lys Leu Val Asn Glu Val Thr Glu
35 40 45

Phe Ala Lys Thr Cys Val Ala Asp Glu Ser Ala Glu Asn Cys Asp Lys
50 55 60

Ser Leu His Thr Leu Phe Gly Asp Lys Leu Cys Thr Val Ala Thr Leu
65 70 75 80

Arg Glu Thr Tyr Gly Glu Met Ala Asp Cys Cys Ala Lys Gln Glu Pro
85 90 95

Glu Arg Asn Glu Cys Phe Leu Gln His Lys Asp Asp Asn Pro Asn Leu
100 105 110

Pro Arg Leu Val Arg Pro Glu Val Asp Val Met Cys Thr Ala Phe His
115 120 125

Asp Asn Glu Glu Thr Phe Leu Lys Lys Tyr Leu Tyr Glu Ile Ala Arg
130 135 140

Arg His Pro Tyr Phe Tyr Ala Pro Glu Leu Leu Phe Phe Ala Lys Arg

145 150 155 160

Tyr Lys Ala Ala Phe Thr Glu Cys Cys Gln Ala Ala Asp Lys Ala Ala
165 170 175

Cys Leu Leu Pro Lys Leu Asp Glu Leu Arg Asp Glu Gly Lys Ala Ser
180 185 190

Ser Ala Lys Gln Arg Leu Lys Cys Ala Ser Leu Gln Lys Phe Gly Glu
195 200 205

Arg Ala Phe Lys Ala Trp Ala Val Ala Arg Leu Ser Gln Arg Phe Pro
210 215 220

Lys Ala Glu Phe Ala Glu Val Ser Lys Leu Val Thr Asp Leu Thr Lys
225 230 235 240

Val His Thr Glu Cys Cys His Gly Asp Leu Leu Glu Cys Ala Asp Asp
245 250 255

Arg Ala Asp Leu Ala Lys Tyr Ile Cys Glu Asn Gln Asp Ser Ile Ser
260 265 270

Ser Lys Leu Lys Glu Cys Cys Glu Lys Pro Leu Leu Glu Lys Ser His
275 280 285

Cys Ile Ala Glu Val Glu Asn Asp Glu Met Pro Ala Asp Leu Pro Ser
290 295 300

Leu Ala Ala Asp Phe Val Glu Ser Lys Asp Val Cys Lys Asn Tyr Ala
305 310 315 320

Glu Ala Lys Asp Val Phe Leu Gly Met Phe Leu Tyr Glu Tyr Ala Arg
325 330 335

Arg His Pro Asp Tyr Ser Val Val Leu Leu Leu Arg Leu Ala Lys Thr
340 345 350

Tyr Glu Thr Thr Leu Glu Lys Cys Cys Ala Ala Ala Asp Pro His Glu
355 360 365

Cys Tyr Ala Lys Val Phe Asp Glu Phe Lys Pro Leu Val Glu Glu Pro
370 375 380

Gln Asn Leu Ile Lys Gln Asn Cys Glu Leu Phe Glu Gln Leu Gly Glu
385 390 395 400

```
Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu
            580                 585
```

<210> 68
<211> 585
<212> PRT
<213> Homo sapiens

<400> 68

```
Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Ser Pro Phe Glu Asp His Val Lys Leu Val Asn Glu Val Thr Glu
```

```
            35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala
                165                 170                 175

Cys Leu Leu Pro Lys Leu Asp Glu Leu Arg Asp Glu Gly Lys Ala Ser
            180                 185                 190

Ser Ala Lys Gln Arg Leu Lys Cys Ala Ser Leu Gln Lys Phe Gly Glu
        195                 200                 205

Arg Ala Phe Lys Ala Trp Ala Val Ala Arg Leu Ser Gln Arg Phe Pro
    210                 215                 220

Lys Ala Glu Phe Ala Glu Val Ser Lys Leu Val Thr Asp Leu Thr Lys
225                 230                 235                 240

Val His Thr Glu Cys Cys His Gly Asp Leu Leu Glu Cys Ala Asp Asp
                245                 250                 255

Arg Ala Asp Leu Ala Lys Tyr Ile Cys Glu Asn Gln Asp Ser Ile Ser
            260                 265                 270

Ser Lys Leu Lys Glu Cys Cys Glu Lys Pro Leu Leu Glu Lys Ser His
        275                 280                 285
```

```
Cys Ile Ala Glu Val Glu Asn Asp Glu Met Pro Ala Asp Leu Pro Ser
    290                 295                 300

Leu Ala Ala Asp Phe Val Glu Ser Lys Asp Val Cys Lys Asn Tyr Ala
305                 310                 315                 320

Glu Ala Lys Asp Val Phe Leu Gly Met Phe Leu Tyr Glu Tyr Ala Arg
                325                 330                 335

Arg His Pro Asp Tyr Ser Val Val Leu Leu Leu Arg Leu Ala Lys Thr
            340                 345                 350

Tyr Glu Thr Thr Leu Glu Lys Cys Cys Ala Ala Ala Asp Pro His Glu
        355                 360                 365

Cys Tyr Ala Lys Val Phe Asp Glu Phe Lys Pro Leu Val Glu Glu Pro
    370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540
```

Lys Ala Val Met Asp Asp Phe Ala Ala Phe Val Glu Lys Cys Cys Lys
545 550 555 560

Ala Asp Asp Lys Glu Thr Cys Phe Ala Glu Glu Gly Lys Lys Leu Val
565 570 575

Ala Ala Ser Gln Ala Ala Leu Gly Leu
580 585

<210> 69
<211> 12
<212> PRT
<213> Unknown

<220>
<223> Description of Unknown: uPA cleavable peptide

<220>
<221> MOD_RES
<222> (4)..(4)
<223> Any amino acid

<220>
<221> MOD_RES
<222> (6) .. (7)
<223> Any amino acid

<220>
<221> MOD_RES
<222> (9) .. (9)
<223> Any amino acid

<400> 69

Leu Ser Gly Xaa Arg Xaa Xaa Ser Xaa Asp Asn His
1 5 10

<210> 70
<211> 12
<212> PRT
<213> Unknown

<220>
<223> Description of Unknown: uPA cleavable peptide

<220>
<221> MOD_RES
<222> (1)..(1)
<223> Any amino acid

<220>
<221> MOD_RES
<222> (7)..(7)
<223> Any amino acid

<220>

<221> MOD_RES
<222> (10)..(12)
<223> Any amino acid

<400> 70

```
Xaa Ser Gly Ser Arg Lys Xaa Arg Val Xaa Xaa Xaa
1               5                   10
```

<210> 71
<211> 6
<212> PRT
<213> Unknown

<220>
<223> Description of Unknown: uPA cleavable peptide

<220>
<221> MOD_RES
<222> (4)..(4)
<223> Any amino acid

<400> 71

```
Ser Gly Arg Xaa Ser Ala
1               5
```

<210> 72
<211> 12
<212> PRT
<213> Unknown

<220>
<223> Description of Unknown: Matriptase cleavable peptide

<220>
<221> MOD_RES
<222> (4)..(4)
<223> Any amino acid

<220>
<221> MOD_RES
<222> (6) .. (7)
<223> Any amino acid

<220>
<221> MOD_RES
<222> (9) .. (9)
<223> Any amino acid

<400> 72

```
Leu Ser Gly Xaa Arg Xaa Xaa Ser Xaa Asp Asn His
1               5                   10
```

<210> 73
<211> 12
<212> PRT

<213> Unknown

<220>
<223> Description of Unknown: Matriptase cleavable peptide

<220>
<221> MOD_RES
<222> (2)..(4)
<223> Any amino acid

<220>
<221> MOD_RES
<222> (7)..(7)
<223> Any amino acid

<220>
<221> MOD_RES
<222> (9)..(10)
<223> Any amino acid

<220>
<221> MOD_RES
<222> (12) .. (12)
<223> Any amino acid

<400> 73

```
Arg Xaa Xaa Xaa Arg Lys Xaa Val Xaa Xaa Gly Xaa
1               5                   10
```

<210> 74
<211> 7
<212> PRT
<213> Unknown

<220>
<223> Description of Unknown: Matriptase cleavable peptide

<220>
<221> MOD_RES
<222> (5)..(5)
<223> Any amino acid

<400> 74

```
Arg Gln Ala Arg Xaa Val Val
1               5
```

<210> 75
<211> 8
<212> PRT
<213> Unknown

<220>
<223> Description of Unknown: Matriptase cleavable peptide

<220>
<221> MOD_RES

<222> (2) .. (3)
<223> Any amino acid

<220>
<221> MOD_RES
<222> (7)..(7)
<223> Any amino acid

<400> 75

Arg Xaa Xaa Arg Lys Val Xaa Gly
1               5

<210> 76
<211> 11
<212> PRT
<213> Unknown

<220>
<223> Description of Unknown: Matriptase cleavable peptide

<400> 76

Lys Arg Arg Lys Gln Gly Ala Ser Arg Lys Ala
1               5                   10

<210> 77
<211> 12
<212> PRT
<213> Unknown

<220>
<223> Description of Unknown: Legumain cleavable peptide

<220>
<221> MOD_RES
<222> (4)..(4)
<223> Any amino acid

<220>
<221> MOD_RES
<222> (6) .. (7)
<223> Any amino acid

<220>
<221> MOD_RES
<222> (9) .. (9)
<223> Any amino acid

<400> 77

Leu Ser Gly Xaa Arg Xaa Xaa Ser Xaa Asp Asn His
1               5                   10

<210> 78
<211> 10
<212> PRT
<213> Unknown

<220>
<223> Description of Unknown: Legumain cleavable peptide

<220>
<221> MOD_RES
<222> (1) .. (6)
<223> Any amino acid

<220>
<221> MOD_RES
<222> (8)..(10)
<223> Any amino acid

<400> 78

```
Xaa Xaa Xaa Xaa Xaa Xaa Asn Xaa Xaa Xaa
1               5                   10
```

<210> 79
<211> 5
<212> PRT
<213> Unknown

<220>
<223> Description of Unknown: Legumain cleavable peptide

<220>
<221> MOD_RES
<222> (4)..(4)
<223> Any amino acid

<400> 79

```
Ala Ala Asn Xaa Leu
1               5
```

<210> 80
<211> 5
<212> PRT
<213> Unknown

<220>
<223> Description of Unknown: Legumain cleavable peptide

<220>
<221> MOD_RES
<222> (4)..(4)
<223> Any amino acid

<400> 80

```
Ala Thr Asn Xaa Leu
1               5
```

<210> 81
<211> 12
<212> PRT
<213> Unknown

<220>
<223> Description of Unknown: PSA cleavable peptide

<220>
<221> MOD_RES
<222> (5)..(5)
<223> Any amino acid

<220>
<221> MOD_RES
<222> (11)..(12)
<223> Any amino acid

<400> 81

```
Ser Ile Ser Gln Xaa Tyr Gln Arg Ser Ser Xaa Xaa
1               5                   10
```

<210> 82
<211> 5
<212> PRT
<213> Unknown

<220>
<223> Description of Unknown: PSA cleavable peptide

<400> 82

```
Ser Ser Lys Leu Gln
1               5
```

<210> 83
<211> 9
<212> PRT
<213> Unknown

<220>
<223> Description of Unknown: MMP2 cleavable peptide

<220>
<221> MOD_RES
<222> (1)..(1)
<223> Any amino acid

<220>
<221> MOD_RES
<222> (3)..(4)
<223> Any amino acid

<220>
<221> MOD_RES
<222> (7) .. (9)
<223> Any amino acid

<400> 83

```
Xaa Pro Xaa Xaa Leu Ile Xaa Xaa Xaa
1               5
```

<210> 84
<211> 9
<212> PRT
<213> Unknown

<220>
<223> Description of Unknown: MMP9 cleavable peptide

<220>
<221> MOD_RES
<222> (4)..(4)
<223> Any amino acid

<220>
<221> MOD_RES
<222> (7)..(7)
<223> Any amino acid

<220>
<221> MOD_RES
<222> (9) .. (9)
<223> Any amino acid

<400> 84

```
Gly Pro Ala Xaa Gly Leu Xaa Gly Xaa
1               5
```

<210> 85
<211> 8
<212> PRT
<213> Unknown

<220>
<223> Description of Unknown: MMP9 cleavable peptide

<400> 85

```
Gly Pro Leu Gly Ile Ala Gly Gln
1               5
```

<210> 86
<211> 6
<212> PRT
<213> Unknown

<220>
<223> Description of Unknown: MMP9 cleavable peptide

<400> 86

```
Pro Val Gly Leu Ile Gly
1               5
```

<210> 87
<211> 8
<212> PRT
<213> Unknown

<220>
<223> Description of Unknown: MMP9 cleavable peptide

<400> 87

His Pro Val Gly Leu Leu Ala Arg
1 5

<210> 88
<211> 11
<212> PRT
<213> Unknown

<220>
<223> Description of Unknown: HNE cleavable peptide

<220>
<221> MOD_RES
<222> (1)..(3)
<223> Any amino acid

<220>
<221> MOD_RES
<222> (8)..(11)
<223> Any amino acid

<400> 88

Xaa Xaa Xaa Val Ile Ala Thr Xaa Xaa Xaa Xaa
1 5 10

<210> 89
<211> 10
<212> PRT
<213> Unknown

<220>
<223> Description of Unknown: Pr3 cleavable peptide

<220>
<221> MOD_RES
<222> (1)..(1)
<223> Any amino acid

<220>
<221> MOD_RES
<222> (7)..(10)
<223> Any amino acid

<400> 89

Xaa Tyr Tyr Val Thr Ala Xaa Xaa Xaa Xaa
1 5 10

<210> 90
<211> 8
<212> PRT
<213> Unknown

<220>
<223> Description of Unknown: Pro-urokinase cleavable peptide

<400> 90

Pro Arg Phe Lys Ile Ile Gly Gly
1               5

<210> 91
<211> 8
<212> PRT
<213> Unknown

<220>
<223> Description of Unknown: Pro-urokinase cleavable peptide

<400> 91

Pro Arg Phe Arg Ile Ile Gly Gly
1               5

<210> 92
<211> 9
<212> PRT
<213> Unknown

<220>
<223> Description of Unknown: TGFbeta cleavable peptide

<400> 92

Ser Ser Arg His Arg Arg Ala Leu Asp
1               5

<210> 93
<211> 14
<212> PRT
<213> Unknown

<220>
<223> Description of Unknown: Plasminogen cleavable peptide

<400> 93

Arg Lys Ser Ser Ile Ile Ile Arg Met Arg Asp Val Val Leu
1               5                   10

<210> 94
<211> 15
<212> PRT
<213> Unknown

<220>
<223> Description of Unknown: Staphylokinase cleavable peptide

<400> 94

Ser Ser Ser Phe Asp Lys Gly Lys Tyr Lys Lys Gly Asp Asp Ala
1 5 10 15

<210> 95
<211> 15
<212> PRT
<213> Unknown

<220>
<223> Description of Unknown: Staphylokinase cleavable peptide

<400> 95

Ser Ser Ser Phe Asp Lys Gly Lys Tyr Lys Arg Gly Asp Asp Ala
1 5 10 15

<210> 96
<211> 4
<212> PRT
<213> Unknown

<220>
<223> Description of Unknown: Factor Xa cleavable peptide

<400> 96

Ile Asp Gly Arg
1

<210> 97
<211> 7
<212> PRT
<213> Unknown

<220>
<223> Description of Unknown: Factor Xa cleavable peptide

<400> 97

Gly Gly Ser Ile Asp Gly Arg
1 5

<210> 98
<211> 6
<212> PRT
<213> Unknown

<220>
<223> Description of Unknown: Gelatinase cleavable peptide

<400> 98

Pro Leu Gly Leu Trp Ala
1 5

<210> 99
<211> 8
<212> PRT

<213> Homo sapiens

<400> 99

Asp Val Ala Gln Phe Val Leu Thr
1 5

<210> 100
<211> 8
<212> PRT
<213> Homo sapiens

<400> 100

Gly Pro Leu Gly Ile Ala Gly Ile
1 5

<210> 101
<211> 8
<212> PRT
<213> Homo sapiens

<400> 101

Tyr Gly Ala Gly Leu Gly Val Val
1 5

<210> 102
<211> 8
<212> PRT
<213> Homo sapiens

<400> 102

Ala Gly Leu Gly Val Val Glu Arg
1 5

<210> 103
<211> 8
<212> PRT
<213> Homo sapiens

<400> 103

Ala Gly Leu Gly Ile Ser Ser Thr
1 5

<210> 104
<211> 8
<212> PRT
<213> Unknown

<220>
<223> Description of Unknown: Autolytic peptide

<400> 104

Val Ala Gln Phe Val Leu Thr Glu
1 5

<210> 105
<211> 8
<212> PRT
<213> Unknown

<220>
<223> Description of Unknown: Autolytic peptide

<400> 105

Ala Gln Phe Val Leu Thr Glu Gly
1 5

<210> 106
<211> 8
<212> PRT
<213> Unknown

<220>
<223> Description of Unknown: Autolytic peptide

<400> 106

Pro Val Gln Pro Ile Gly Pro Gln
1 5

<210> 107
<211> 4
<212> PRT
<213> Unknown

<220>
<223> Description of Unknown: Factor Xa cleavable peptide

<400> 107

Ile Glu Gly Arg
1

<210> 108
<211> 8
<212> PRT
<213> Unknown

<220>
<223> Description of Unknown: Cleavable peptide

<400> 108

Gly Pro Glu Gly Leu Arg Val Gly
1 5

<210> 109
<211> 7
<212> PRT

<213> Unknown

<220>
<223> Description of Unknown: Cleavable peptide

<220>
<221> MOD_RES
<222> (1)..(3)
<223> Any amino acid

<220>
<221> MOD_RES
<222> (5)..(7)
<223> Any amino acid

<400> 109

```
Xaa Xaa Xaa Asn Xaa Xaa Xaa
1               5
```

<210> 110
<211> 12
<212> PRT
<213> Artificial Sequence

<220>
<223> Description of Artificial Sequence: Synthetic peptide

<400> 110

```
Cys Glu Arg Val Ile Gly Thr Gly Trp Val Arg Cys
1               5                   10
```

<210> 111
<211> 5
<212> PRT
<213> Artificial Sequence

<220>
<223> Description of Artificial Sequence: Synthetic peptide

<400> 111

```
Gly Gly Gly Gly Ser
1               5
```

<210> 112
<211> 6
<212> PRT
<213> Artificial Sequence

<220>
<223> Description of Artificial Sequence: Synthetic peptide

<400> 112

```
Gln Leu Leu Phe Asn Lys
1               5
```

<210> 113
<211> 7
<212> PRT
<213> Artificial Sequence

<220>
<223> Description of Artificial Sequence: Synthetic peptide

<400> 113

Gly Gln Leu Leu Phe Asn Lys
1 5

<210> 114
<211> 7
<212> PRT
<213> Artificial Sequence

<220>
<223> Description of Artificial Sequence: Synthetic peptide

<400> 114

Gln Gly Leu Leu Phe Asn Lys
1 5

<210> 115
<211> 4
<212> PRT
<213> Artificial Sequence

<220>
<223> Description of Artificial Sequence: Synthetic peptide

<400> 115

Gly Gly Ser Gly

1

<210> 116
<211> 4
<212> PRT
<213> Artificial Sequence

<220>
<223> Description of Artificial Sequence: Synthetic peptide

<400> 116

Ser Gly Gly Gly
1

<210> 117
<211> 4

<212> PRT
<213> Artificial Sequence

<220>
<223> Description of Artificial Sequence: Synthetic peptide

<400> 117

```
Gly Ser Gly Ser
1
```

<210> 118
<211> 6
<212> PRT
<213> Artificial Sequence

<220>
<223> Description of Artificial Sequence: Synthetic peptide

<400> 118

```
Gly Ser Gly Ser Gly Ser
1               5
```

<210> 119
<211> 8
<212> PRT
<213> Artificial Sequence

<220>
<223> Description of Artificial Sequence: Synthetic peptide

<400> 119

```
Gly Ser Gly Ser Gly Ser Gly Ser
1               5
```

<210> 120
<211> 10
<212> PRT
<213> Artificial Sequence

<220>
<223> Description of Artificial Sequence: Synthetic peptide

<400> 120

```
Gly Ser Gly Ser Gly Ser Gly Ser Gly Ser
1               5                   10
```

<210> 121
<211> 12
<212> PRT
<213> Artificial Sequence

<220>
<223> Description of Artificial Sequence: Synthetic peptide

<400> 121

Gly Ser Gly Ser Gly Ser Gly Ser Gly Ser Gly Ser
1               5                   10

<210> 122
<211> 6
<212> PRT
<213> Artificial Sequence

<220>
<223> Description of Artificial Sequence: Synthetic peptide

<400> 122

Gly Gly Ser Gly Gly Ser
1               5

<210> 123
<211> 9
<212> PRT
<213> Artificial Sequence

<220>
<223> Description of Artificial Sequence: Synthetic peptide

<400> 123

Gly Gly Ser Gly Gly Ser Gly Gly Ser
1               5

<210> 124
<211> 12
<212> PRT
<213> Artificial Sequence

<220>
<223> Description of Artificial Sequence: Synthetic peptide

<400> 124

Gly Gly Ser Gly Gly Ser Gly Gly Ser Gly Gly Ser
1               5                   10

<210> 125
<211> 8
<212> PRT
<213> Artificial Sequence

<220>
<223> Description of Artificial Sequence: Synthetic peptide

<400> 125

Gly Gly Ser Gly Gly Gly Ser Gly
1 5

<210> 126
<211> 12
<212> PRT
<213> Artificial Sequence

<220>
<223> Description of Artificial Sequence: Synthetic peptide

<400> 126

Gly Gly Ser Gly Gly Gly Ser Gly Gly Gly Ser Gly
1 5 10

<210> 127
<211> 50
<212> PRT
<213> Artificial Sequence

<220>
<223> Description of Artificial Sequence: Synthetic polypeptide

<220>
<221> MISC_FEATURE
<222> (1)..(50)
<223> This sequence may encompass 1-10 "Gly Gly Gly Gly Ser" repeating units

<400> 127

Gly Gly Gly Gly Ser Gly Gly Gly Gly Ser Gly Gly Gly Gly Ser Gly
1 5 10 15

Gly Gly Gly Ser Gly Gly Gly Gly Ser Gly Gly Gly Gly Ser Gly Gly
20 25 30

Gly Gly Ser Gly Gly Gly Gly Ser Gly Gly Gly Gly Ser Gly Gly Gly
35 40 45

Gly Ser
50

<210> 128
<211> 10
<212> PRT
<213> Artificial Sequence

<220>
<223> Description of Artificial Sequence: Synthetic peptide

<400> 128

Gly Glu Asn Leu Tyr Phe Gln Ser Gly Gly
1 5 10

<210> 129
<211> 8
<212> PRT
<213> Artificial Sequence

<220>
<223> Description of Artificial Sequence: Synthetic peptide

<400> 129

```
Ser Ala Cys Tyr Cys Glu Leu Ser
1               5
```

<210> 130
<211> 5
<212> PRT
<213> Artificial Sequence

<220>
<223> Description of Artificial Sequence: Synthetic peptide

<400> 130

```
Arg Ser Ile Ala Thr
1               5
```

<210> 131
<211> 17
<212> PRT
<213> Artificial Sequence

<220>
<223> Description of Artificial Sequence: Synthetic peptide

<400> 131

```
Arg Pro Ala Cys Lys Ile Pro Asn Asp Leu Lys Gln Lys Val Met Asn
1               5                   10                  15

His
```

<210> 132
<211> 36
<212> PRT
<213> Artificial Sequence

<220>
<223> Description of Artificial Sequence: Synthetic polypeptide

<400> 132

Gly Gly Ser Ala Gly Gly Ser Gly Ser Gly Ser Ser Gly Gly Ser Ser
1 5 10 15

Gly Ala Ser Gly Thr Gly Thr Ala Gly Gly Thr Gly Ser Gly Ser Gly
20 25 30

Thr Gly Ser Gly
35

<210> 133
<211> 17
<212> PRT
<213> Artificial Sequence

<220>
<223> Description of Artificial Sequence: Synthetic peptide

<400> 133

Ala Ala Ala Asn Ser Ser Ile Asp Leu Ile Ser Val Pro Val Asp Ser
1 5 10 15

Arg

<210> 134
<211> 36
<212> PRT
<213> Artificial Sequence

<220>
<223> Description of Artificial Sequence: Synthetic polypeptide

<400> 134

Gly Gly Ser Gly Gly Gly Ser Glu Gly Gly Gly Ser Glu Gly Gly Gly
1 5 10 15

Ser Glu Gly Gly Gly Ser Glu Gly Gly Gly Ser Glu Gly Gly Gly Ser
20 25 30

Gly Gly Gly Ser
35

<210> 135
<211> 12
<212> PRT
<213> Artificial Sequence

<220>
<223> Description of Artificial Sequence: Synthetic peptide

<400> 135

Gly Gly Gly Ser Gly Gly Gly Ser Gly Gly Gly Ser
1 5 10

<210> 136
<211> 20
<212> PRT
<213> Artificial Sequence

<220>
<223> Description of Artificial Sequence: Synthetic peptide

<400> 136

Ser Gly Gly Gly Ser Gly Gly Gly Ser Gly Gly Gly Ser Gly Gly Gly
1 5 10 15

Ser Gly Gly Gly
20

<210> 137
<211> 18
<212> PRT
<213> Artificial Sequence

<220>
<223> Description of Artificial Sequence: Synthetic peptide

<400> 137

Gly Gly Ser Gly Gly Gly Ser Gly Gly Gly Ser Gly Gly Gly Ser Gly
1 5 10 15

Gly Ser

<210> 138
<211> 6
<212> PRT
<213> Artificial Sequence

<220>
<223> Description of Artificial Sequence: Synthetic 6xHis tag

<400> 138

His His His His His His
1 5

<210> 139
<211> 25
<212> PRT
<213> Artificial Sequence

<220>
<223> Description of Artificial Sequence: Synthetic peptide

<220>

<221> MISC_FEATURE
<222> (1) .. (25)
<223> This sequence may encompass 3-5 "Gly Gly Gly Gly Ser" repeating units

<400> 139

```
Gly Gly Gly Gly Ser Gly Gly Gly Gly Ser Gly Gly Gly Gly Ser Gly
1               5                   10                  15

Gly Gly Gly Ser Gly Gly Gly Gly Ser
            20                  25
```

<210> 140
<211> 15
<212> PRT
<213> Artificial Sequence

<220>
<223> Description of Artificial Sequence: Synthetic peptide

<220>
<221> MISC_FEATURE
<222> (1)..(15)
<223> This sequence may encompass 2-3 "Gly Gly Gly Gly Ser" repeating units

<400> 140

```
Gly Gly Gly Gly Ser Gly Gly Gly Gly Ser Gly Gly Gly Gly Ser
1               5                   10                  15
```

<210> 141
<211> 25
<212> PRT
<213> Artificial Sequence

<220>
<223> Description of Artificial Sequence: Synthetic peptide

<220>
<221> MISC_FEATURE
<222> (1) .. (25)
<223> This sequence may encompass 2-5 "Gly Gly Gly Gly Ser" repeating units

<400> 141

```
Gly Gly Gly Gly Ser Gly Gly Gly Gly Ser Gly Gly Gly Gly Ser Gly
1               5                   10                  15

Gly Gly Gly Ser Gly Gly Gly Gly Ser
            20                  25
```

<210> 142
<211> 18
<212> PRT
<213> Artificial Sequence

<220>

<223> Description of Artificial Sequence: Synthetic peptide

<220>
<221> MISC_FEATURE
<222> (1)..(18)
<223> This sequence may encompass 3-6 "Gly Gly Ser" repeating units

<400> 142

```
Gly Gly Ser Gly Gly Ser Gly Gly Ser Gly Gly Ser Gly Gly Ser Gly
1               5                   10                  15

Gly Ser
```